# EUROPÄISCHE PATENTSCHRIFT

(11) **EP 0 398 288 B1**
(45) Veröffentlichungstag und Bekanntmachung des Hinweises auf die Patenterteilung: **18.10.1995**
(21) Anmeldenummer: 90109235.3
(22) Anmeldetag: 16.05.1990
(51) Int. Cl.: C07C 57/30, C07C 51/15, C07B 53/00

(54) **Verfahren zur Herstellung optisch aktiver 2-Aryl-Alkansäuren, insbesondere von 2-Aryl-Propionsäuren**
Process for preparing optically active 2-arylalkanoic acids, in particular 2-arylpropionic acids
Procédé de préparation d'acides 2-arylalcanoiques optiquement actifs, en particulier d'acides 2-arylpropioniques

(30) Priorität: 16.05.1989 US 352269
(43) Veröffentlichungstag der Anmeldung: 22.11.1990
(73) Patentinhaber: MEDICE Chem.-Pharm. Fabrik Pütter GmbH & Co. KG, 58638 Iserlohn (DE)
(72) Erfinder: Paradies, Henrich H., Prof. Dr. Dr., 5860 Iserlohn (DE); Hanna, Samir B., Prof. Ph.D., Rolla, Missouri 65401 (US); Schneider, Bernd, 5860 Iserlohn (DE)
(74) Vertreter: Reinhard - Skuhra - Weise & Partner

(56) Entgegenhaltungen:
- DE-A- 2 605 650
- GB-A- 1 471 910
- JOURNAL OF ORG. CHEMISTRY, Band 38, Nr. 10, 1973, Seiten 1870-1877; S. YAMAGUGHI et al.: "Asymmetric reductions with chiral reagents from lithium aluminium hydride and (+)-(2S,3R)-4-dimethylamino-3-methyl-1,2-diphenyl1-2-butanol"
- JOURNAL OF THE AMERICAN CHEMICAL SOCIETY, Band 101, Nr. 11, 23. Mai 1979, Seiten 3129-3131; R. NOYORI et al.: "Virtually complete enantioface differentiation in carbonyl group reduction by a complex aluminum hydride reagent"
- JOURNAL OF THE AMERICAN CHEMICAL SOCIETY, Band 109, Nr, 18, 2. September 1987, Seiten 5551-5553; E.J. COREY et al.: "Highly enantioselective borane reduction of ketones catalyzed by chiral oxazaborolidines. Mechanism and synthetic implications"
- JOURNAL OF THE AMERICAN CHEMICAL SOCIETY, Band 108, Nr. 9, 30. April 1986, Seiten 2490-2491; J.F. GARST: "Radical isomerization during grignard reagent formation. A quantitative treatment"

## Beschreibung

Die Erfindung betrifft eine stereospezifische, chemische Synthese optisch reiner Enantiomere der 2-Aryl-Alkansäuren, insbesondere von 2-Aryl-Propionsäuren, in hohen chemischen Ausbeuten und großen Mengen.

1981 gab Shen (Shen T.Y., in : Wolff, M.F. (ed) Burger's Medicinal Chemistry, 4. Ausgabe, Teil III, Wiley, Interscience, New York, S. 1205-1271) einen Überblick über die medizinischen Aspekte von Aryl-Essigsäuren und ihren 2-Methylanalogen, insbesondere den 2-Aryl-Propionsäuren. Insbesondere wurde dargestellt, daß die in vitro-entzündungshemmende Aktivität dem S-Enantiomer zukommt, welches ein optisch aktives Enantiomer des Razemats (R,S)-2-Aryl-propionsäure ist, welches bis zu 150 mal aktiver ist als sein R-Enantiomer, wie es von Adams et al. (S. Adams et al., J.Pharm. Pharmacol., 28, 1976, 256; A.J. Hutt und J. Caldwell, Chemical Pharmacokinetics 9, 1984, 371 beschrieben wurde. Weiterhin unterstützt die chirale Inversion der 2-Aryl-Propionsäuren des R-(-) Enantiomer zum biologischen aktiven S-(+) Enantiomer durch den menschlichen Stoffwechsel, insbesondere im Fall von Ibuprofen (R, S-2- (4-Isobutylphenyl)-propionsäure), das pharmakologisch aktive Prinzip des S-(+)-Enantiomers, was ebenfalls durch die Untersuchungen des S-Enantiomers von Naproxen gestützt wird (A.J. Hutt und J. Caldwell, J.Pharm. Pharmacol., 35, 1983, 693-693). Weiterhin gibt es keine metabolische chirale Inversion der S-(+)-Form zum entsprechenden R-(-)-Enantiomer im Menschen, obwohl eine gewisse stereochemische Inversion in Ratten gelegentlich beobachtet wurde, wahrscheinlich aufgrund von unbekannten stereochemischen Wechselwirkungen der (S)-(+) und R-(-)-Enantiomere am Wirkungsort.

Da die Umwandlung der R-(-)-2-Aryl-Propionsäuren zum pharmakologisch aktiven S-(+)-Enantiomer eine Reaktion von großer medizinischer Auswirkung ist, ist es wahrscheinlich, daß bestimmte Vorteile durch die Verwendung des S-(+)-Enantiomers der 2-Aryl-Alkansäuren als Verbindungen im Gegensatz zu den Razematen erzielt werden. Die Verwendung der S-(+)-Enantiomere würde eine Verringerung der gegebenen Dosis ermöglichen, die gastro-intestinalen Nebenwirkungen sowie die akute Toxizität verringern, die Variabilität in der Geschwindigkeit und dem Grad der Inversion beseitigen und zusätzlich jede Toxizität aufgrund unspezifischer Reaktionen vermindern.

Deshalb besteht die Notwendigkeit eines Verfahrens, welches im industriellen Maßstab arbeitet, um ökonomisch attraktive Ausbeuten dieser S-(+)-Enantiomere von hoher optischer Reinheit > 98 % durch die Anwendung eines stereospezifisch-chemischen Verfahrens herzustellen. Optisch reine Enantiomere der 2-Aryl-Alkansäuren, insbesondere 2-Aryl-Propionsäure, welche sich für eine pharmazeutische Verwendung als ein reines, optisch aktives Stereoisomer , z.B. S-(+)-(6-methoxy-2-naphthyl)-propionsäure (Naproxen) oder S-(+)-Ibuprofen, bewahrt haben, können durch die Verwendung herkömmlicher Wege zur razemischen Abtrennung durch die Anwendung optischer aktiver Basen, z.B. 2-Phenyl-ethyl-amin, N-Methyl-glucamin, Cinchonidin, Brucin oder D-(-)-Threo-1-p-nitrophenyl-2-amino-propan-1,3-diol oder durch biochemische Razematabtrennung (P-Cesti und P. Piccardi, Eur. Pat. Anm. EP 195 717; 1986, J.S. Nicholson, und J.G. Tantum, U.S. Patent 4 209 638, 1980) oder durch Hochleistungs-Flüssigkeitschromatographietechniken (vergl. G. Blaschke, Angew. Chem. 92, 14-25, 1980) erhalten werden. Jedoch haben diese Verfahren der Anwendung optisch aktiver Basen oder Enzyme (Schweineleber-Esterase) den allen diesen Verfahren eigenen Nachteil von hohen Materialkosten, Arbeitsaufwendigkeit und Ausrüstung für die Wiedergewinnung und Razemisierung des unerwünschten optischen Stereoisomers, nicht eingerechnet die Energie, welche für die Redestillation der Lösungsmittel nötig ist und die geringen Ausbeuten an kristallinen Verbindungen von hoher optischer Reinheit aus den Ausgangsflüssigkeiten. Die Beseitigung dieser Auflösungsschritte wird demnach in einer wesentlichen Einsparung an Materialkosten, Herstellungsaufwand und Ausrüstung resultieren.

Verfahren zur Synthese razemischer 2-Aryl-Alkansäuren, insbesondere von 2-Aryl-Propionsäuren und insbesondere von R, S-Ibuprofen sind bekannt; vergl. z.B. Tanonaka, T., et al., DE 3523082 A1, (1986), der Mikroorganismen verwendet ; JP-PSEN 40-7491 (1965); 47-18105, (1972); JP-OS 50-4040, (1975); DE 2404159 (1974); DE-1443429 (1968) durch J.S. Nicholson und S.S. Adams; DE 2614306 durch Bruzzese, T., et al., (1976); DE 2605650 durch Gay, A., (1976); DE 2545154 durch Heusser, J., (1976); und DE 2404160 durch Kogure, K., et a., (1974).

Überraschenderweise sind nur wenige Verfahren für eine stereospezifisch-chemische Synthese von 2-Aryl-Alkansäuren, insbesondere von 2-Aryl-Propionsäuren, bekannt. Piccolo et al. (J. Org. Chem. 50, 3945-3946, 1985) beschreibt eine stereospezifische Synthese durch die Alkylierung von Benzol oder Isobutylbenzol mit (S)-Methyl-2-[(chlorsulfonyl)-oxy] oder 2-(mesyloxy)-propionat in Gegenwart von Aluminiumchlorid, wobei (S)-Methyl-2-phenyl-propionat in guter chemischer Ausbeute (50-80%) und einer ausgezeichneten optischen Aktivität von > 97 %, bestimmt mittels Rotation durch Inversion der Konfiguration an den angreifenden Kohlenstoffatommen, entsteht. Die Reaktions-bedingungen sind sehr ähnlich denen, die in einigen Patenten (Jpn. Kokai Tokkyo Koho 5808045; Chem. Abstracts, 1983, 98; 143138 k; Jpn. Kokai Tokkyo Koho 7979246; Chem. Abstracts, 1980, 92, 6253 f) beschrieben werden, bei denen razemische Reagenzien verwendet wurden. Die Ausweitung dieser Art von Reaktionen auf andere aromatische Substrate, z.B. Toluol, Isobutylbenzol, Tetralin, Anisol, Naphthalin, 2-Methoxy-naphthalin sind beschrieben in Jpn. Kokai Tokkyo Koho 7971932; Chem. Abstracts 1979, 91, 20125 b; Jpn. Kokai Tokkyo Koho 78128327; Chem. Abstracts 1978; 89, 23975 y; Jpn. Kokai Tokkyo Koho 81145241; Chem. Abstracts 1982, 96, 68650 z; Jpn. Kokai Tokkyo Koho 78149945; Chem. Abstracts 1979; 90, 168303 h; Jpn. Tokkyo Koho 7844537; Chem. Abstracts 1978, 89, 108693 h; Jpn. Kokai Tokkyo 77131551; Chem. Abstracts 1978, 88, 104920h. In einer kürzlich erfolgten Veröffentlichung beschreiben Piccolo et al. (J. Org. Chem. 52, 10, 1987) eine Synthese, welche zu R-(-)-Ibuprofen führt, während Tsuchihashi et al. (Eur. Pat. Anm. EP 67 698, (1982); Chem. Abstracts 98, 178945 y, (1983) eine stereospezifische Synthese von R-(-)-Ibuprofen-methylester mit ausgzeichneten Ausbeuten von etwa 75,0 % und hoher optischer Reinheit (> 95%) beschreibt, und zwar im Gegensatz zu Piccolo et al. ( J. Org. Chem. 32, 10, 1987), welcher eine optische Reinheit von nur 15 % für das R-(-)-Ibuprofen erzielt. Jedoch haben die gleichen Autoren chemische Ausbeuten von 68 % an S-(+)-Ibuprofen mit einer optischen Reinheit von nur 75 - 78 % beschrieben. Hayashi et al. (J. Org. Chem. 48, 2195, 1983; in: Asymmetric Reactions and Processes In Chemistry; eds. E.L. Eliel und S. Otsuka, ACS-Symposium Ser. 1985, 1982, 177) beschreiben eine stereospezifische Synthese von S-(+)-Ibuprofen durch eine asymmetrische Grignard-Kreuzkopplung, welche durch chirale Phosphin-Nickel- und Phosphin-Palladium-Komplexe katalysiert wird. Der enantiomere Überschuß der Kopplungsprodukte mit verschiedenen Alkenylhalogeniden unter Einfluß der oben erwähnten Metallphosphin-Komplexe, einschl. von Aminosäuren, hängt stark vom Liganden ab und reicht bis zu 94 % mit enantiomeren Überschüssen im Bereich von 60 - 70 %. Ein sehr nützlicher Ligand wurde mit den chiralen 2-Aminoalkylphosphinen gefunden, wodurch vernünftige chemische Ausbeuten und eine hohe optische Reinheit erreicht wurde. Weiterhin wurden optisch aktive 2-Aryl-Alkonate über eine Friedel-Crafts-Synthese durch Sato und Murai (Jpn. Kokai Tokkyo Koho JP 61 210 049 t 86 210 049, 1986) synthetisiert, wobei eine Ausbeute von 46 % S-(+)-Ibuprofen erhalten wurde. Giordano et al. (EP Anm. 0 158 913, 1985) beschrieben ein Verfahren zur Herstellung optisch aktiver 2-Aryl-Alkansäuren und deren Zwischenstufen durch Halogenierung am aliphatischen Kohlenstoffatom an die Ketalgruppe und Umlagerungen der Haloketale, wobei pharmakologisch aktive 2-Aryl-Alkansäuren entstehen. Eine stereochemische Synthese von 2-Aryl-Propionsäuren wird von Robertson et al. (EP Anm. 0 203 215 A2, 1986) beschrieben, wobei 2-(R₁)-Alkan als Kohlenstoffquelle für den Pilz Cordyceps, insbesondere für den Pilz Cordiceps militaris, verwendet wird und enantiomere S-(+) Produkte von hoher optischer Reinheit entstehen.

Journal of Org. Chemistry, Band 38, Nr. 10, 1973, Seiten 1870-1877; S. Yamagughi et al. beschreiben die Stereoselektivität bei Reduktionen von Carbonylverbindungen durch ein chirales Reagenz, das durch Zugabe eines Aminoalkohols zu Lithiumaluminiumhydrid in Ether hergestellt wurde; die Stereoselektivität beträgt ca. 40 - 75 %. Die Verwendung von Molekularsieben wird nicht erwähnt.

Journal of the American Chemical Society, Band 101, Nr. 11, 23.5.1979, Seiten 3129-3131; R. Noyori et al. beschreibt die Bildung eines Lithiumaluminiumhydridkomplexes zur asymetrischen Reduktion von Carbonylverbindungen. Die Verwendung von Molekularsieben wird nicht erwähnt.

Journal of the Amercian Chemical Society, Band 109, Nr. 18, 2.9.1987, Seiten 5551-5553; E.J. Corey et al. beschreibt die durch chirale Oxazaborolidine katalysierte Boranreduktion von Ketonen mit hoher Enantioselektivität. Die Verwendung von Molekularsieben wird nicht erwähnt.

Die Synthese von α-Hydroxy Estern, welche als chirale Reagentien fungieren um eine stereospezifische Synthese von 2-Aryl-propionsäuren durchzuführen ist von Larsen et al in J. Amer. Chem. Soc. (1989), 11, 7650-7651 beschrieben worden.

In diesem Falle wird eine asymmetrische Transformation von razemischen 2-Aryl-propionsäuren entweder zu den entsprechenden S- oder R-Enantiomeren in Gegenwart eines tertiären Amines durch chirale Alkohole, wie z. B. (S)-Ethyl-Laktat, R-Isobutyl-Laktat oder den -Hydroxy-Laktonen über Aryl-methyl-ketonen als Zwischenstufe mit hoher Enantioselektivität durchgeführt.

Methoden zur Synthese von entzündungshemmenden 2-Aryl-Propionsäuren sind ebenfalls im Übersichtsartikel von Rieu et al. (J. P. Rieu, A. Boucherle, H. Coussee und G. Mouzin, Tetrahedron Report No. 205, 4095-4131, 1986) aufgelistet. Dieser Bericht beschäftigt sich jedoch mehr mit den Razematen als mit der Aufklärung der stereospezifischen chemischen Synthese von 2-Aryl-Propionsäuren.

Es ist eine Aufgabe dieser Erfindung, ein stereochemischspezifisches Verfahren zur Herstellung optisch aktiver 2-Aryl-Alkansäuren, insbesondere von 2-Aryl-Propionsäuren, von hoher optischer Reinheit zu schaffen.

Diese Aufgabe wird erfindungsgemäß dadurch gelöst, daß ein Verfahren bereitgestellt wird zur Herstellung einer pharmazeutisch aktiven Verbindung in stereospezifischer Form, ausgewählt aus der Gruppe von Verbindungen mit der Formel : 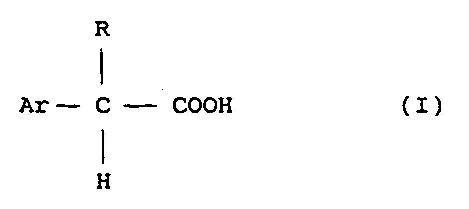
und deren physiologisch verträglichen Salzen und Estern, wobei R ein C₁ - C₉-Alkyl und Ar eine monozyklische, polyzyklische oder orthokondensierte polyzyklische aromatische Gruppe mit bis zu 12 Kohlenstoffen im aromatischen Ring ist, wobei der aromatische Ring substituiert oder nicht substituiert sein kann, bestehend aus den Schritten :
a) Umsetzen eines Carbonylsubstrats der Formel : 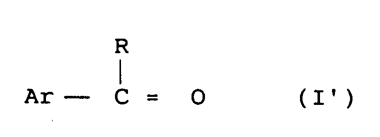 wobei R und Ar den oben gegebenen Bedeutungen entsprechen, mit einem stereospezifischen Reagenz in Gegenwart eines Reduktionsmittels, eines organischen Lösungsmittels und eines Molekularsiebs zum enantiomeren R- oder S-Karbinol und
b) Umsetzen des enantiomeren R- oder S-Karbinols mit SO₂X₂ oder SOX₂ oder Cyanurchlorid, wobei X = Cl oder Br ist, zum entsprechenden enantiomeren R- oder S-Halogenid, und Umsetzen des Halogenids mit
   (i) Magnesium in etherischer oder THF-Lösung, unter Beibehaltung der S- oder R-Konfiguration, zur entsprechenden R- oder S-metallorganischen Grignard-Verbindung und Durchströmen der die Grignard-Verbindung enthaltenden Lösung mit CO₂; oder
   (ii) einer Alkyl- oder organometallischen Verbindung in Gegenwart von CO₂; oder
   (iii)Natriumtetracarbonylferrat (II) (Na₂Fe(CO)₄) in Gegenwart von Triphenylphosphin (Ph₃P), wobei als Zwischenprodukt 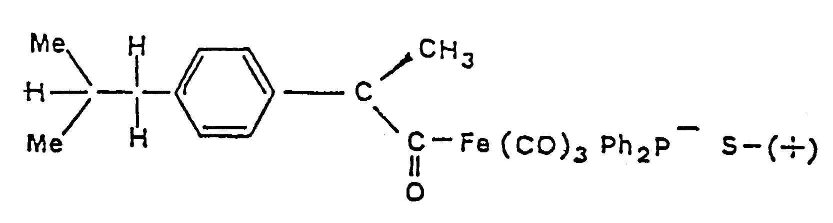 entsteht, welches anschließend mit Jod-Wasser oxidiert wird; oder
   (iv) Natriumtetracarbonylferrat (II) (Na₂Fe(CO)₄) in Gegenwart molarer Mengen von Triphenylphosphin (Ph₃P) und eines sekundären Amins zum entsprechenden Amid, das anschließend hydrolysiert wird; oder
   (v) (Na₂Fe(CO)₄) in Gegenwart von CO, wobei als Zwischenprodukt 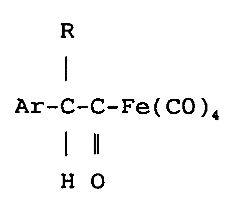 entsteht, das anschließend mit Sauerstoff oder NaOCl oxidiert und anschließend mit Säure hydrolysiert wird; oder
   (vi) Nickelcarbonyl (Ni(CO)₄) in Gegenwart eines Alkohols und seiner konjugierten Base gemäß der nachfolgenden Reaktion 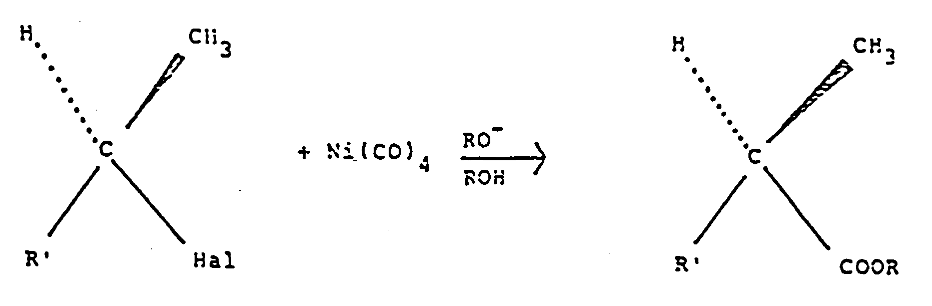 wobei
      Hal = Halogenid;
      ROH = Alkohol;
      R' = ein Arylrest, wie oben näher definiert; oder
   (vii) in Wasser gelöstem Alkalizyanid und Umsetzung des Produkts mit einer Base und Wasserstoffperoxid; oder
   (viii) Alkalizyanid mit nachfolgender Säurehydrolyse, um die entsprechende Säure (I) zu bilden.

Im folgenden wird das erfindungsgemäß bereitgestellte Verfahren beispielhaft an einigen ausgewählten, erfindungsgemäß einsetzbaren Verbindungen und Verfahrensschritten beschrieben. Es ist jedoch für den Durchschnittsfachmann ohne weiteres erkennbar, daß auch die in den Ansprüchen gekennzeichneten Verbindungen und Verfahrensschritte oder weitere, naheliegende Verbindungen oder Verfahrensschritte erfindungsgemäß einsetzbar sind.

Erfindungsgemäß ist R bevorzugt ein C₁ - C₄, insbesondere bevorzugt ein C₁-Alkyl und die aromatische Gruppe eine Phenyl-, Diphenyl- oder Naphthylgruppe , wobei die Substituenten dieser aromatischen Gruppen aus einem oder mehreren Halogenatomen, C₁ - C₄ Alkylen, Benzyl-, Hydroxy-, C₁ - C₂ Alkoxy-, Phenoxy- und Benzoyl-Gruppen bestehen können, daß Ar aus der Gruppe , bestehend aus
4-Isobutylphenyl
6-Methoxy-2-naphthyl
3-Phenoxy-phenyl
2'-Fluor-4-diphenyl
4'-Fluor-4-diphenyl
5-Chlor-6-methoxy-2-naphthyl
5-Brom-6-methoxy-2-napthyl
4-Chlor-phenyl
4-Difluor-methoxy-phenyl
6-Hydroxy-2-naphthyl und
5-Brom-6-hydroxy-2-naphthyl
ausgewählt wird, und bevorzugt,
daß das Carbonylsubstrat die Formel besitzt : 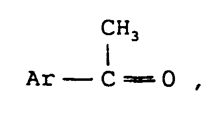
und bevorzugt,
daß das stereospezifische Reagenz (+)-4-Dimethylamino-3-methyl-1,2-diphenyl-2-butanol oder (1S,2S)-(+)-2-amino-1-phenyl-propan-1,3-diol oder (1R,2R)-(-)-2-amino-1-phenyl-propan-1,3-diol oder 2,2'-Dihydroxy-1,1'-dinaphthyl oder S-(-)-1,1-Diphenyl-Prolinol ist,
wobei für das stereospezifische Reagenz die Reduktionsmittel im Zusammenhang mit den chiralen Komplexmitteln ausgewählt werden aus der Gruppe, umfassend:
a) LiAlH₄, LiBH₄, NaBH₄ sowie KBH₄, wobei LiAlH₄ bevorzugt wird,
b) NaAlH₄, AlH₃.THF, Mg(AlH₄)₂, BH₃.THF
c) Al(BH₄)₃.THF; Ca(BH₄)₂.THF; Sr(BH₄)₂.Et₂O; Ba(BH₄)₂-Et₂O(THF), wobei bevorzugt
   das stereospezifische Reagenz einen Komplex aus BH₃.THF und S-(-)-1,1-Diphenyl-Prolinol oder
   einen Komplex aus LiAlH₄ und (+)-4-Dimethylamino-3-methyl-1,2-diphenyl-2-butanol oder einen Komplex aus LiAlH₄ und 2,2'-Dihydroxy-1,1'-dinaphthyl bildet.

Das organische Lösungsmittel ist Tetrahydrofuran (THF) oder 1,2-Dimethoxyethan oder ein Ether, bevorzugt ein Diethylether und das stereospezifische Reagenz ist in Gegenwart von Molekularsieben aktiv,
wobei bevorzugt als Molekularsiebe Zeolithe verwendet werden, die bevorzugt einen bestimmten Porenkanal besitzen, der durch ein 12 Ring, 10 Ring oder 8 Ring begrenzt wird, wobei die Molekularsiebe eine große Kapazität für Wasser und Sauerstoff haben,
sodaß bevorzugt die Adsorptionskapazität für Wasser zwischen 10 - 20 cm³/100 g aktivierter Molekularsiebe und die Adsorptionskapazität für Sauerstoff vorzugsweise bei 10 - 14 cm³/100 g aktivierter Molekularsiebe liegt, wobei weiterhin bevorzugt als Molekularsieb ein ZSM-5 Zeolith eingesetzt wird und bevorzugt das Verhältnis von Molekularsieben zur stereospezifischen Katalysatormenge zwischen 1 - 15 g pro mmol, bevorzugt zwischen 2 - 10 g pro mmol, und weiterhin bevorzugt 2 g pro mmol, und insbesondere bevorzugt,
daß das Verhältnis von ZSM-5 Zeolith zur stereospezifischen Katalysatormenge 5 g pro mmol oder 10 g pro mmol beträgt.

Bevorzugt wird das Carbonylsubstrat nach der Bildung des LiAlH₄-Komplexes zugegeben und das stereospezifische Reagenz mit dem Lithiumaluminiumhydrid liegt in der organischen Lösung als Komplex vor und zur Bildung des LiAlH₄-Komplexes werden bevorzugt zu einem organischen Lösungsmittel unter Rühren ein stereospezifisches Reagenz und LiAlH₄ zugegeben, wobei die Bildung des LiAlH₄-Komplexes bevorzugt in Gegenwart von Molekularsieben erfolgt.

Bevorzugt werden zur Bildung des LiAlH₄-Komplexes zu 10 bis 100 ml eines organischen Lösungsmittels 1,0 bis 100 ml eines stereospezifischen Reagenz und 0,5 bis 15 g, bevorzugt 0,5 bis 10 g, insbesondere bevorzugt 0,5 bis 5 g eines Molekularsiebes zugegeben und bei -60 bis 22°C gelöst werden und zu dieser Lösung bei -60 bis 22°C unter stetigem Rühren 10 bis 100 ml einer 1,0 bis 2,0 M Lösung von LiAlH₄ hinzugegeben, wobei bei einer Geschwindigkeit von 10 - 20 rpm solange weiter gerührt wird, bis sich eine homogene Suspension bildet, wobei bevorzugt als Reduktionsmittel zur Komplexbildung eine der in den Ansprüchen gekennzeichneten Verbindungen verwendet wird und die homogene Suspension bei einer Temperatur von -60 bis 22°C5 bis 100 Min. refluxiert wird.

Erfindungsgemäß wird bevorzugt, daß der LiAlH₄-Komplex nach seiner Bildung 5 bis 100 Min. bei einer Temperatur von 0 bis - 60°C stehengelassen oder 5 bis 100 Min. gerührt wird, um bei der Umsetzung des Carbonylsubstrats die R-(-)-Form des Carbinols zu erhalten, oder
daß der LiAlH₄-Komplex nach seiner Bildung 10 bis 100 Min. bei einer Temperatur von 0 bis 22°C stehengelassen oder 10 bis 100 Min. gerührt wird, um die S-(+)-Form des Carbinols zu erhalten, oder
daß der LiAlH₄-Komplex 8 Stunden lang in etherischen oder THF-Lösungen bei Temperaturen zwischen -7°C bis 0°C stehengelassen wird, bevor das Carbonylsubstrat zugegeben wird, um das S-Enantiomer des Carbinols zu erhalten,
oder daß die Bildung des LiAlH₄-Komplexes bei einer Temperatur von 0°C bis -60°C erfolgt und 0 bis 10 Min. nach Bildung des Komplexes das Carbonylsubstrat zugegeben wird, um das R-Enantiomer des Carbinols zu erhalten, wobei bevorzugt die Umsetzung des Carbonylsubstrats in Abwesenheit von Sauerstoff und Wasser durchgeführt wird.

Bevorzugt wird, wenn das Molverhältnis von LiAlH₄ zum stereospezifischen Reagenz zwischen 1,0 : 0,2 bis 1,0 : 3,0 liegt, bevorzugt daß das Molverhältnis von LiAlH₄ zum stereospezifischen Reagenz zwischen 1,0 : 2,3 bis 1,0 : 2,5 liegt, weiterhin bevorzugt,daß das Molverhältnis des LiAlH₄-Komplexes zum Carbonylsubstrat 1,0 : 0,2 - 1,0 : 2,5 beträgt, ebenfalls bevorzugt
daß das Molverhältnis des LiAlH₄-Komplexes zum Carbonylsubstrat 1,0 : 2,5 beträgt, oder
daß das Molverhältnis von LiAlH₄ zum stereospezifischen Reagenz 1,0 zu 0,7 beträgt, oder
daß das Molverhältnis von LiAlH₄ zum stereospezifischen Reagenz 1,0 zu 1,0 beträgt, oder
daß das Molverhältnis von LiAlH₄ zum stereospezifischen Reagenz 1,0 zu 2,0 beträgt, oder
daß das Molverhältnis von LiAlH₄ zum stereospezifischen Reagenz 1,0 zu 2,5 beträgt, oder
daß das Molverhältnis von LiAlH₄ zum stereospezifischen Reagenz 1,0 zu 3,0 beträgt, und daß vorzugsweise
als organisches Lösungsmittel Benzol oder Toluol oder Pentan oder Hexan verwendet wird, wenn Methoxy- und/oder Chlor- und/oder Brom- und/oder Fluor-Substituenten in den Arylgruppen des Carbonylsubstrats lokalisiert sind.

Im erfindungsgemäßen Verfahren, werden
a) das erhaltene enantiomere R- oder S-Carbinol einer stereospezifischen Halogenierung unterworfen, um das enantiomere R- oder S-Halogenid zu erhalten, und
b) dieses Halogenid wird durch Metallierung unter Beibehaltung der Konfiguration zur Metall-organischen Verbindung und
c) diese Metall-organische Verbindung durch Carbonisierung unter Beibehaltung der Konfiguration zum Endprodukt umgesetzt.

Weiterhin bevorzugt ist, daß die optische Reinheit / Aktivität des Halogenids wenigstens 80 % beträgt, bevorzugt, daß das S-Carbinol mit SO₂Cl₂, SO₂Br₂, SOBr₂, SOCl₂ oder Cyanurchlorid zum S-Halogenid umgewandelt wird, welches zu wenigstens 95 - 98 Gew.-% die S-Konfiguration des Halogenids aufweist, oder daß das R-Carbinol durch SO₂Cl₂, SO₂Br₂, SOCl₂, SOBr₂ oder Cyanurchlorid zum R-Halogenid umgewandelt wird, welches zu wenigstens 95 -98 Gew.-% die R-Konfiguration des Halogenids aufweist, oder daß das R- oder S-Carbinol durch SO₂Cl₂ oder SO₂Br₂ oder SOCl₂ oder SOBr₂ in Gegenwart von Pyridin und H₂O zum S-oder R-Halogenid umgewandelt wird, wobei wenigstens 95 - 98 Gew.-% der Halogenide in S- oder R-Konfiguration als Ausbeute erhalten werden.

Bevorzugt wird, daß die stereospezifische Halogenierung in wasserfreiem 1,4-Dioxan oder in trockenem Pyridin in Gegenwart von Thionylchlorid oder Thionylbromid oder Cyanurchlorid durchgeführt wird, weiterhin bevorzugtdaß das enantiomere Carbinol in Gegenwart von pulverisiertem Cyanurchlorid (1 Mol) oder in Gegenwart einer Base auf 10°C - 20°C über den Siedepunkt der Carbinole erhitzt wird, und nach 1 bis 1,5 Stunden dieses Reaktionsgemisch abgekühlt, gefiltert und unter Hochvakuum destilliert wird, worauf die Metallierung und Carbonisierung erfolgen, weiterhin bevorzugt daß die stereospezifische Halogenierung mit entsprechendem molarem Thionylchlorid oder Thionylbromid bei einer Temperatur von -10 bis -20°C, bevorzugt -20°C durchgeführt wird, wobei anschließend die Metallierung und die Carbonisierung erfolgen.

Weiterhin bevorzugt ist, daß
a) das R- oder S-Halogenid mit Magnesium in etherischen oder THF-Lösungen bei einer Temperatur von 4°C bis 15°C unter Beibehaltung der S- bzw. R-Konfiguration zur entsprechenden R- oder S-Metall-organischen-Grignard-Verbindung umgesetzt wird, und
b) durch diese die Grignard-Verbindung enthaltende Lösung CO₂ geleitet wird, um das Endprodukt zu erhalten,
wobei bevorzugt die S-Halogenide zu den organischen S-Magnesiumhalogeniden umgewandelt werden, welche zu wenigstens 90 Gew.-% die S-Konfiguration der organischen Magnesiumhalogenide aufweisen, weiterhin bevorzugt, daß die R-Halogenide zu den organischen R-Magnesiumhalogeniden umgewandelt werden, welche zu wenigstens 90 Gew.-% die R-Konfiguration der organischen Magnesiumhalogenide aufweisen, weiterhin bevorzugt, daß die erfindungsgemäßen S- oder R-Enantiomere dadurch erhalten werden, daß Kohlendioxid durch eine Lösung aus S- oder R-Magnesiumhalogeniden geleitet wird, wobei S- oder R-Carbonsäuren entstehen, welche zu wenigstens 95 - 98 Gew.-% die S- oder R-Konfiguration der Carbonsäuren aufweisen.

Es ist weiterhin bevorzugt, daß die Metallierung mit Methyllithium durchgeführt wird, oder daß die Metallierung mit Quecksilberverbindungen durchgeführt wird, und bevorzugt, daß als Quecksilberverbindungen HgCl₂, HgBr₂, Hg(CN)₂ oder Hg(SCN)₂ verwendet werden, weiterhin bevorzugt, daß die R-Halogenide zu den organischen R-Quecksilberhalogeniden umgewandelt werden, welche zu wenigstens 95 - 98 Gew.-% die R-Konfiguration der organischen Quecksilberhalogenide aufweisen, ebenfalls bevorzugt, daß die S-Halogenide zu den organischen S-Quecksilberhalogeniden umgewandelt werden, welche zu wenigstens 95 - 98 Gew.-% die S-Konfiguration der organischen Quecksilberhalogenide aufweisen, oder daß die S- oder R-Halogenide zu den S- oder R-quecksilberorganischen Halogeniden umgewandelt werden, welche zu wenigstens 95 Gew.-% die S- oder R-Konfiguration der quecksilberorganischen Halogenide aufweisen. Erfindungsgemäß können die enantiomeren R- oder S-Halogenide mit Natriumtetracarbonylferrat (II) (Na₂Fe(CO)₄) in Gegenwart von Triphenylphosphin (Ph₃P) umgesetzt werden, wobei als Zwischenprodukt 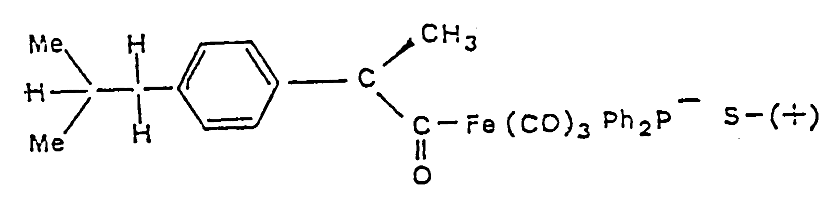
entsteht,
welches durch Oxidation mit Jod-Wasser zur entsprechenden Säure umgewandelt wird; weiterhin können erfindungsgemäß die enatiomeren R- oder S-Halogenide mit Natriumtetracarbonylferrat (II) (Na₂Fe(CO)₄) in Gegenwart von molaren Mengen an Triphenylphosphin (Ph₃P) und einem sekundären Amin zum entsprechenden Amid umgewandelt werden; weiterhin bevorzugt, wird das Natriumtetracarbonylferrat (II) durch die Behandlung von Fe(CO)₅ mit Natriumamalgam (NaHg) in THF hergestellt wird, wobei bevorzugt als sekundäre Amine niedere n-Dialkylamine mit Alkylresten von C₁ - C₆ verwendet werden und insbesondere bevorzugt, werden als sekundäre Amine Dimethylamin, Diethylamin oder Dibutylamin verwendet.

Erfindungsgemäß können die enantiomeren R- oder S-Halogenide mit Na₂ Fe(CO)₄ in Gegenwart von CO umgesetzt werden, wobei als Zwischenprodukt 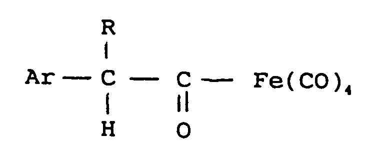
entsteht,
welches mit Sauerstoff oder NaOCl und anschließender saurer Hydrolyse zur entsprechenden enantiomeren Säure umgesetzt wird, oder
die enantiomeren R- oder S-Halogenide können mit Nickelcarbonyl (Ni (CO)₄) in Gegenwart eines Alkohols und seiner konjugierten Base gemäß der Reaktion 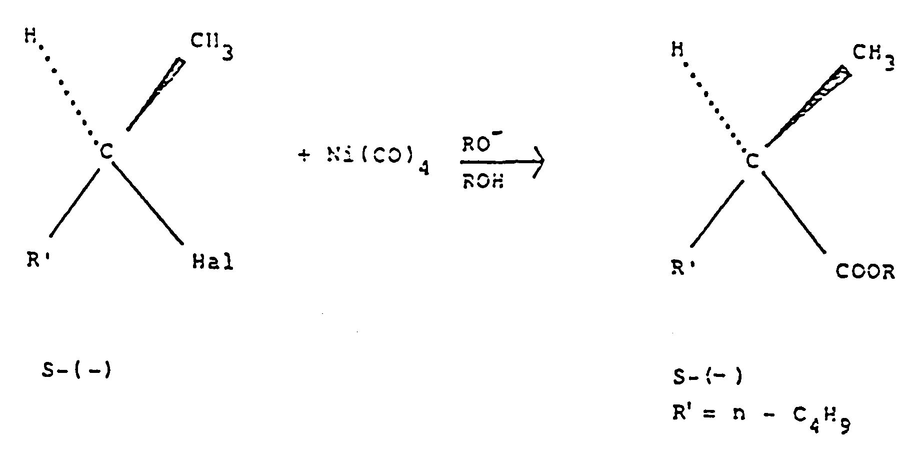
umgewandelt werden, wobei
Halogenid = Br, Cl, J
ROH = Butanol
R' = Aryl.

Bevorzugt wird, daß das R-Halogenid zur entsprechenden S-Carbonsäure umgewandelt wird, welche zu wenigstens 95 - 98 Gew.% die S-Konfiguration der Carbonsäure aufweist, bevorzugt,daß das R-Halogenid mit Natriumcyanid oder Kaliumcyanid, gelöst in Wasser, zum entsprechenden S-Cyanid und weiter mit NaOH und H₂O₂ zur entsprechenden S-Carbonsäure umgesetzt wird, weiterhin bevorzugt, daß das S-Halogenid mit Natriumcyanid oder Kaliumcyanid, gelöst in Wasser, zum entsprechenden R-Cyanid und weiter mit NaOH und H₂O₂ zur entsprechenden R-Carbonsäure umgesetzt wird, weiterhin bevorzugt, daß das S-Halogenid durch Quecksilber-(II)-Cyanid zum entsprechenden S-Quecksilbercyanid umgesetzt wird, welches zu wenigstens 95 Gew.% die S-Konfiguration des Quecksilber-organischen Cyanids aufweist, ebenfalls bevorzugt, daS die optisch aktiven R-Carbinole in Gegenwart von Quecksilber-(II)-Cyanid zu den entsprechenden S-Quecksilber-organischen Cyaniden umgesetzt werden, welche zu wenigstens 95 Gew.% die S-Konfiguration der organischen Quecksilbercyanide aufweisen.

Insbesondere bevorzugt wird, daß das Endprodukt Ibuprofen oder Naproxen ist.

Weiterhin ist bevorzugt, daß die Salze Komplexe aus 2-Aryl-alkansäuren und D-Glukamin im stöchiometrischen Verhältnis von 1 : 1 sind, wobei sie zu wenigstens 70 - 95 Gew.-% , insbesondere bevorzugt zu wenigstens 90 Gew.-%, die S-Konfigurationen des Komplexes aufweisen, oder ebenfalls bevorzugt, daß die Salze Komplexe aus 2-Aryl-alkansäuren und D-Ribamin im stöchiometrischen Verhältnis von 1 : 1 sind, wobei sie zu wenigstens 70 - 98 Gew.-%, insbesondere bevorzugt zu wenigstens 90 Gew.-%,die S-Konfiguration des Komplexes aufweisen, oder ebenfalls bevorzugt, daß die Salze Komplexe aus 2-Aryl-alkansäuren und kationischen Detergentien mit einer Kettenlänge von C₁₄ - C₁₆ sind, oder ebenfalls bevorzugt, daß die Salze Komplexe aus 2-Aryl-alkansäuren und Hexadecylpyridinium im stöchiometrischen Verhältnis 1 : 1 sind, wobei sie zu wenigstens 70 - 98 Gew.-% die S-Konfiguration des Komplexes aufweisen, oder ebenfalls bevorzugt, daß die Salze Komplexe aus 2-Aryl-alkansäuren und Benzethonium im stöchiometrischen Verhältnis von 1 : 1 sind, wobei sie zu wenigstens 70 - 98 Gew.-% die S-Konfiguration des Komplexes aufweisen.

Weiterhin wird bevorzugt, daß die Salze antimicrobielle Aktivitäten aufweisen.

Bevorzugt ist, daß die Komplexe Oberflächenaktivitäten mit einer Kritischen Mizellenkonzentration (KMK) von 1 x 10⁻² M/l bis 1 x 10⁻⁴ M/l aufweisen, bevorzugt, daß die 1 : 1 Komplexe aus S-(+)-Ibuprofen und R-Lysin bestehen, wobei die optische Reinheit des S-(+)-Ibuprofen 94 - 98 % beträgt, weiterhin bevorzugt, daß die 1 : 1 Komplexe aus S-(+)-Naproxen und R-Lysin bestehen, wobei die optische Reinheit des S-(+)-Naproxen 94 - 98 % beträgt, weiterhin bevorzugt, daß die 1 : 1 Komplexe aus S-(+)-Ibuprofen und R-Arginin bestehen, wobei die optische Reinheit des S-(+)-Ibuprofen 94 - 98 % beträgt, weiterhin bevorzugt, daß die 1 : 1 Komplexe aus S-(+)-Naproxen und R-Arginin bestehen, wobei die optische Reinheit des S-(+)-Naproxen 96 - 98 % beträgt, weiterhin bevorzugt, daß die 1 : 1 Komplexe aus S-(+)-Ibuprofen und N-Methyl-2-D-Glucosamin bestehen, wobei die optische Reinheit des S-(+)-Ibuprofens 94 - 98 % beträgt, weiterhin bevorzugt, daß die 1 : 1 Komplexe aus S-(+)-Naproxen und N-Methyl-α-D-Glukosamin bestehen, weiterhin bevorzugt, daß die 1 : 1 Komplexe aus S-(+)-Ibuprofen und N-Methyl-α-D-Galaktosamin bestehen, weiterhin bevorzugt, daß die 1 : 1 Komplexe aus S-(+)-Naproxen und N-Methyl-α-D-Galaktosamin bestehen, weiterhin bevorzugt, daß die 1 : 1 Komplexe aus S-(+)-Ibuprofen und Cholin bestehen, weiterhin bevorzugt, daß die 1 : 1 Komplexe aus S-(+)-Naproxen und Cholin bestehen, wobei vorzugsweise als anorganische Salze insbesondere Alkali- und Erd-alkali-Salze des S-Ibuprofen und S-Naproxen verwendet werden, weiterhin bevorzugt die Magnesiumsalze des S-(+)-Ibuprofen und S-(+)-Naproxen verwendet werden und bevorzugt die Natriumsalze des S-(+)-Ibuprofen und S-(+)-Naproxen in Gegenwart von 10 % (w/w) von Natriumkarbonat verwendet werden.

Weiterhin wird erfindungsgemäß bevorzugt, daß die hergestellten Erzeugnisse zur Herstellung von pharmazeutischen Zubereitungen mit antiinflammatorischen und antipyretischen Aktivitäten verwendet werden.

Die vorliegende Erfindung beschreibt die stereospezifische Synthese von S- oder R-Enantiomeren von 2-Aryl-Alkansäuren, bevorzugt von 2-Aryl-Propionsäuren, welche leicht in chemischen Anlagen vorgenommen werden kann. Der Vorteil dieses Verfahrens ist die Verwendung von einfach erhältlichen und ökonomischen Chemikalien, z.B. Ketonen, und die asymmetrische Reduktion mit chiralen Reagenzien aus Lithiumaluminiumhydrid-Komplexen mit (2S, 3R)-4-Dimethylamino-3-methyl-1,2-diphenyl-2-butanol oder 2,2′-Dihydroxy-1,1′-dinaphtyl [welches nach der Reaktion wieder verwendet werden kann], Thionylchlorid (Bromid), Magnesium oder Cyanid in Verbindung mit Natriumcarbonylferrat (Na₂Fe(CO)₄, Collman's Reagenz), um ökonomische Ausbeuten an S-(+) oder R-(-)-2-Aryl-Alkansäuren, bevorzugt S-(+)-Ibuprofen und S-(+)-Naproxen von hoher optischer Reinheit (> 98 %) herzustellen.

Ausgehend von asymmetrischen Ketonen, hergestellt z.B. durch eine Friedel-Crafts-Reaktion, wird die stereospezifische Reduktion je zur S- oder R-enantiomeren Form des entsprechenden Carbinols durch ein komplexiertes Reduktionsmittel, bestehend z. B. aus Lithiumaluminiumhydrid und einem optisch reinen Diaminoalkohol, erreicht, wobei hohe chemische Ausbeuten erzielt werden und das Enantiomer (R oder S) eine hohe optische Reinheit besitzt. Die nachfolgenden chemischen Schritte bei der chemischen Synthese beinhalten eine Halogenierung unter Erhaltung der Retention der chiralen Konfiguration in einer beinahe quantitativen Reaktion. Die Erfindung betrifft ein chemisches Verfahren zur Herstellung optisch aktiver 2-Aryl-Alkansäuren, insbesondere von 2-Aryl-propionsäuren, mit hohen chemischen Ausbeuten und einer optischen Reinheit von >98% einschl. neuer Zwischenprodukte von ausgezeichneter optischer Ausbeute ( > 97 %, bestimmt durch Rotations- und NMR-Methoden ). Diese Erfindung betrifft insbesondere ein neues chemisches Verfahren zur Darstellung einer stereoselektiven Synthese eines chiralen Alkohols, einer chiralen Magnesium-oder einer quecksilberorganischen Verbindung, bestehend aus zwei Hauptschritten : eine stereoselektive Halogenierung eines chiralen Alkohols, gefolgt von einer Metallierung oder von einer Reaktion mit Alkalicyanid und anschließender Umwandlung unter Retention der Konfiguration zur Carbonsäure. Diese Erfindung betrifft insbesondere eine gesamt-enantiomer-selektive chemische Synthese, weil sie die Herstellung von beiden enantiomeren Formen, R und S, getrennt mit hoher chemischer Ausbeute und ausgezeichneter optischer Reinheit ermöglicht, und zwar nur durch die Veränderung des Lösungsmittels, der Temperatur oder durch Zugaben des "chiralden" Komplexes, ohne Razemisierung.

Es können drei verschiedene Wege eingeschlagen werden, um optisch reine 2-Aryl-Alkansäuren bei hohen chemischen Ausbeuten zu erhalten :
i) Die Halogenide werden mit Magnesium oder Quecksilber metalliert und anschließend mit Kohlendioxid behandelt;
ii) Herstellen der entsprechenden Nitrile durch Veränderung der Konfiguration der chiralen Kohlenstoffatome ( R → S, oder S → R) und anschließende Hydrolyse zu den 2-Aryl-Alkansäuren;
iii) Behandlung der enantiomeren Halogenide mit Natriumtetracarbonylferrat-II [Na₂Fe (CO)₄] in Gegenwart von Kohlenmonoxid (CO) und nachfolgende Behandlung mit Natriumhypochlorit (NaOCl) und saurer Hydrolyse oder Behandlung mit einem Halogen (z.B. J₂) in Gegenwart eines Alkohols, um den enantiomeren Ester zu ergeben, oder in Gegenwart von J₂ und Wasser, um die entsprechende freie Carbonsäure zu erhalten.

Diese chemischen Verfahren ergeben optisch hochreine 2-Aryl-Alkansäuren, insbesondere 2-Aryl-Propionsäuren, bei hohen chemischen Ausbeuten.

Die erfindungsgemäß hergestellten 2-Aryl-Alkansäuren fallen unter die folgende chemische Formel : 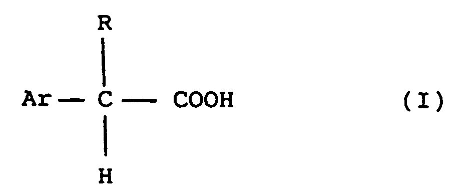
wobei R ein C₁-C₉-Alkyl, Ar bevorzugt eine monocyclische, polycyclische oder ortho-kondensierte polycyclische aromatische Gruppe mit bis zu 12 Kohlenstoffatomen im aromatischen System, z.B. Phenyl, Diphenyl und Naphthyl, ist. Die Substituenten dieser aromatischen Gruppen bestehen aus einem oder mehreren Halogenatomen, C₁-C₄ Alkylen, Benzyl-, Hydroxy-, C₁-C₂ Alkoxy-, Phenoxy- und Benzoyl-Gruppen. Beispiele für derart substituierte Aryle sind : 4-Isobutyl-phenyl, 3-Phenoxy-phenyl, 2-Fluor-4-diphenyl, 4′-Fluor-4-diphenyl, 6-Methoxy-2-naphthyl, 5-Chlor-6-methoxy-2-naphthyl und 5-Brom-6-methoxy-naphthyl, 4-Chlor-phenyl, 4-Difluor-methoxy-phenyl, 6-Hydroxy-2-naphthyl, und 5-Brom-6-hydroxy-2-naphthyl.

Die Bedeutung der nachfolgenden Bezeichnungen und Ausdrücke, die in dieser Patentanmeldung verwendet werden, werden zur Klarstellung wie folgt definiert:

Chiral bezeichnet eine chemische Struktur, welche wenigstens ein asymmetrisches Zentrum besitzt. Die Konfiguration des asymmetrischen Kohlenstoffatoms wird als "R" oder "S" in Übereinstimmung mit den Cahn-Ingold-Prelog-Regeln klassifiziert. Enantiomer oder Enantiomorph definiert ein Molekül, welches mit seinem jeweiligen Spiegelbild nicht zur Deckung gebracht werden kann. Enantiomerer Überschuß, "e.Ü." betrifft eine Definition mit der Bedeutung : Prozentgehalt des vorherrschenden Enantiomers, substrahiert vom anderen Enantiomer.

Die Ketone der unten angegebenen chemischen Formel (II) sind bekannt und sind mittels bekannter Verfahren durch Friedel-Crafts-Acylierung leicht herstellbar, falls sie nicht kommerziell erhältlich sind.

Die stereospezifische Reduktion zum entsprechenden S-(+)-2 (4-Isobutylphenyl)-2-hydroxy-ethan (III) wird durch die Umsetzung des asymmetrischen Ketons (II) mit LiAlH₄ im Komplex mit (+)-4-Dimethyl-3-methyl-1,2-diphenyl-2-butanol in etherischen (oder THF) Lösungen gebildet. Aufgrund verschiedener Bedingungen bei der Durchführung dieser spezifischen Reaktion, z.B. der Temperatur, Zugabe von reduzierenden Reagenzien und Reaktionszeit, kann leicht das S-2-(4-Isobutylphenyl)-2-hydroxy-ethan oder das entsprechende R-Enantiomer in guten chemischen Ausbeuten ( fast 100 % chemische Ausbeute) und hoher optischer Reinheit (> 95 %) erhalten werden.

Ein schematischer Weg für die erfindungsgemäße Synthese des S- oder R-Enantiomers ist unten in Fig. 1 gezeigt, während die Figuren 2 und 3 das Verfahren zur Darstellung von S-(+) oder R(-)-Ibuprofen zeigen. Fig. 1 steht für einen allgemeinen Weg, um reine Enantiomere der 2-Aryl-Propionsäuren zu erhalten, welche für industrielle Verfahren nutzbar sind.

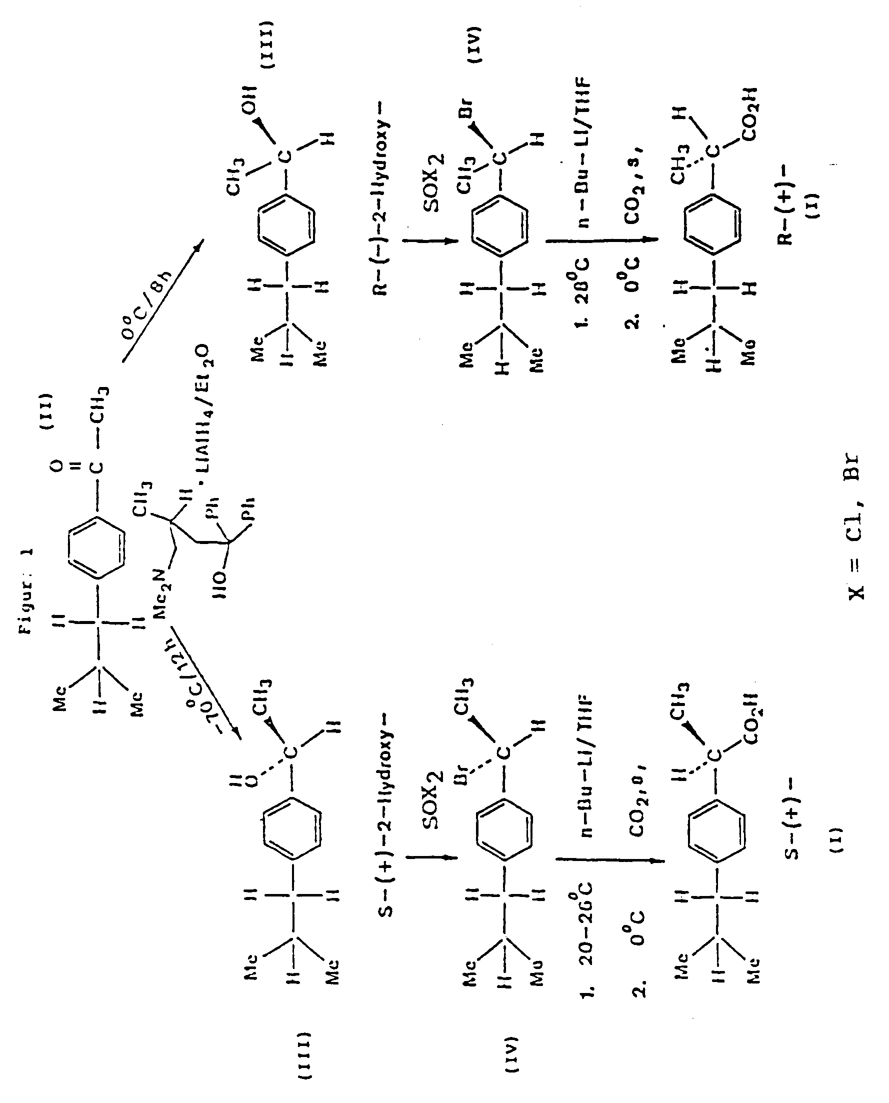

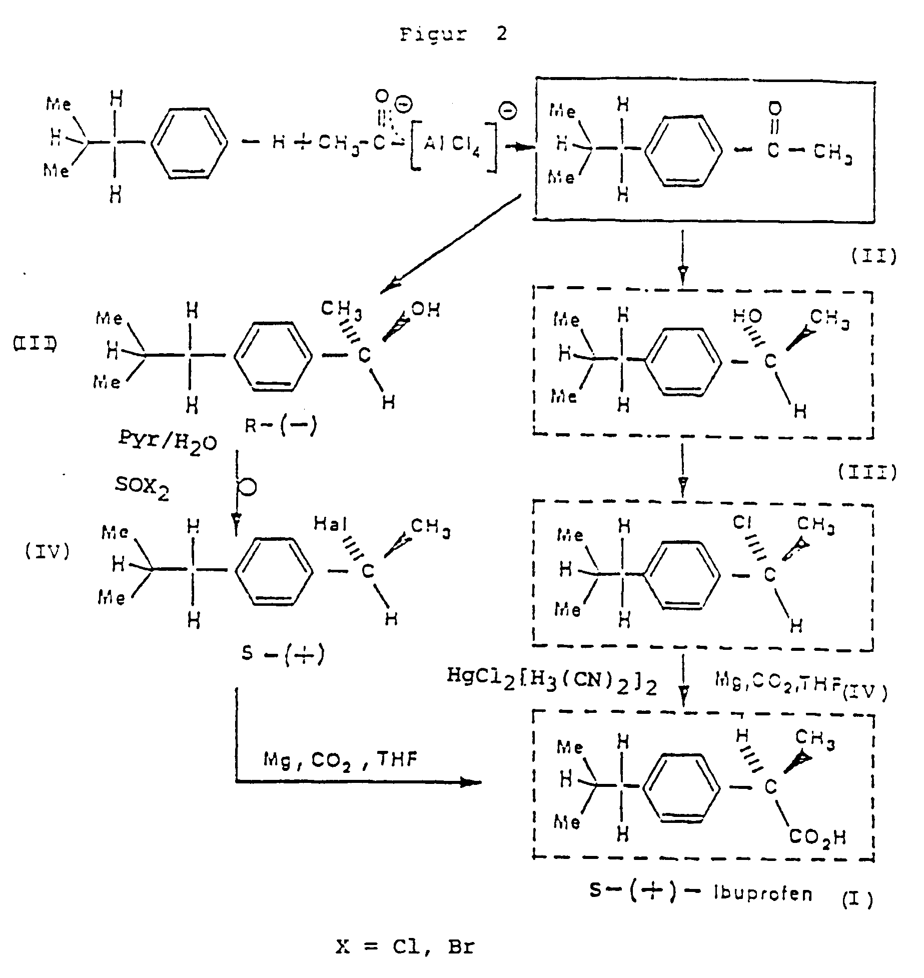

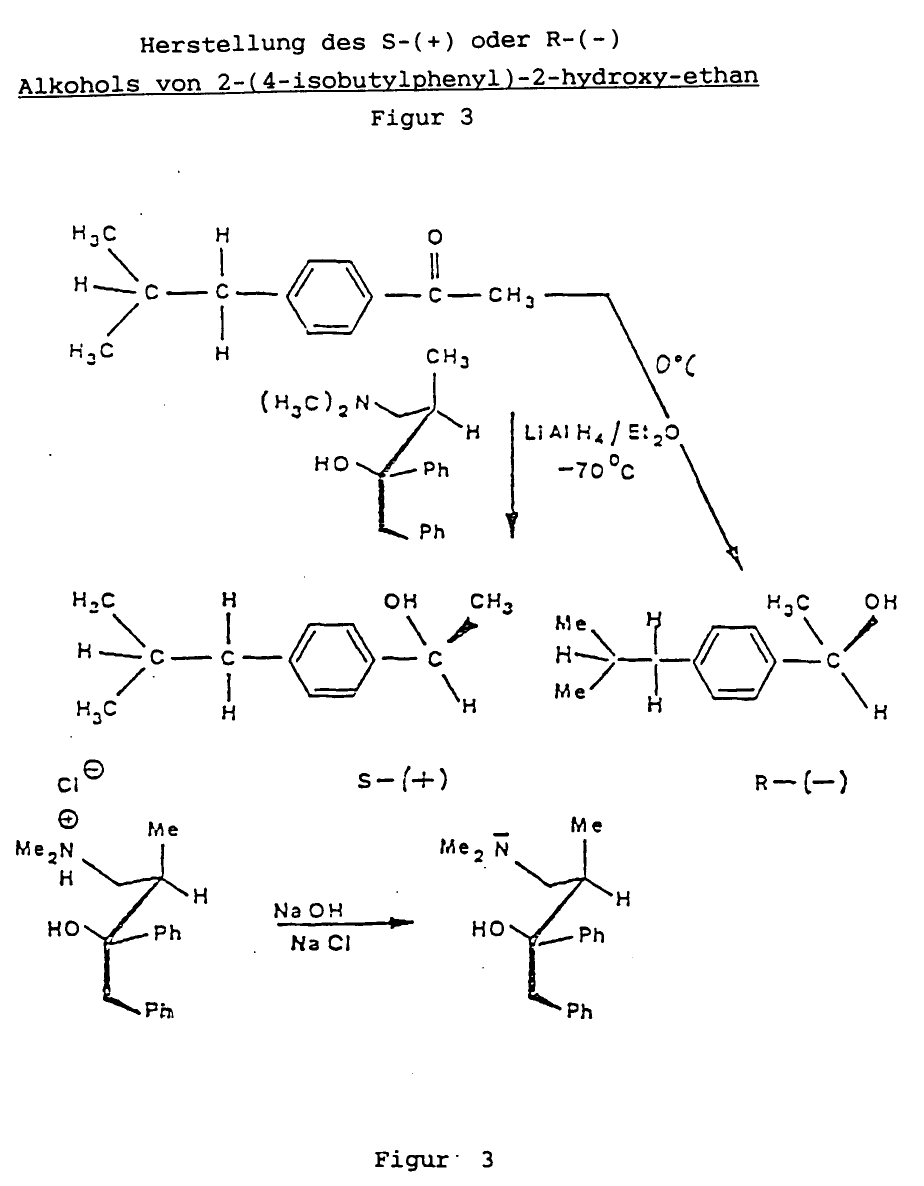

Die asymmetrische Reduktion mit Lithiumaluminiumhydrid (LiAlH₄) im Komplex mit (+)-(2S, 3R)-4-Dimethylamino-3-methyl-1,2-diphenyl-2-butanol (S. Yamaguchi und H.S. Mosher, J. Org. Chem. 38, 1870, 1973) des Ketons (II) ergibt entweder (R)-(-) oder (S)-(+)-2-(4-Isobutylphenyl)-2-hydroxy-ethan in einer enantiomeren Reinheit von 98 - 99 % , abhängig von der Verwendung dieses Reagenz sofort nach seiner Herstellung oder nach der Alterung über Nacht oder nach dem "refluxen" für wenige Minuten. Diese spezielle Umkehr in der Stereoselektivität mit der Alterung des komplexierenden Reagenz ("chiraldid reagent") kann verwendet werden, um 2-substituierte-2-Hydroxy-ethan-Derivate von hoher optischer Reinheit und sehr guten chemischen Ausbeuten von etwa 95 % und mehr bereitzustellen. Diese chemischen Ausbeuten von hoher optischer Reinheit können bevorzugt aus verschiedenen Carbonylsubstraten der Formel (I′) erhalten werden, 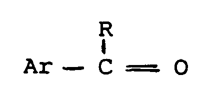
wobei R = 4-Isobutylphenyl
= 6-Methoxy-2-naphthyl
= 3-Phenoxy-phenyl
= 2′-Fluor-4-diphenyl
= 4′-Fluor-4-diphenyl
= 5-Chlor-6-methoxy-2-naphthyl
= 5-Brom-6-methoxy-2-naphthyl
= 4-Chlor-phenyl
= 4-Difluor-methoxy-phenyl
= 6-Hydroxy-2-naphthyl
= 5-Brom-6-hydroxy-2-naphthyl

Die Bestimmung der Stereoselektivität wurde mit Hilfe von NMR-Methoden durch die Behandlung des erhaltenen Carbinols mit einem Überschuß an Säurechlorid aus (R)-(+)-2-Methoxy-2-trifluormethylphenyl-essigsäure in Pyridin durchgeführt, beschrieben bei J.A. Dale, D.C. Dull, und H.S. Mosher, J. Org. Chem. 34, 2543, (1969). Die Signale je der O-Methyl und α-Methyl-Gruppen des R,R-Diastereomers aus Methylphenylcarbinol erscheinen bei höheren Feldern als diejenigen der R, S-Diastereomere. Die Signale (peaks) sind auf einem T-60-Instrument klar voneinander getrennt, und es wurde gezeigt, daß die relativen Signalhöhen eine gute Näherung der isomeren Zusammensetzung geben. Weiterhin können die ¹⁹F-Resonanzen für die 2 -CF₃-Gruppe bei 94,1 MHz ebenfalls verwendet und leicht integriert werden. Die Anwendung eines NMR-Verschiebungsreagenz, z.B. Fn (fod)₃(0,1M), kann ebenfalls dazu benutzt werden, zwischen den verschiedenen R, R-Diastereomeren und R, S-Diastereomeren zu unterscheiden, so daß eine quantitative Integration der jeweiligen O-Methylsignale leicht möglich ist.

Eine andere wirkungsvolle asymmetrische Reduktion der prochiralen Carbonyl-Verbindungen gemäß der Formel (II) mit einem Hydrid-Reagenz, enthaltend einen chiralen Hilfsliganden, kann erreicht werden durch die Verwendung von LiAlH₄ in Komplex mit optisch reinem 2,2′-Dihydroxy-1,1′-dinaphthyl in Gegenwart einer hydroxylen Verbindung R'OH. Die Enantio-Selektivität steht praktisch vollständig (vergl. Fig. 4 unten) in Übereinstimmung mit den jüngsten Untersuchungen von Noyari et al. (R. Noyari, I. Tomino und Y. Tanimoto, J. Amer. Chem. Soc. 101, 3129-3130, 1979).

Da je die R- und S-Formen der Carbinole in ihren optisch reinen Formen leicht zugänglich sind, ermöglichen beide Methoden die Synthese beider Enantiomere dieser Carbinole aus Carbonyl-Verbindungen. Weiterhin ergab die Reduktion von Einzel-Dialkylketonen keine zufriedenstellenden optischen Ausbeuten wie diese asymmetrisch-substituierten Ketone der Formel II.

Bezüglich der Reaktion von LiAlH₄-(+)-4-dimethylamino-3-methyl-1,2-diphenyl-2-butanol mit den Ketonen (II) wurde gefunden, daß die optische Ausbeute durch Absenkung der Reaktionstemperatur zunimmt, wenn Ether als Lösungsmittel verwendet wird. Jedoch ist bei Verwendung von Tetrahydrofuran (THF) oder 1,2-Dimethoxy-ethan die Reaktionstemperatur nicht kritisch, wenn die Reaktionstemperatur unterhalb von 30°C liegt. In beiden Fällen betragen die chemischen Ausbeuten fast 95 %.

Das Verfahren, um hohe chemische Ausbeuten an optisch reinen S- oder R-Enantiomeren des substituierten 2-Hydroxy-ethans zu erhalten, ist abhängig von der Synthese der R-(-) oder der S-Form aus dem Keton (II), einschließlich des anschließenden Syntheseweges zum S- oder R-Enantiomer der Carbonsäuren. Es wurde gefunden, daß die Einflüsse der Temperatur, der Konzentration des Lösungsmittels, der Zeit, des Verhältnisses der Reaktanten und der Rührgeschwindigkeit auf die Stereoselektivität dieser Reduktion für die Erreichung hoher chemischer Ausbeuten (> 90 %) an optisch reinen S- oder R-enantiomeren Formen von Bedeutung sind.

Von herausragender Bedeutung ist die beobachtete Zunahme der chemischen Ausbeute der entsprechenden Enantiomere (R oder S) von hoher optischer Reinheit gemäß dieser Reduktion zum Carbinol, wobei die Stereoselektivität stark abhängig von der Zeitdauer ist, die das Reagenz, z.B. LiAlH₄R*OH (R*OH ist (+)-(2S, 3R)-4-Dimethylamino-3-methyl-1,2-diphenyl-2-butanol), stehen gelassen wurde, bevor es zur Reduzierung des Ketons als Substrat verwendet wird. Dementsprechend sind die Rührgeschwindigkeit und die Zeit wichtige Faktoren bei der Herstellung der R- oder S-enantiomeren Formen mit hoher chemischer Ausbeute und optischer Reinheit, während der Zeitparameter die Stereoselektivität beeinflußt, wenn R-(-) oder S(+) aus dem Carbonylsubstrat hergestellt wird, und das Rühren (Mischen) in Abwesenheit jeglichen Sauerstoffs und jeglicher Feuchtigkeit hauptsächlich die chemische Ausbeute bestimmt. Erfindungsgemäß wird, wenn z.B. 2-(4-Isobutylphenyl)-methyl-keton oder 1-[4-2-Methylpropyl]-ethanon zum Reagenz hinzugefügt wird, z.B. LiAlH₄R*OH, bei 4°C, (0°C-4°C) entweder 1 Minute oder 5 Minuten nach seiner Herstellung, eine fast quantitative Ausbeute des R-(-)-2-(4-Isobutyl-phenyl)-2-hydroxy-ethan gebildet, wenn kontinuierlich gerührt wird. Die Herstellung des Reduktionsmittels wird durch das Mischen von LiAlH₄R*OH mit dem Keton in einem Molverhältnis von 1,0 : 2,5 durchgeführt, wobei normalerweise ein breiartiger Kuchen in etherischer Lösung bei 0 - 4 °C erhalten wird; jedoch muß ein kontinuierliches Rühren erfolgen, um ein aktives Reduktionsmittel zu erhalten.

Die Herstellung des Reduktionsmittels, z.B. LiAlH₄R*OH in etherischer Lösung, insbesondere der kolloidale Zustand und weniger der "kuchenförmige" oder suspendierte Zustand, beeinflußt die optische Reinheit; die fast quantitative Reduktion des Carbonylsubstrats, welches bestimmt wird durch das Rühren der Mischung aus LiAlH₄R*OH und dem Keton und dem kolloidalen Zustand des Reduktionsmittels LiAlH₄R*OH, entscheidet über die optische Reinheit des Reagenz, welche beinahe 95 - 98 % beträgt.

Wenn jedoch das Reduktionsmittel für 8 Stunden stehengelassen wird oder es in Ether refluxiert, ohne daß gerührt wird, bevor das gleiche Carbonylsubstrat hinzugegeben wird, wird eine 95 - 98% Ausbeute der S-(+)-enantiomeren Form des Carbinols, welches zu 95 % optisch rein ist, erhalten.

Die beobachtete Umkehr in der Stereoselektivität ist verbunden mit dem kolloidalen Zustand des LiAlH₄(R*OH)ₙ und der mehr löslichen Form von "LiAlH₄₋ₙ(R*OH)ₙ" + nH₂, immer noch im kolloidalen Zustand, jedoch mehr in einem Zustand der Mikroemulsion. Rühren (stetiges Mischen) für eine kurze Zeitdauer (Zeitskala in Minuten) ist wesentlich gemäß dem Reaktionsschema :

LiAlH₄ + nR*OH = LiAlHₙ₋₄ (OR*) + nH₂

was durch die Entwicklung von Wasserstoff und der Bildung einer Mikroemulsion, und weniger der eines Präzipitates, gemessen werden kann. Praktisch ist die Dichtekonstanz des Zustands von LiAlH₄₋ₙ (OR*) oder vom anfänglichen Komplex LiAlH₄₋ₙR*OH in etherischen oder THF-Lösungen eine vernünftige Voraussetzung für die Stereoselektivität, und jede Veränderung, die am kolloidalen System vorgenommen wird, beeinflußt eher die chemischen Ausbeuten als die optische Reinheit. In Übereinstimmung mit der Erfindung ist die erfolgreiche Herstellung des S-Enantiomers des Carbinols aus dem entsprechenden Keton (I oder I′) als ein Substrat bevorzugt, wobei das Reduktionsmittel 8 Stunden lang in etherischen oder THF-Lösungen bei Temperaturen zwischen -7°C -0°C stehengelassen wird, bevor das entsprechende Keton hinzugegeben wird. Die Reduktion wird durchgeführt und vervollständigt in 10 - 12 Stunden bei 20°C unter stetigem Mischen.

Das R-Enantiomer des Carbinols aus dem entsprechenden Keton (II) wird bevorzugt erhalten durch die Bildung des Komplex-Reduktionsmittels, LiAlH₄₋ₙ (OR*) bei 0°C ( bis zu -50°C) und sofortiger Zugabe (zwischen 5-10 Minuten) des entsprechenden Ketons in etherischen oder THF-Lösungen, gefolgt von stetigem Rühren über eine Zeitdauer von 8 - 12 Stunden bei 20°C.

Es wurde gefunden, daß die Stereoselektivität der Reduktion zum R-Carbinol mit abnehmender Temperatur (-70°C bis 0°C bei der Herstellung des aktiven Reduktionskomplexes zunimmt, während die Stereoselektivität des löslichen aktiven Reduktionsreagenz für das S-Carbinol mit der Temperatur abnimmt. Die Reaktionstemperaturen für die Bildung der aktiven Reaktionsspezies, z.B. LiAlH₄₋ₙ(OR*)ₙ und das Altern (Zeit für das Stehenlassen von LiAlH₄₋ₙ(R*)ₙ) ist für die Stereoselektivität wichtig. Jedoch sollte die Hauptdichte der Reaktionsmischung, bestehend aus LiAlH₄₋ₙ (OR*)ₙ und dem Keton bezüglich des industriellen Verfahrens über die gesamte Reihe an Ketonzugaben konstant sein. Wenn sich demnach die Volumenzuflußgeschwindigkeit des Ketons bezüglich des aktiven Reduktionsmittels im Fließgleichgewicht (steady state) befindet, wird die Ausflußgeschwindigkeit aus der Reaktion die gleiche sein wie die Volumenzuflußgeschwindigkeit des reagierenden Ketons. Technisch ausgedrückt kann sie als eine homogene Reaktionsmischung behandelt werden, weil ein vollständiges Vermischen auf molekularer Ebene ohne besondere Verweilzeit stattfindet. Wenn die Zufuhr aus einer Suspension kolloidaler Partikeln besteht, obwohl es eine Verteilung der Verweilzeiten unten den einzelnen Partikeln gibt, entspricht die mittlere Verweilzeit dem Verhältnis von Volumen zu Volumenzuflußgeschwindigkeit, wenn das System ideal gerührt und gemischt wird.

Die Reaktionskinetiken zur Umwandlung des Ketons zu den entsprechenden enantiomeren Carbinolen können dadurch erhöht werden, daß die Reaktion in Gegenwart von 3A oder 4A Molekularsieben (Zeolithen) während der Reaktion durchgeführt wird. Die Vorteile bei Verwendung dieser Siebe umfassen, wie bei der Anwendung dieses Verfahrens gefunden wurde, Ökonomie, leichte Isolierung, zunehmende chemische Ausbeuten einschließlich verbesserter optischer Reinheiten und enantiomerer Überschuß und die Möglichkeit für eine in situ-Derivatisierung des Produktes. Erfindungsgemäß wird die stereospezifische Reduktion der Ketone zu den entsprechenden enantiomeren Carbinolen in Abwesenheit von Wasser durchgeführt; Wasser (i), welches von unvollständig getrockneten Reagenzien, Lösungsmitteln, Ausrüstung und Feuchtigkeit herrührt, (ii) Diolether, erzeugt in situ während Nebenreaktionen in Gegenwart von Wasser, (iii) Hydroperoxide und (iv) ein ungeeigneter kolloidaler Zustand des reduzierenden chiralen Komplexes sind nachteilig, wenn keine Molekularsiebe während der Reaktion verwendet werden, wodurch eine Abnahme der chemischen Ausbeuten der entsprechenden, optisch reinen Carbinole bewirkt wird.

Es wurde gefunden, daß die höchsten Stereoselektivitäten für beide Formen des reaktiven Reduktionsmittels, LiAlH₄₋ₙ(OR*)ₙ, für Verhältnisse LiAlH₄ zu R*OH erhalten werden, die, in Übereinstimmung mit Yamaguchi und Mosher (J.Org. Chem. 38, 1870, 1973), zwischen 1,0 : 2,3 bis 1,0 : 2,5 liegen, und sie scheinen für die hier beschriebenen Verfahren nicht kritisch zu sein. Es wurde gefunden, daß die Herstellung des aktiven Reduktionsmittels, LiAlH₄₋ₙ (OR*)ₙ, in Benzol, Toluol, Pentan und Hexan anstelle von Ether, THF, 1-2-Dimethoxy-ethan, die Stereoselektivitäten über mehr polare Lösungsmittel, z.B. Ether usw., nicht verbessert. Jedoch sind nichtpolare Lösungsmittel sehr nützlich bei der Reduktion von Ketonen mit dem aktiven Reduktionsmittel, wenn Methoxy- und/oder Chlor-und/oder Brom- und/oder Fluor-Substituenten in den Arylgruppen lokalisiert sind, weil sie in diesen Lösungsmitteln nicht durch das Reduktionsmittel beeinflußt und demnach hohe chemische Ausbeuten mit hohen optischen Reinheiten erhalten werden. Ein Vorteil bei der Verwendung dieses chiralen Reduktionsmittels zur Erstellung enantiomerer Carbinole von hoher optischer Reinheit in großem Maßstab ist die Wiedergewinnung des (+)-4-Dimethylamino-3-methyl-1,2-diphenyl-2-butanol nach der Reaktion mit Salzsäure und anschließender Neutralisation mit Natriumcarbonat. Demnach kann der optisch aktive Diaminoalkohol wiederverwendet werden, wodurch dieser Reduktionsschritt bei niedrigen Kosten und fast quantitativen Ausbeuten sehr ökonomisch ist.

Das gleiche Verfahren kann bei Verwendung von 2,3′-Dihydroxy-1,1′-dinaphthyl als auch für die Wiedergewinnung dieses speziellen Reduktionsmittels angewandt werden (vergl. Fig. 4 unten).

Eine weitere, sehr wirtschaftliche Herstellung des chiralen Alkohols entsprechend des Reaktionschemas Fig. 3 besteht in der Reduktion des asymmetrischen Ketons, z. B. II, durch den Komplex bestehend aus Borhydrid und THF (Tetrahydrofuran) [BH₃:THF] und dem chiralen Amino-Alkohol S-(-)-1,1-Diphenyl-Prolinol (Corey et al. J. Amer. Chem. Soc., 109, 5551, 1987). Die stereospezifische Reduktion ist in Fig. 5a dargestellt. Die chemischen Ausbeuten dieser Reduktion liegen bei 95-99,7 %, der enantiomere Überschuß bei 97 %.

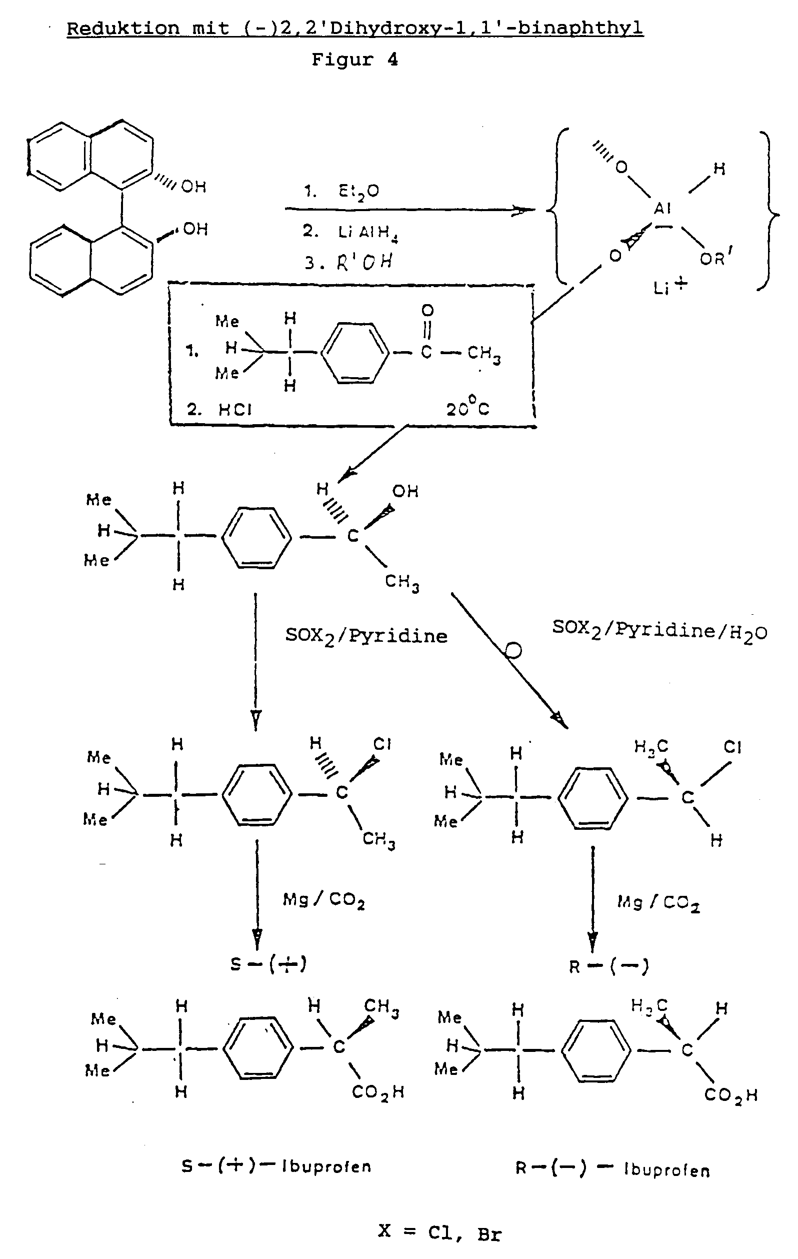

Die stereospezifische Halogenierung des enantiomeren Carbinols, R oder S, (III), unter Erhaltung der Retention der Konfiguration des chiralen Kohlenstoffs kann entweder mit Thionylchlorid oder Thionylbromid oder Cyanurchlorid bei hohen chemischen Ausbeuten (fast quantitativ) und hoher optischer Reinheit durchgeführt werden. Bevorzugt wird die Halogenierung in wasserfreiem 1,4-Dioxan durchgeführt, wenn große Mengen an Carbinolen verwendet werden, während auch trockenes Pyridin verwendet werden kann. Die enantiomeren Carbinole werden normalerweise in 1,4-Dioxan bei 20°C durch tropfenweise Zugabe der stöchiometrischen Mengen von Thionylchlorid unter stetigem Mischen über eine Zeitdauer von 1 Stunde gelöst. Die Reaktion sollte im Fall von Thionylchlorid oder -bromid für weitere 30 Minuten fortgeführt werden. Der Überschuß an SO₂Cl₂ oder SO₂Br₂ wird dadurch eliminiert, daß ein trockner Strom an Stickstoff durch die Reaktionslösung bei 20°C für etwa 5 Stunden geleitet wird, bis das R- oder S-enantiomere Chlorid durch Hochvakuum-Destillation wiedergewonnen wird.

Ein typisches Verfahren zur Herstellung des enantiomeren Chlorids beinhaltet das Erhitzen des enantiomeren Carbinols mit pulverisiertem Cyanurchlorid (1 Mol) auf 10 - 20°C über den Siedepunkt der Carbinole oder in Gegenwart einer Base (0,5 Mol NaOCH₃ oder NaO Bu). Nach der Zugabe (ca. 1- 1,5 Std.) wird das Reaktionsgemisch abgekühlt, gefiltert und unter Hochvakuum destilliert. Die Ergebnisse aus diesem Verfahren deuten darauf hin, daß keine Isomerisierung oder Razemisierung auftrat.

Das Verfahren zur Umwandlung der enantiomeren Carbinole zu den Chloriden oder Bromiden unter Verwendung von Thionylchlorid oder -bromid hat den Vorteil, daß die enantiomeren R- oder S-Halogenide für die Herstellung der Magnesium-organischen Verbindung (IV, Fig. 2) nicht mehr destilliert werden müssen und als letzer Schritt bei der Herstellung der S- oder R-enantiomeren-2-Propionsäuren (Fig. 2, 4) die Carbonierung erfolgt.

Zum Beispiel wurde durch die Verwendung eines optisch aktiven (+)-1-Brom-1-methyl-2,2-diphenylcyclopropans ein optisch aktives Grignard-Reagenz ((H.M. Walborsky und A.E. Young, J. Amer. Chem. Soc., 83, 2595 (1961)) und eine aktive organo-Lithium-Verbindung (H.M. Walborsky und F.J. Impastato J. Amer. Chem. Soc., 81, 5835 (1959)) hergestellt. Bezeichnenderweise kann die organo-Lithium-Verbindung karboniert werden unter 100 % Retention der optischen Aktivität und der Konfiguration.

Normalerweise stimmen die wissenschaftlichen Daten zur Bildung von Grignard-Verbindungen mit dem "D-Modell" überein, insbesondere für primäre Alkylhalogenide, was sich teilweise aus einem freien Diffundieren in der Lösung zu allen Zeiten ergibt (Garst et al., J. Amer. Chem. Soc., 1985, 108, 2490), woraus folgt, daß eine Razemisierung und ein geringer enantiomerer Überschuß erhalten werden. Jedoch kann dieser Schluß nicht einfach auf andere Substrate übertragen werden. Wir haben gefunden, daß die Reaktion des chiralen 2-S-(+)-Chlor- oder Brom-(4-isobutyl-phenyl)-ethans oder des entsprechenden 2-R-(-) Enantiomers ( Fig. 1 und Fig. 2) fast quantitativ mit Magnesium in etherischen oder THF-Lösungen bei Temperaturen zwischen 4 - 15°C unter Beibehaltung der S- oder R-Konfiguration ohne jede signifikante Razemisierung reagiert. Die gleichen Reaktionen können auch in protonenfreien Lösungsmitteln durchgeführt werden, wobei die gleichen Ergebnisse hinsichtlich der chemischen Ausbeute und der optischen Reinheit unter Beibehaltung der Retention der Konfiguration erhalten werden. Um die chemische Synthese auszuführen, ist es nicht notwendig, die S-(+)- oder R-(-)-Grignard-Verbindungen des entsprechenden chiral-2-substituierten Ethans zu isolieren, um die chiralen-2-Aryl-Alkansäuren zu erhalten. Die enantiomeren 2-Aryl-Alkansäuren, insbesondere die der 2-Aryl-Propionsäuren, können leicht dadurch erhalten werden, daß Kohlendioxid durch die die Grignard-Verbindungen enthaltende Lösung geleitet werden. Es ist gefunden worden, daß die Ausbeute an Grignard-Verbindungen und die anschließende Behandlung mit CO₂ fast quantitativ ist und es kann als Regel gelten, daß die optische Reinheit mit zunehmendem s-Charakter des einbezogenen Orbitals zunimmt, was weiterhin untermauert wird durch die Bildung der entsprechenden Quecksilber-II-Verbindungen von hoher optischer Reinheit und der Beibehaltung der Konfiguration (Fig. 2). Im Falle der stabilen Quecksilber-Kohlenstoff-Bindung kann die Situation leicht dadurch erklärt werden, daß eines der 6 s-Elektronen auf ein freies 6-p-Orbital gehoben wird (4f¹⁴5d¹⁰6s² → 4f¹⁴d¹⁰6s¹6p¹), wobei sich zwei halbgefüllte Orbitale, die nicht äquivalent sind, ergeben. Aus Energiegründen ist die vom 6 p-Orbital gebildete Bindung stabiler als die vom 6-s-Orbital darüber gebildete, weil mit dem 6 p-Orbital eine größere Überlappung möglich ist. Eine sehr stabile Situation wird durch die Quecksilberverbindungen der entsprechenden R- oder S-Enantiomere der Halogenide (Fig. 2) erreicht, wenn im Falle der Bindungsbildung die 6 s- und die 6 p-Orbitale sich verbinden, um neue Orbitale (zwei-sp-Orbitale), die äquivalent sind, zu bilden.

Um gute chemische Ausbeuten unter Beibehaltung der Konfiguration im Falle der Bildung von Grignard-Verbindungen (Fig. 2, IV) und der anschließenden Umwandlung der Magnesium-organischen-chiralen Verbindungen zu den entsprechenden Carbonsäuren zu erreichen, ist es notwendig, reine und sehr reaktive Magnesiumoberflächen zu haben, da die auf der Magnesiumoberfläche adsorbierten Radikale (δ -Radikale) an den Oberflächen in hohem Maße gebunden werden, und sie erfindungsgemäß nicht dimerisieren, wenn zusätzlich CO₂ auf der aktiven äußeren Oberfläche vorhanden ist. Demnach gibt es keine Dimerisierung oder Disproportionierung in etherischen, THF oder protonenfreien Lösungsmitteln, z.B. Hexan, Benzol, Toluol, weil das Radikal nicht in Lösung hergestellt wird, jedoch ist es an der Oberfläche des Magnesiummetalls stabilisiert.

Ein anderer Weg der Metallisierung unter Beibehaltung der Konfiguration mit guten chemischen Ausbeuten der entsprechenden R- und S-Enantiomeren und anschließender Carbonierung, wobei optisch hochreine 2-Aryl-Propionsäuren der R- und S-Enantiomere gebildet werden, ist die Reaktion mit Methyllithium (CH₃Li, R-Li) (Fig. 2).

Der stereoselektive Einbau von CO₂ ist von äußerster Bedeutung bei der Herstellung von metallorganischen Verbindungen von hoher optischer Reinheit des entsprechenden Enantiomers mit Magnesium und Methyl-lithium als auch für die Quecksilber-Verbindungen R′-HgX, mit z.B. X = Br, Cl, CH₃COO⁻. Dies impliziert nicht notwendigerweise, daß diese stereospezifischen Verbindungen, obwohl neu und in dieser hohen optischen Reinheit bis heute noch nicht erhalten, isoliert und anschließend mit CO₂ behandelt werden müssen. Die Bildung der metall-organischen Verbindungen gemäß den Figuren 1 und 2 kann an die Behandlung mit CO₂ auf einer Stufe gekoppelt werden, so daß es nicht notwendig ist, die metall-organischen Verbindungen zu isolieren.

Ein anderer Weg zur Herstellung von optisch hochreinen Enantiomeren, R oder S, der 2-Aryl-Alkansäuren, insbesondere der 2-Aryl-Propionsäuren, die erfindungsgemäß leicht aus 2-Aryl-2-halogenid-ethan (Figuren 3 und 4) herstellbar sind, ist die direkte Umwandlung der 2-Aryl-2-halogenide mit Natriumtetracarbonyl-ferrat (-II) (Na₂Fe(CO)₄) in Gegenwart von Triphenylphosphin (Ph₃P) und anschließender Oxidation mit Jod-Wasser zur entsprechenden Säure oder in Gegenwart eines sekundären Amins, um das optisch reine Amid (Fig. 5) zu erhalten. Das Reagenz Na₂Fe(CO)₄ kann durch die Behandlung von Fe(CO)₅ mit Natriumamalgam (NaHg) in THF hergestellt werden.

Ein anderes Verfahren für die Umwandlung des enantiomeren, reinen 2-Aryl-2-halogenid-ethans (Fig. 2) zu den Säurederivaten macht ebenfalls von Na₂Fe(CO)₄ Gebrauch. Jedoch wird in Gegenwart von CO (Fig.6) und der Behandlung des Zwischenproduktes (IV) mit Sauerstoff oder Natriumhypochlorit und anschließender Hydrolyse die entsprechende enantiomere Säure mit hoher optischer Reinheit und chemischen Ausbeuten von 75 - 80% (vergl. Fig. 6) hergestellt.

Die Anwendung des Komplexes je zwischen Natrium-tetracarbonylferrat (II) und Phosphin (Ph₃P) oder Kohlenmonoxid ist insbesondere bei der Synthese von 2-Alkyl-Alkansäuren hilfreich, und zwar wegen seiner hohen Nucleophilicität und der Einfachheit der integrierenden Inversionsreaktionen dieses Systems. Demnach reagieren die Halogenide, die gemäß dieser Erfindung erhalten wurden und die Tosylate mit Na₂Fe(CO)₄ mit einer typischen Sₙ2-Kinetik, Stereochemie (Inversion), um koordinierte, gesättigte anionische d⁸-Alkyl-Eisen-(O)-Komplexe zu ergeben. Gemäß den Figuren 5 und 6, schafft dieses Verfahren Wege von den Alkyl- und Säurehalogeniden zu den Alkanen, Aldehyden, Ketonen und stereospezifischen Carbonsäuren, einschließlich ihrer Derivate.

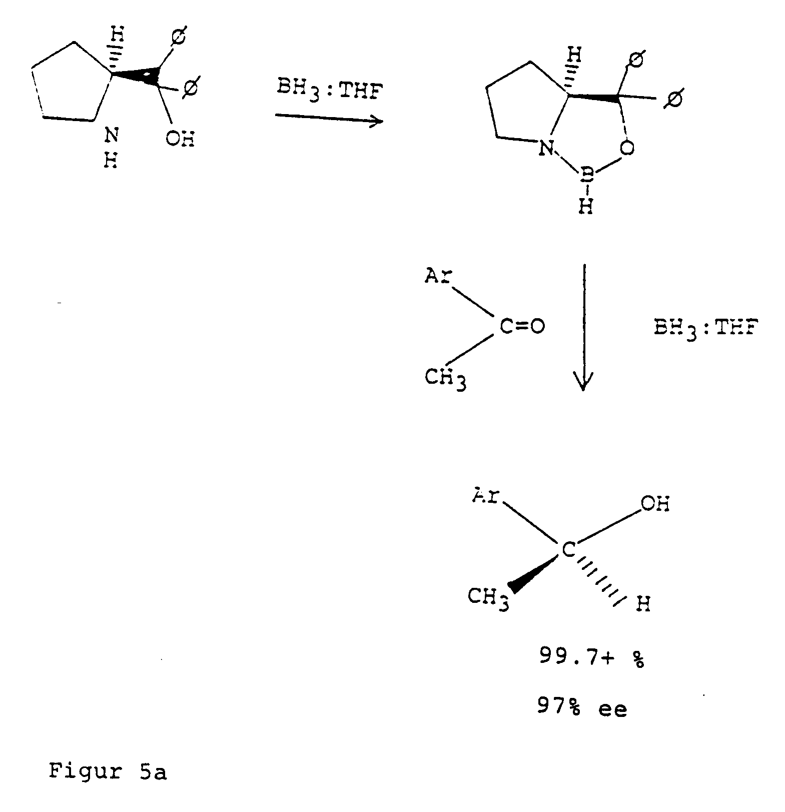

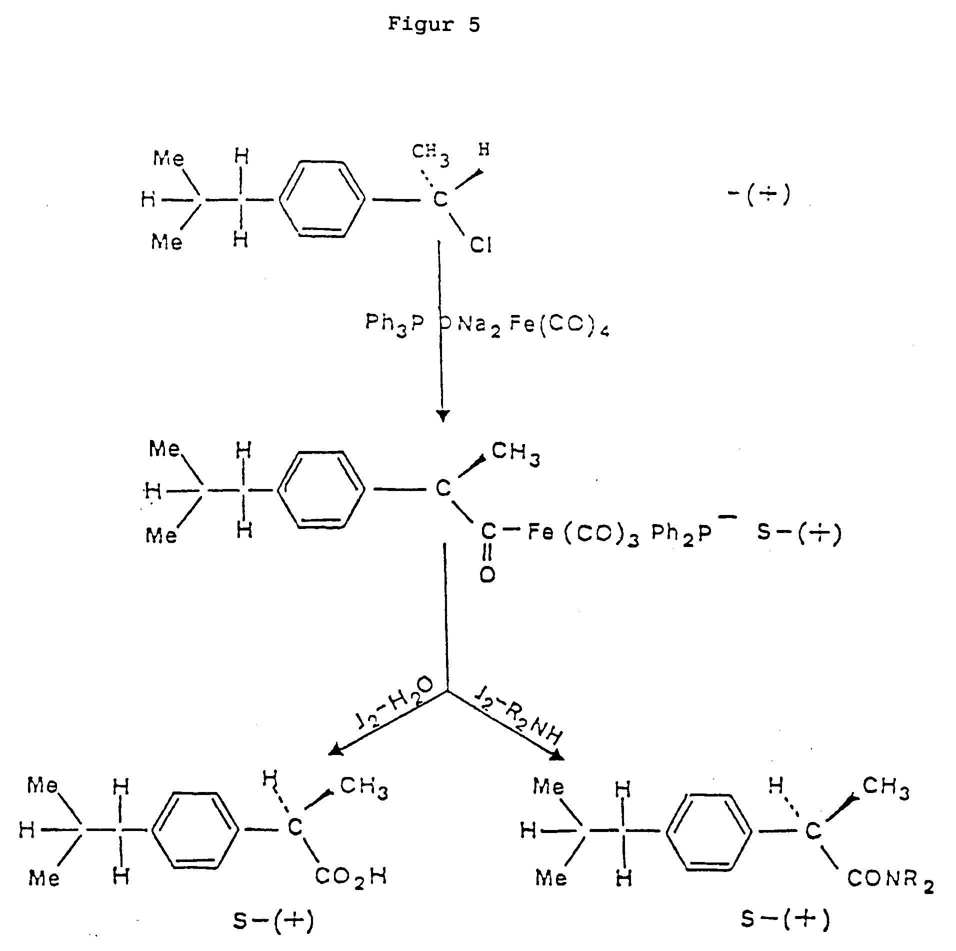

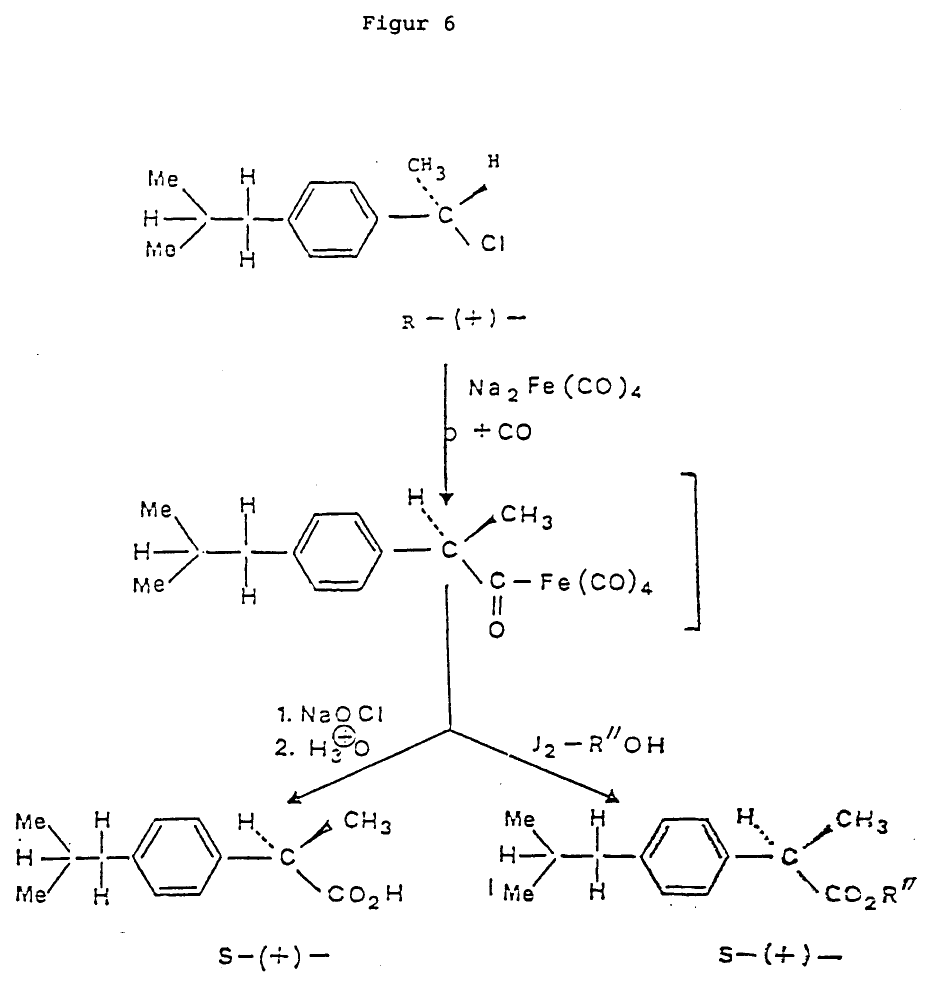

Ein ähnliches Verfahren verwendet die Umwandlung der Halogenide (Fig. 2, IV) zu den Estern durch die Behandlung des enantiomeren Halogenids ( R oder S ) mit Nickelcarbonyl (Ni(CO)₄) in Gegenwart eines Alkohols, bevorzugt eines primären Butanols, und der konjugierten Base, gemäß der Reaktion: 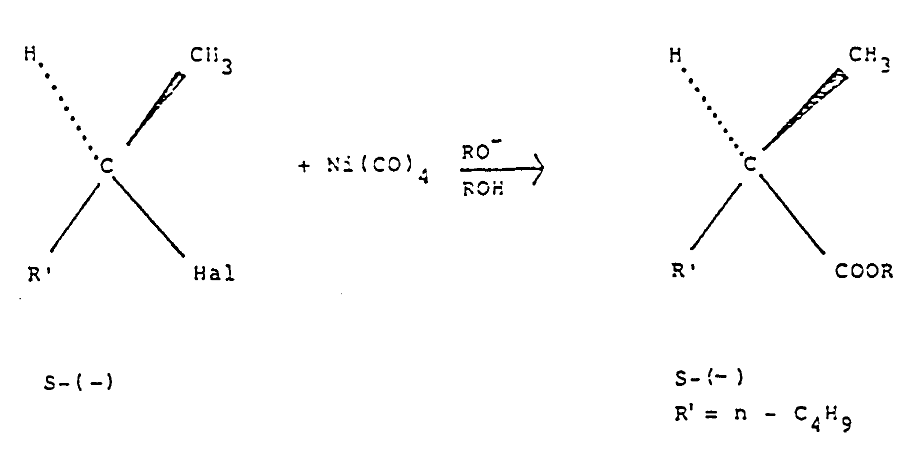
Die besten chemischen Ausbeuten werden für Hal = Br erhalten, wo hingegen Hal = Cl und J geringere Ausbeuten ( ca. 65%) ergeben. Jedoch ergeben alle Halogenide hohe optische Reinheiten (>95%).

Nachdem ein stereoselektives Verfahren zur Reduktion des Ketons zum entsprechenden enantiomeren Alkohol mit einer hohen optischen Reinheit (> 97%) und sehr hohen chemischen Ausbeuten (>90%) etabliert wurde, ist es möglich, entweder direkt aus dem R-Alkohol die S-Carbonsäuren oder über die R-Form des Halogenides (Figuren 2, 7 und 8) das entsprechende Nitril in Gegenwart von NaCN und DMSO bei 40 - 50°C herzustellen.

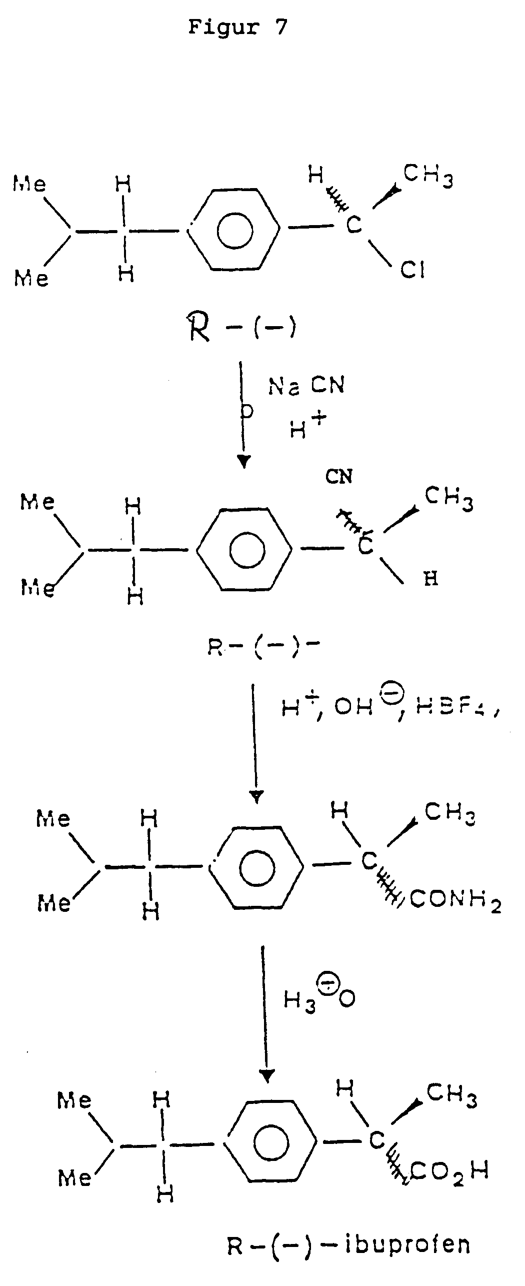

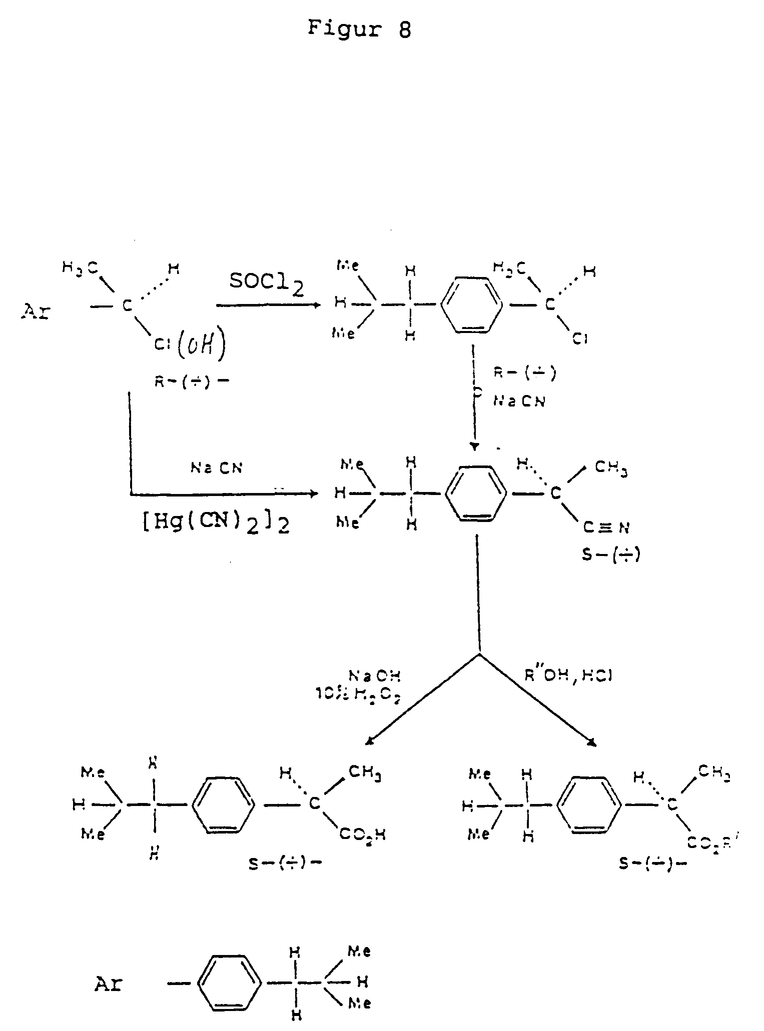

Anschließend können die enantiomeren S-Nitrile hydrolysiert werden, um entweder Amide oder die entsprechenden Säuren zu ergeben. Wenn die S-Säure gewünscht wird, ist das Reagenz der Wahl wäßrige NaOH, enthaltend etwa 6-8% H₂O₂, obwohl ebenso erfolgreich eine säure-katalysierte Hydrolyse ausgeführt werden kann. Die chemischen Ausbeuten können durch die Verwendung eines streng-polaren, protonenfreien komplexierenden Lösungsmittels wie HMPT für die Synthese der 2-Aryl-Propionsäuren oder durch die Komplexierung des Cyanidions als quaternäres Ammoniumsalz verbessert werden. Dieses Verfahren hat den Vorteil, daß die Kondensation leicht in einem kontinuierlichen Verfahren beobachtet werden kann, z.B. als Et₄⁺CN⁻ oder C₆H₅·CH₂·N⁺CN, wobei eine Phasentransferkatalyse angewandt wird oder Kristalle wie Dicyclohexan-18-crown-6.

Die Herstellung der S-enantiomeren Nitrile durch Et₄N⁺CN oder Na(K)CN kann entsprechend den bekannten Verfahren durchgeführt werden, beschrieben z.B. in : J.M. Teulon et al., J. Med. Chem. 21 (9) 901, (1978), N. Tokutake, Chem. Abstracts 88, 50512f; S. Kothicki et al., Chem. Abstracts 90, 1036526; H. Kobler et al., Liebig's Ann. Chem. 1946, (1978); T. Amano et al., Chem. Abstracts, 13, 2611 p; Nissan Chemical Industries, Ltd., Chem. Abstracts, 101 90603e, (1984), Nissan Chemical Industries, Ltd., Chem. Abstracts, 101, 6855 h; J.A. Foulkes and J. Hutton, Synth. Commun. 9 (7), 625 (1979). Diese erwähnten Verfahren führen jedoch nur zu Razematen.

Gewöhnlich sind die 2-Aryl-Alkansäuren, insbesondere die der 2-Aryl-Propionsäuren, kaum in Wasser löslich, weshalb am Ende der Reaktionen die optisch aktiven 2-Aryl-Propionsäuren leicht durch Filtration usw. isoliert werden können. Um jedoch eine Filtration oder Kristallisationen von organischen Lösungsmitteln usw. zu vermeiden, ist eine Destillation bei hohem Vakuum (ca. 0,06 mm Hg) aufgrund der geringen Schmelz- und Siedepunkte der entsprechenden Enantiomeren der 2-Aryl-Propionsäuren ein geeignetes Verfahren für die weitere Aufreinigung. Weiterhin wird ein pharmazeutisches Erzeugnis, welches so rein ist, wie es vom US-Arzneibuch vorgeschrieben wird, durch eine Säure-Base-Behandlung des durch Filtration, Präzipitation oder Destillation in Hochvakuum isolierten Produktes erhalten.

Die Hauptvorteile der vorliegenden stereospezifischen Synthese von 2-Aryl-Propionsäuren sind aus industrieller Sicht folgende:
i) Das Verfahren ist enantio-selektiv und liefert 2-Aryl-Propionsäuren in hohen chemischen Ausbeuten und mit einem enantiomeren Verhältnis , welches höher ist als das epimere Verhältnis der bekannten synthetischen Verfahren;
ii) die Reaktionslösungsmittel verursachen ökonomisch niedrige Kosten und weisen Vorteile in der Sicherheit auf;
iii) die chiralen Komplexe können wiederverwendet werden und wirken bei hohem enantiomeren Überschuß für die R- und S-Enantiomeren; die Kosten für neue chirale Komplexe werden somit verringert;
iv) es werden keine weiteren Komplexe benötigt, da die Reaktionen entweder durch die Erhaltung der Konfiguration oder, im Falle der Herstellung von optisch aktiven Nitrilen, in hohen chemischen Ausbeuten und mit hoher optischer Reinheit ohne Razemisierung, stattfinden;
v) Hilfssubstanzen sind ökonomisch und verursachen geringe Kosten;
vi) die verschiedenen chemischen Schritte können in zwei Reaktoren durchgeführt bzw. auf diese verringert werden, da die Zwischenprodukte nicht isoliert werden müssen;
vii) die optisch aktiven 2-Aryl-Propionsäuren (S oder R) können aus der Reaktionsmischung einfach durch Filtration, Präzipitation oder Destillation in Hochvakuum abgetrennt werden;
viii) es sind keine hohen Energiekosten für die Durchführung der Synthese im industriellen Maßstab erforderlich.

Eine geeignete Verbindung, die von der 2-Aryl-Propionsäure, bevorzugt in der S-Form, für die pharmazeutische Verwendung gebildet wird, ist der Komplex zwischen 1-Amino-1-desoxy-D-glucit (D-Glucamin) und den S-(+)-2-Aryl-Propionsäuren. Diese Verbindungen weisen den Vorteil auf, daß sie wasserlöslich und lipophiler sind, wenn sie als oder in transdermalen Abgabesystemen verwendet werden und sie zeigen in vivo und in vitro antimikrobielle Aktivitäten aufgrund ihrer Oberflächenaktivität und bilden gemischte Mizellen mit Phospholipiden.

Es wurde z.B. gefunden, daß 1-Amino-1-desoxy-D-glucit, z.B. mit R-(-)-Ibuprofen einen 1:1-Komplex bildet, der über Wasserstoff gebunden ist (Fig.9). Der Kristall weist Zellabmessungen von a = 8,275 Å, b = 40,872 Å und c = 6,420 Å mit vier Molekülen in einer Zelleinheit auf, wobei sie die Raumgruppe P 2₁2₁2₂ (# 19) besitzen. Die Komplexstruktur des 1:1-Komplexes zeigt eine starke Bindung zwischen dem Wasserstoff der Carboxylgruppe des S-Ibuprofens und dem O₃-Sauerstoff des 1-Amino-1-desoxy-D-glucits, ohne daß der Wasserstoff der 1-Amino-Gruppe beteiligt wäre (Fig. 9), ähnlich dem S-(+)-Ibuprofen-D-glucamin-Komplex.

Das (R,S)-Ibuprofen bildet ebenfalls einen 1:1-Komplex, wobei es sowohl die enantiomere (S)-(+)-Ibuprofen-D-glucamin-Struktur als auch die entsprechende R-(-)-Ibuprofen-D-glucamin-Struktur zeigt (Fig. 10).

Nachdem die stöchiometrischen Komplexe zwischen S-(+)-Ibuprofen und D-Glucamin oder D-Ribamin hergestellt sind, ist es möglich, eine pharmazeutische Formulierung auf der gleichen chemischen Basis herzustellen, z.B. durch eine Wechselwirkung zwischen den Carbonsäuren und dem Hydroxyl des D-Glucamins in einer Schmelze. Das Harz dieser Schmelze besteht z.B. aus Polyoxyethylenglycolaten oder Polyoxyethylen-Einheiten, welche im allgemeinen ein durchschnittliches Molekulargewicht von 400 bis höchstens 6000 aufweisen. Polyoxypropylenoxide sind ebenfalls sehr nützlich, weil sie in der Lage sind, Akzeptoren für Wasserstoffe bereitzustellen, welche von den Carbonsäuren aufgrund der Deprotonierung geliefert werden, wobei sehr stabile Wasserstoffbindungen zwischen den 2-Aryl-Alkansäuren und dieser Matrix gebildet werden. Es gibt verschiedene Vorteile einer solchen pharmazeutischen Formulierung gegenüber Mikrokristallen des reinen Arzneimittels, z.B. S-(+)-Ibuprofen, nämlich die Auflösungseigenschaften, das Abfüllen in Hartkapseln aufgrund einfacher Handhabung und, was am wichtigsten ist, das S-(+)-Ibuprofen, das Naproxen und andere 2-Aryl-Propionsäuren in enantiomer-reiner Form verhalten sich physikalisch in der Schmelze genauso wie in wäßrigen Lösungen, wobei sich eine halbverdünnte molekulare Lösung von z.B. S-(+)-Ibuprofen innerhalb des Salzes ergibt. Dieses unerwartete Verhalten sowohl in der Schmelze als auch in wäßriger Lösung, was durch Streuungsversuche nachgewiesen wurde und in Zusammenhang mit einem Beispiel beschrieben wird, sichert die Stabilität des S-(+)-Ibuprofens und die Retention der chiralen Konfiguration ohne jede Razemisierung, welche z.B. dann auftritt, wenn auf die R-(-)-Ibuprofen-Tabletten oder Mikrokristalle aufgrund einer Änderung des molalen Volumens der R-enantiomeren Form Druck ausgeübt wird. Dies kann bei Druckausübung ebenfalls bei S-(+)-Ibuprofen-Mikrokristallen passieren, so daß es wünschenswert ist, eine feste pharmazeutische Formulierung zu haben, welche u.a. diese Nachteile vermeidet. Weiterhin können die Harze, aufgebaut aus Polyoxyethylenoxid-Einheiten mit einer Kettenlänge gemäß den entsprechenden Molekulargewichten von zwischen 400 bis 6000 und mit Schmelzpunkten von etwa 50 - 55°C S-(+)-Ibuprofen vollständig in Form einer molekularen Lösung in der Schmelze auflösen. Das Arzneimittel verhält sich in dieser Schmelze als eine molekulare Lösung eines gelösten Stoffes (Arzneimittel, z.B. S-(+)-Ibuprofen, S-Naproxen) in einem Lösungsmittel (Polyoxyethylenoxid) gemäß den Gesetzen der Lösungschemie im physikalischen Sinn. Dies impliziert, daß es keine Abtrennung des S-(+)-Ibuprofens aus dieser Schmelze gibt, es sei denn, daß die feste Lösung durch das Arzneimittel übersättigt ist. Diese isotropen Lösungen des S-(+)-Ibuprofens in Polyoxyethylenoxid (Lösungsmittel) kann sowohl in der Schmelze als auch verdünnt im wäßrigen Medium durch Röntgenkleinwinkel- und Neutronenstreuung verfolgt werden und kann ebenso mit dem festen Zustand der Schmelze verglichen werden (Figuren 11 und 12).

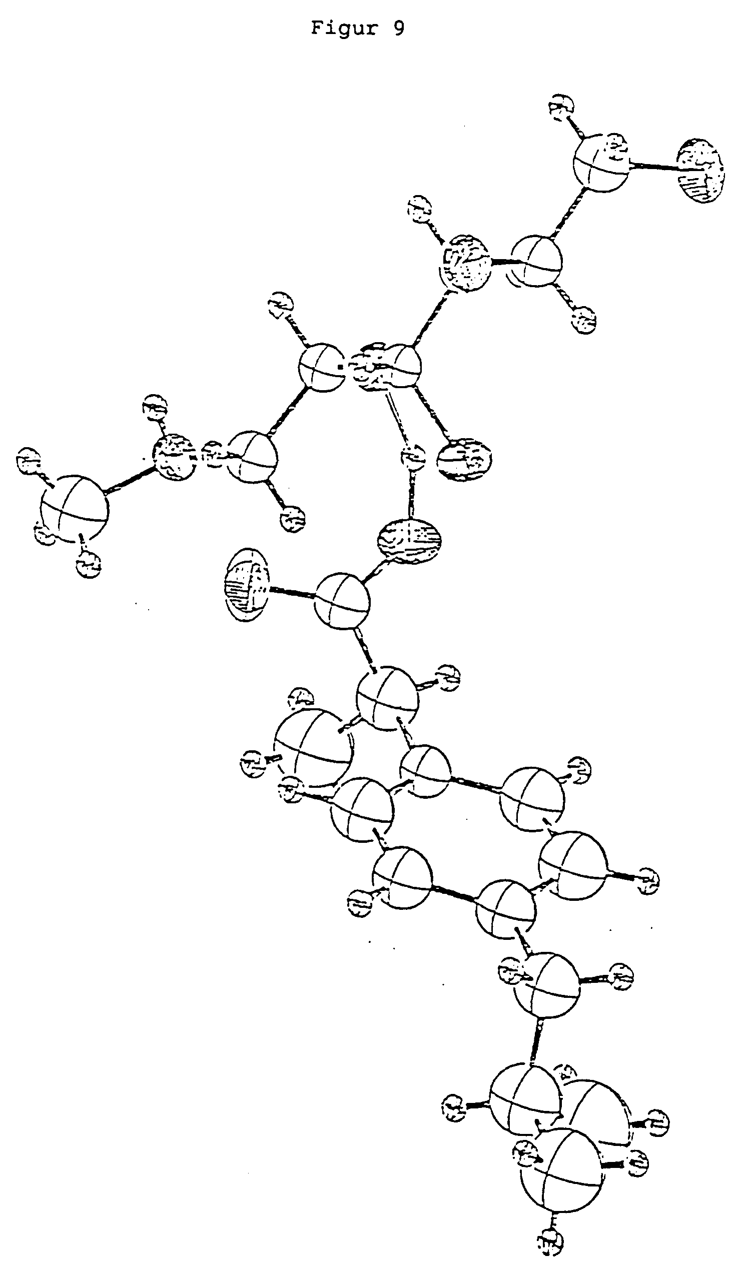

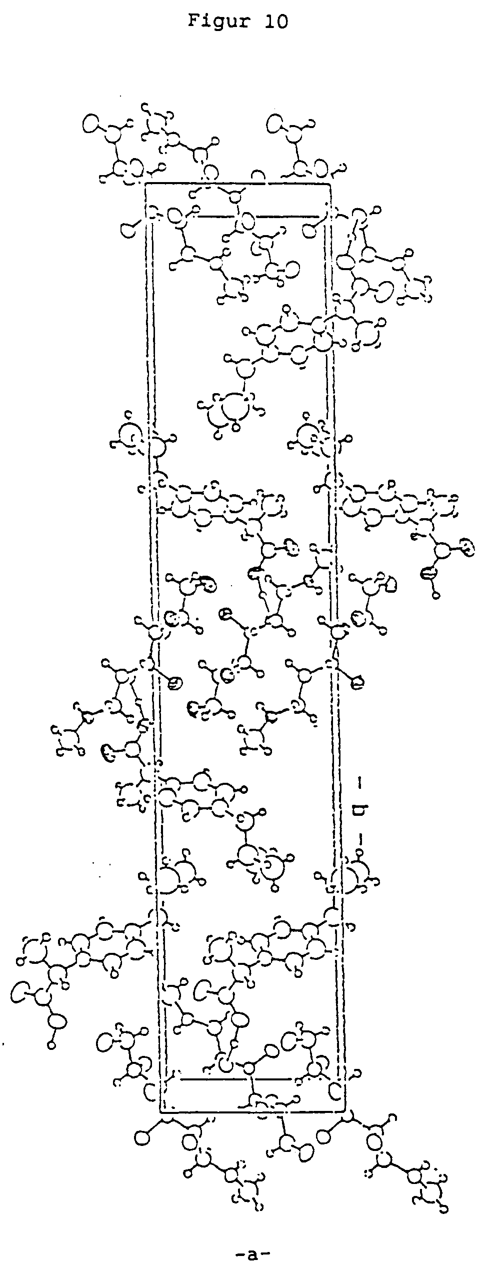

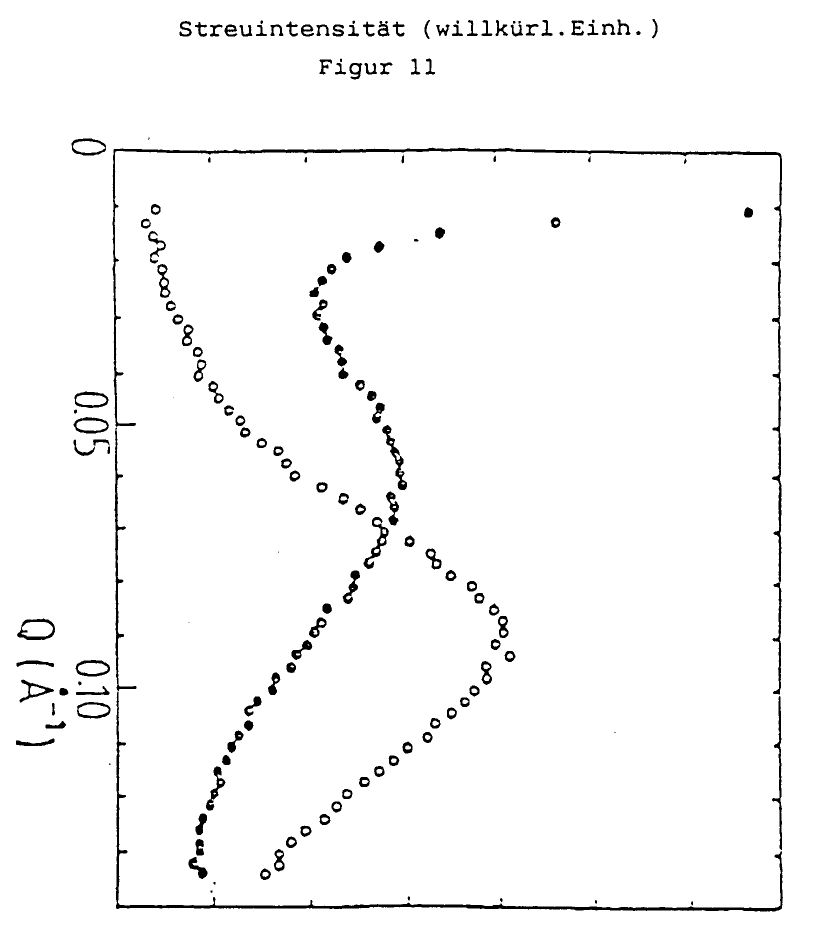

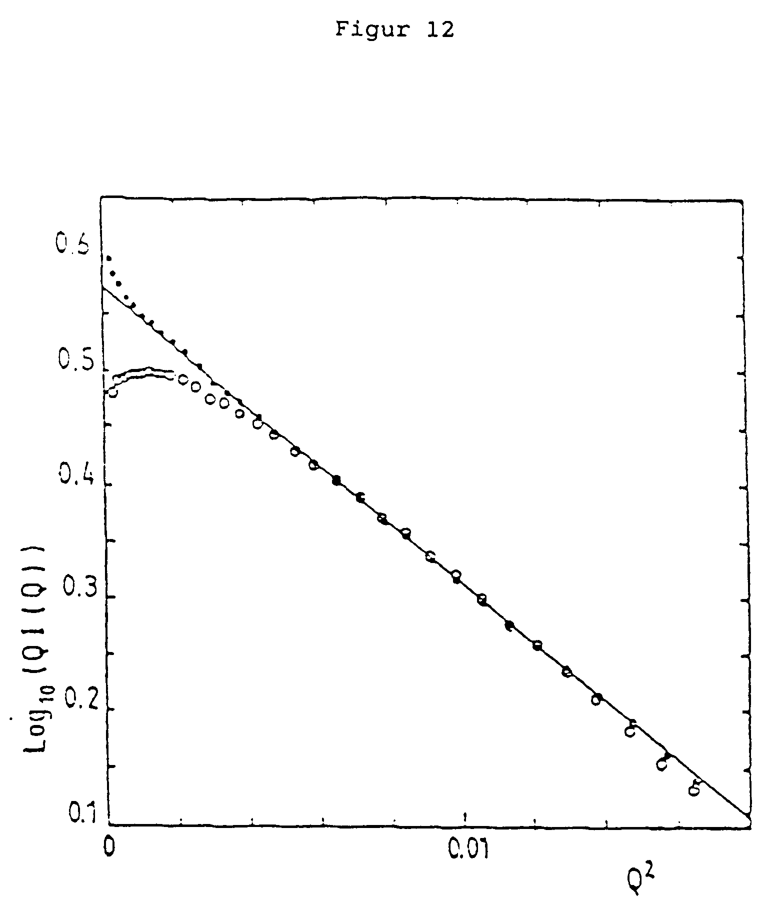

Insbesondere können chirale Verbindungen, die als Arzneimittel in bestimmten pharmazeutischen Formulierungen verwendet werden, Schwierigkeiten verursachen, z.B. durch Razemisierung, Wechsel in eine andere polymorphe physikalische Form, Verschlechterung bzw. Zerstörung des enantiomeren aktiven Arzneimittels als auch unerwünschte Nebenwirkungen bezüglich der in vivo und in vitro Auflösung. Die Verbesserung der Stabilität und der Retention der Konfiguration der pharmazeutischen Formulierung hinsichtlich der reinen enantiomeren 2-Aryl-Alkansäuren, z.B. des S-(+)-Ibuprofens und des S-(+)-Naproxens einschl. geeigneter Auflösungsgeschwindigkeiten in vitro und in vivo wird erfindungsgemäß dadurch erreicht, daß die enantiomeren, reinen 2-Aryl-Propionsäuren in einer Schmelze, bestehend aus Polyoxyethylenglycol, Polyoxyethylenoxid oder ihren Mischungen mit durchschnittlichen, gewichtsmäßigen Molekulargewichten zwischen 300 bis 6000 gelöst werden. Außerdem sind diese Harze meist wasserlöslich, wenn sie in Kontakt mit wäßrigen Lösungen kommen, z.B. mit Emulsionen, Suspensionen einschl. Mikroemulsionen, und in Matrizen mit Öl in Kombination mit Wachs oder Paste, welche normalerweise für die Füllungen in Weichgelatinekapseln verwendet werden. Diese und andere nicht-wäßrige Lösungen, Emulsionen usw. von pharmazeutischem Interesse sind beschrieben in Schick et al., "Emulsions and Emulsions Technology," Vol. 6, Surfactant Science Series, II, Chapter 13, "Cosmetic Emulsion," 1974, 729-730, ed. J. Tissaut-Marcel Dekker Inc., N.Y., U.S., M.J. Schick in "Nonionic Surfactants," Physical Chemistry, 1988, Marcel Dekker, Inc. N.Y. und Basel. Die EP-Nr.: 83109839.4 "Anhydrous Emulsion and the Size Thereof" lehrt die Herstellung einer pharmazeutischen Zubereitung, welche bei 37°C schmilzt, jedoch auf der physikalisch-chemischen Basis einer Emulsion. Diese Zubereitungen zeigen jedoch den Nachteil, aufgrund thixotropischer Prozesse undicht zu sein, wenn Hartgelatinekapseln gefüllt werden.

Dieses neu beschriebene Verfahren für eine Schmelze in einer geeigneten Matrix, enthaltend bestimmte Mengen an enantiomeren 2-Aryl-Alkansäuren oder 2-Aryl-Propionsäuren, weist folgende Vorteile auf :
i) eine wirkliche Lösung im physikalischen Sinne;
ii) eine sehr verläßliche Dosierungsform von hoher Homogenität, wie sie in wirklich physikalischen Lösungen zu finden ist;
iii) leichte technische Handhabung, wenn sie als Hartgelatinekapseln verwendet werden;
iv) keine Entmischungs-Phänomene auf molekularer Basis;
v) Erreichen bester in vitro und in vivo Auflösung und
vi) schnelle Resorption des Arzneimittels.

Die Oberflächenaktivität der enantiomeren 2-Aryl-Propionsäuren ist nur an die S-Form gebunden. Diese spezielle Aktivität kann erhöht werden durch die Komplexierung mit D-Glucamin, wie oben angegeben wurde, oder durch D-Ribamin als auch durch kationische Reagenzien, insbesondere durch n-Hexadecylpyridinium oder Benzethonium-Kationen durch Bindung an die Carboxogruppen der S-2-Aryl-Propionsäuren. Die Erhöhung der Oberflächenaktivität und damit der antimikrobiellen Aktivität steht in Beziehung mit der hydrophoben Kette der Hexadecylpyridinium- oder Benzethonium-Reste aufgrund ihrer geringen kritischen Mizellen Konzentrationen (KMK) von ungefähr 1,5 x 10⁻⁵ Mol/l, wenn sie z.B. mit S-(+)-Ibuprofen komplexiert sind. Ein Vorteil dieser pharmazeutischen Formulierung ist eine Dosisverringerung aufgrund einer schnelleren Penetration des S-(+)-Ibuprofens durch die Membranen und die Haut und ein schnelleres Erreichen der Zielzellen aufgrund der Mizellisierung und Zielsteuerung des S-(+)-Ibuprofens.

Deshalb verringert aus medizinischer Sicht die Verwendung der Komplexe zwischen 2-(S)-Aryl-Propionsäuren oder -Alkansäuren mit kationischen Detergentien, D-Glucamin oder D-Ribamin die Dosis der nicht-steroidalen Substanzen aufgrund der Trägerfunktion der kationischen oberflächenaktiven Mittel oder der D-Zucker-Alkohole stärker als die Verwendung von Alkali oder Erdalkalimetallen als auch der (S,R)-Lysin-Salze oder der 2-Aryl-Alkansäuren.

Röntgenbeugungsdiagramme als auch Röntengkleinwinkelstreuungsprofile (Fig. 11, 12) zeigen ein strukturloses Verhalten im Sinne einer diffusen Streuung. Es ist deshalb möglich, die Streuungsdaten bei hohen und niederen Streuungswinkeln in thermodynamischer Weise zusätzlich zur Gesamtelektronenverteilung der Polyoxyethylenkette (Matrix) und des löslich gemachten Arzneimittels, z.B. S-(+)- oder R-(-)-Ibuprofen, zu behandeln. Es wurde gefunden, daß die Streuungskurven der Schmelze (Ligand), enthaltend die Matrix und das in der Schmelze gelöste Arzneimittel (S-(+)-Ibuprofen), das gleiche Streuungsprofil zeigt, wenn die feste Schmelze in einem wäßrigen Medium gelöst wird. Es war wichtig zu finden, daß ein bestimmter Grad an Clusterung der S-(+)-Ibuprofen oder S-(+)-Naproxen-Moleküle in der flüssigen Schmelze als auch in der festen Schmelze stattgefunden hat, teilweise entlang der hydrophilen linearen Makromoleküle, wie z.B. von PEG 1500 oder den Polymorphylenoxiden. Es wurde gefunden, daß in der flüssigen Schmelze das durchschnittliche, gewichtsmäßige Molekulargewicht, MWₐᵥ, in der Größenordnung von 25.000 +/- 5.000 ist; ähnliches gilt für die feste Schmelze und für die in wäßriger Lösung in Gegenwart von S-(+)-Ibuprofen. Der durchschnittliche, elektronische Massenradius, Rg, lag im Bereich von Rg = 45,0 ±/- 5,0 Å, so daß die Konformation der linear verknüpften -CH₂ oder -OCH₂-Gruppen einer linearen Zick-Zack- oder Mäander-Konformation entspricht. Durch Zugabe von S-(+)-Ibuprofen oder einer anderen enantiomeren 2-Aryl-Alkansäure ist MWₐᵥ eine Funktion der Konzentration des enantiomeren pharmazeutischen Wirkstoffs, was durch eine Änderung des zweiten Virialkoeffizienten und des isothermen Kompressibilitätskoeffizienten einer Einkomponentenlösung beschrieben werden kann. Die Aufnahme des Wertes des zweiten Virialkoeffizienten, beobachtet für S-(+) oder R-(-)-Ibuprofen, steht in Beziehung mit einer Zunahme des eingeschlossenen Volumens der enantiomeren Form innerhalb der Schmelze. Die molekulare Erklärung für dieses unerwartete Lösungsverhalten in der Schmelze (fest, flüssig) und in der wäßrigen Lösung liegt in der Bindung der exponierten hydrophoben Bereiche der enantiomeren 2-Aryl-Propionsäuren, z.B. von Isobutylbenzol oder Naphthyl-Gruppen an CH₂-Gruppen. Die freie Energie G dieser spezifischen Bindung der enantiomeren pharmazeutischen Wirkstoffe der 2-Aryl-Propionsäuren liegt bei 0,5 - 0,7 k_{B}.T pro PEG Molekül, für S-(+)-Ibuprofen bei einem Wert von 0,56 k_{B}.T pro PEG oder 0,50 k_{B}.T für eine Polyoxyethyleneinheit. Ähnliche Wert, wenn auch verschieden zu den für die S-Enantiomeren gefundenen, wurden für das R-(-)-Ibuprofen ermittelt, und zwar 0,45 -0,51 k_{B}.T pro PEG-Molekül oder 0,46 k_{B}.T pro -CH-O-Einheit. Das Razemat weist einen G-Wert von 2,5 k_{B}.T/PEG auf, der sehr verschieden von den reinen Enantiomeren ist. Eine sehr wahrscheinliche, geometrische Beschreibung dieser Schmelze als auch dort, wo es in einer wäßrigen Lösung gelöst ist, ist die einer "Halskette". Die zufälligen Knäuel ("coils") der PEO- oder PEG-Einheiten sind um die hydrophoben Kernstücke der S-(+)-Ibuprofen- oder Naproxen-Moleküle gewickelt, während die hydrophile Carbonsäure nahe der Oxiethylen (-CH₂-O)-Einheiten oder der (CH₂)OH-Einheiten des PEG lokalisiert ist. Die Protonen der 2-Aryl-Propionsäure-Stereoisomere wechseln zwischen den etherischen oder Hydroxyl-Gruppen der PEO und PEG-Moleküle und den Carboxo-Gruppen, wie ebenfalls durch FT-IR-Untersuchungen gezeigt werden konnte. Diese Situation tritt ebenfalls auf, wenn sie in wäßrige Lösung gehen, unterstützt durch die Wechselwirkung der Wassermoleküle mit den PEO- und PEG-Resten, wobei die 2-Aryl-Propionsäure-Stereoisomere gegen den pH, eine unerwünschte Protonierung oder Razemisierung bei einem alkalischem pH geschützt und die Retention der Konfiguration erhalten wird. Die Streuungskurven (Figuren 11 und 12) der festen Schmelzen, z.B. PEG oder PEO mit dem gelösten S-(+)-Ibuprofen, zeigen keine Interpartikel-Interferenzwirkungen, auch dann nicht, wenn die Versuche in wäßrigen Lösungen durchgeführt werden. Es wurde gefunden, daß diese speziellen Schmelzen, welche S-(+)-Ibuprofen enthalten, kein Demixing-Phänomen zeigen, wenn sie mit zunehmender Temperatur flüssig werden, wie dies bei PEG und PEO in wäßriger Lösung auftritt. Interessanterweise wurde dies ebenso in wäßrigen Lösungen gefunden, wenn die Schmelzen (fest) zwischen 37-40°C in Lösung gehen. Normalerweise trennen sich Mischungen dieser Art mit zunehmender Temperatur (Phasenabtrennung), wobei dies von den Bestandteilen der Mischung abhängt, wenn sie die Phasen-Abtrennungstemperatur erreichen. Dies ist gemäß dieser Erfindung nicht der Fall, weil die repulsiven Kräfte durch den Zusatz der enantiomeren 2-Aryl-Propionsäuren sowohl in den Schmelzen als auch in den wäßrigen Lösungen reduziert werden. Dies wird ebenso durch die Aufzeichnung der Streuungskurven bei hohen Streuungsvektoren der Schmelzen deutlich
(Figuren 13, 14).

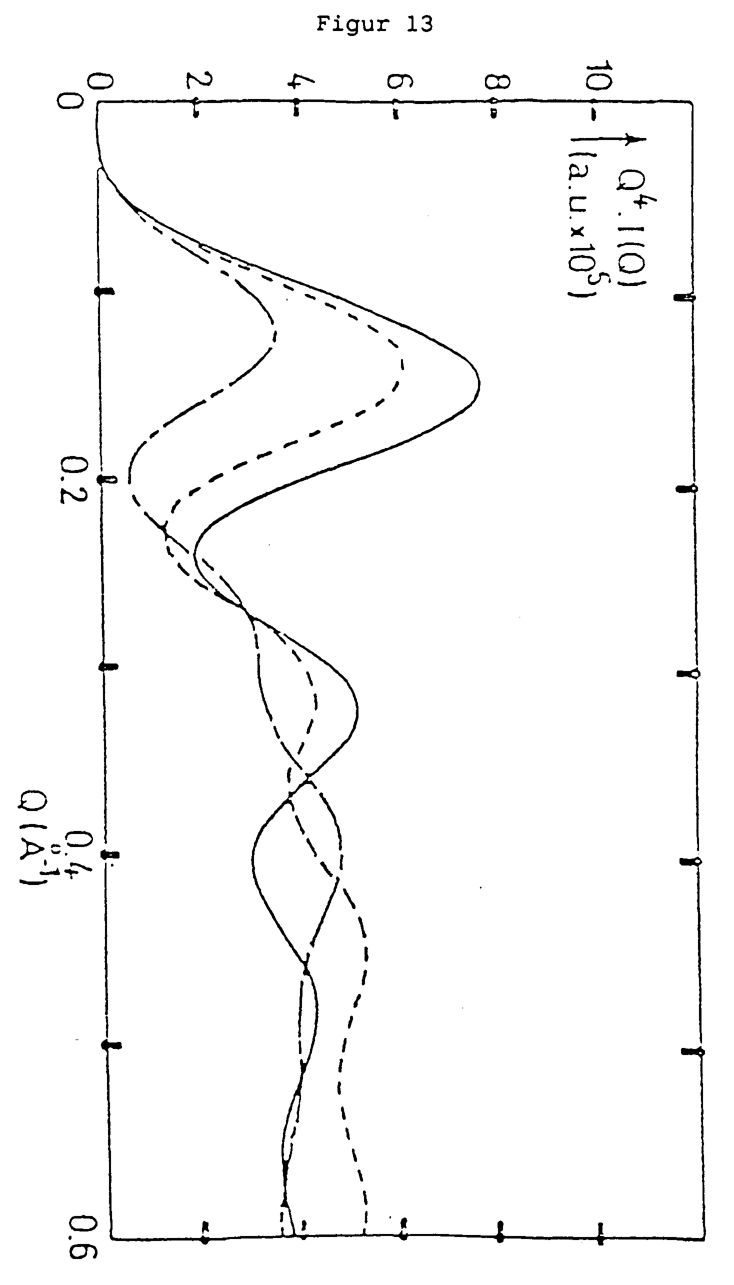

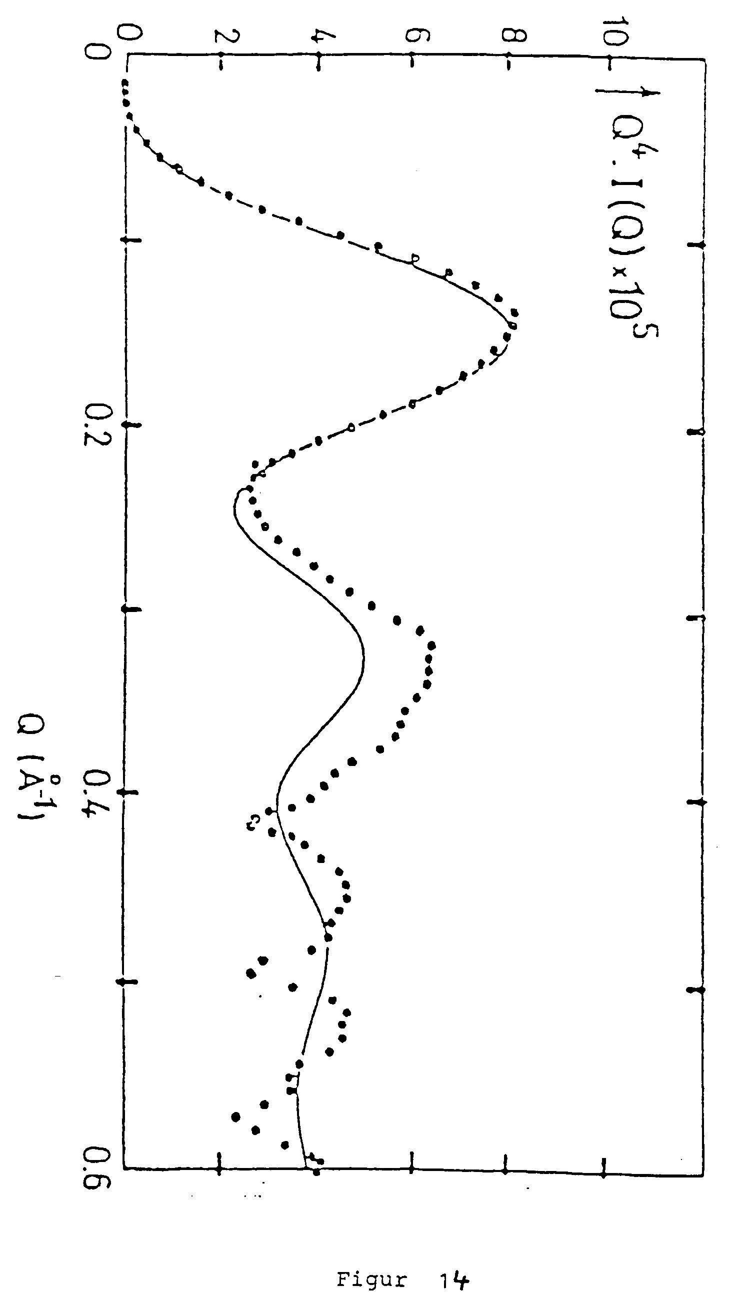

Weitere Aufgabenstellungen, Merkmale und Vorteile der vorliegenden Erfindung ergeben sich aus der folgenden Beschreibung anhand von Ausführungsbeispielen.

Alle synthetischen Verfahren zur Herstellung der enantiomeren 2-Carbinole wurden in Abwesenheit von Feuchtigkeit ausgeführt, bevorzugt unter Stickstoffatmosphäre.

Das (+)-(2S, 3R)-4-Dimethylamino-3-methyl-1,2-diphenyl-2-butanol (R*OH) sollte ein [α]_{D}27 + 8,00° (c 9,52, EtOH) Sp 56°C haben und in einem Exsikkator über P₂O₅ in Gegenwart von N₂ gelagert werden. Dieser Alkohol kann aus Hydrochlorid wiedergewonnen und wiederholt wiederverwendet werden. Die Lösungsmittel wie Ether, (ET₂O), THF, 1,2-Dimethoxy-ethan und Benzol werden über LiAlH₄ destilliert und sollten über Molekularsieben aufbewahrt werden. Die Vorratslösungen von LiAlH₄ sollten unter N₂ aufbewahrt werden und ein Glasfilter (1G2) unter N₂ vor ihrer Verwendung passieren.

### BEISPIEL 1

### Herstellung von R-(+)-2-(4-Isobutyl-phenyl-) hydroxy ethan

10g R*OH werden in 10 ml Et₂O bei 0°C gelöst und zu dieser etherischen Lösung von R*OH bei 0°C werden unter stetigem Rühren 10 ml einer 1 M-Lösung von LiAlH₄ hinzugegeben. Ein weißes, pastöses Präzipitat entwickelt sich bei 0°C. Es ist wichtig, daß dieses Präzipitat gut gerührt wird, so daß sich eine weiße, homogene Suspension entwickeln kann. Nachdem sich der R*OH LiAlH₄-Komplex gebildet hat, was innerhalb von 5 Minuten bei 0°C beendet sein sollte, werden 10 g (5mmol) von 1-(4-[2-Methylpropyl]-phenyl)ethanon, gelöst in 10 ml Ether bei 0°C, tropfenweise unter stetigem Mischen zur Suspension hinzugegeben, wobei die Temperatur zwischen 0°C bis 5°C für eine Stunde konstant gehalten wird. Nach der Zugabe des Ketons ergibt die Suspension eine klare, transparente Lösung, welche zur Vervollständigung der Reaktion 12 Stunden lang unter stetigem Mischen zwischen 0°C bis 5°C stehengelassen wird. Diese Mischung wird mit 0,5 ml Wasser hydrolysiert und mit Salzsäure verdünnt, welche hinzugegeben wird, um R*OH für eine spätere Wiederverwendung zu lösen. Das Mischen wird für weitere 2 Stunden bei 20°C fortgesetzt, bis sich wiederum eine transparente Lösung ergibt. Die klare Lösung wird mit Et₂O extrahiert, wobei R*OH HCl in der wäßrigen Phase und R-(+)-2-(4-Isobutyl)-hydroxy-ethan in der organischen Phase verbleibt. Die etherischen Extrakte werden vereinigt, konzentriert und in Hochvakuum (0,1 mm Hg; Kp 80°C) destilliert, was eine klare, transparente, ziemlich viskose Flüssigkeit (11,5 g, 94% Ausbeute) ergibt, welche, gemessen durch HPLC-Techniken, kein nicht-reduziertes Keton mehr enthält. Die optische Reinheit wird durch die Umwandlung zum MTPA-Derivat bestimmt und die Messung des NMR-Spektrums ergibt einen Wert von 98% e.Ü. (enantiomerer Überschuß).

Die Neutralisation des Säureextrakts ergibt wiedergewonnenes chirales R*OH mit [α]_{D}20 + 8,21° (C11,0 EtOH). Es wurde beobachtet, daß die Zugabe von Molekularsieben, z.B. von Zeolithen, die Kinetik der Bildung des enantiomeren R-(+)-Carbinols bei 0°C aus der Reduktion des 1-(4-[Methylpropyl] phenyl)-ethanons erhöht. 5 g von R*OH werden in 5 ml Et₂O oder THF, Benzol, 1,2-Dimethoxy-ethan oder Toluol in Gegenwart von 0,2 g kommerziell erhältlichen, aktiven 4A Molekularsieben gelöst und unter einem N₂-Strom stark gerührt. Zu dieser Mischung wird unter stetigem Mischen eine Lösung von 5ml einer 1M-Lösung von LiAlH₄ bei 0°C hinzugegeben. Die Reaktionszeit für die Umwandlung des asymmetrischen Ketons zum entsprechenden enantiomeren R-Carbinol kann auf 2 Stunden oder weniger verringert werden. Aus technischen Gründen können die Molekularsiebe zentrifugiert und in gleicher Weise, wie für R*OH oben beschrieben, wiederverwendet werden.

Bei einem anderen Verfahren wird das Keton sofort nach der Bildung des Reagenz, R*OH LiAlH₄, in Gegenwart der Molekularsiebe hinzugegeben, was den Vorteil hat, daß kein reduziertes Keton im hergestellten R-Carbinol vorhanden ist. Dies ist insbesondere wichtig, um fast quantitative chemische Ausbeuten an nicht-substituiertem R-2-Naphthyl-2-hydroxy-ethan oder R-2-[2-Fluor-4-diphenyl]-2 hydroxy-ethan, R-2-[4-Chlor-2-phenyl]-2-hydroxy- ethan, R-2-[6-Hydroxy-2-naphthyl]-2-hydroxy-ethan und R-2-[6-Methoxy-2-naphthyl]-2 hydroxy-ethan zu erreichen.

### BEISPIEL 2

### Herstellung von S-(4-Isobutyl)-2-hydroxy-ethan

10g R*OH (35 mmol) werden in 20 ml Et₂O gelöst und 15,6 mmol LiAlH₄, gelöst in 30 ml Et₂O, werden hinzugegeben und bei 20°C in Gegenwart von 0,2g an Molekularsieben gerührt. Diese Suspension wird für 10 Minuten unter stetigem Rühren in Gegenwart der Molekularsiebe refluxiert. Die Lösung, welche in Gegenwart der Molekularsiebe einen klaren Überstand haben sollte, wird bei 20°C für 20-24 Stunden im Falle eines nicht-schnellen Mischens aufbewahrt; jedoch ist bei schnellem Mischen bei 20°C die Bildung des chiralen Komplexes in Gegenwart von Molekularsieben nach 2 Stunden vollständig. Die Reduktion wird, wie beschrieben, durch tropfenweise Zugabe von 10 mmol (2,0g) an 1-[4-(2-Methylpropyl)]-phenyl-ethanon ausgeführt und die Lösung 8 Stunden lang der Reaktion unterzogen. Die Reaktionszeit kann hinsichtlich der Reduktion zum entsprechenden S-(-)-Carbinol durch schnelles Mischen in Gegenwart der Molekularsiebe ohne Erhöhung der Temperatur über 20°C verringert weden. Die Verarbeitung des S-(-)-Carbinols verläuft wie oben beschriebenen.

Die optische Reinheit wurde mit 97% bestimmt, und zwar mit NMR-Verfahren, und die chemische Ausbeute an reinem Produkt liegt bei beinahe 95%.

Die Reduktion des Ketons kann auch in protonenfreien Lösungsmitteln, z.B. Benzol, Toluol oder Hexan, durchgeführt werden. Um gute chemische Ausbeuten der enantiomeren Carbinole zu erreichen, ist es notwendig, diese Reaktionen unter kräftigem Rühren in Gegenwart von Molekularsieben und Glasperlen durchzuführen. Die Reaktionszeiten, die Molekularsiebe zur Zugabe zu den asymmetrischen Ketonen, das Verhältnis R*OH zu LiAlH₄ und die Temperaturbedingungen sind die gleichen, wie sie oben beschrieben wurden.

Die oben beschriebene Reduktion kann in gut gerührten, kontinuierlichen Tankreaktoren durchgeführt werden, weil sie insbesondere für Flüssigphasen-Reaktionen zur Herstellung im großen industriellen Maßstab geeignet ist. Man erhält eine konsistente Produktqualität (optische Reinheit), die automatische Kontrolle ist einfach und die einzusetzende menschliche Arbeitskraft ist gering. Da in einem Rühr-Tankreaktor die Reaktanten, z.B. R*OH.LiAlH₄ und das Keton sofort nach Eintreten in den Behälter verdünnt werden, was die gewünschte Reaktion (konstantes Verhältnis von LiAlH₄ R*OH) fördert, und die Bildung von Nebenprodukten unterdrückt, können das Volumen und die Temperatur des Tanks leicht kontrolliert werden, so daß das Auftreten von "hot spots" weniger wahrscheinlich wird, und zwar insbesondere in Gegenwart von Molekularsieben, wenn das stetige Rühren gut angepaßt ist.

Die chemischen Ausbeuten liegen im Bereich von 85 - 98% in Abwesenheit von Feuchtigkeit und einer Hochvakuum-Destillation der enantiomeren Carbinole.

### BEISPIEL 3

### Reduktion des Ketons mit (-) 2,2-Dihydroxy-1,1-dinaphthyl zu S- (-)-2-(-4-Isobutylphenyl)-2-hydroxy-ethan

Zu einer 1,5M THF-Lösung von LiAlH₄ (8,0 mmol) unter Stickstoffatmosphäre in Gegenwart von Molekularsieben (0,2g 4A Zeolithe) wird Ethanol in THF (2M, 8,50 mmol) bei 0°C hinzugegeben. Diese Lösung wird stetig gerührt, während S-(-)-2,2′-Dihydroxy-1,1-dinaphthyl-Reagenz (8,5 mmol) THF (0,64 mmol) bei 0°C hinzugegeben wird. Nach Zugabe des S-(-)-2,2′-Dihydroxy-1,1-dinaphthyl-Reagenz bei 0°C wird die Lösung stetig 1 Stunde lang bei 20°C gerührt, ohne daß sich ein weißes Präzipitat bildet, was normalerweise beobachtet wird. Das gebildete chirale Reagenz wird auf -20°C abgekühlt und 2,50 mmol an 1-[4-(2-Methylpropyl)]-phenyl-ethanon, gelöst in THF (1M-Lösung), wird unter stetigem Mischen hinzugegeben und 8 Stunden lang bei -20°C unter stetigem Rühren gehalten. Die Reaktion wird durch Zugabe von 1,0N HCl bei -20°C abgestoppt, das chirale Reagenz wiedergewonnen und die Aufarbeitung mit Ether, gefolgt durch eine Destillation, lieferte optisch reines S-(-)-(4-Isobutylphenyl)-2-hydroxy-ethan, 310 mg, 81%, in einer optischen Ausbeute von fast 95 % in enantiomeren Überschuß und Konfiguration. Das chirale Reagenz kann nach Rekristallisation aus Benzol ohne jede bemerkenswerte Razemisierung wiederverwendet werden.

### BEISPIEL 4

### Herstellung von S-(+)-2-[4-Isobutylphenyl]-propionsäure

10 g S-(-)-2-[Isobutylphenyl]-2-hydroxy-ethan (56 mmol) werden in 20 ml 1,4-Dioxan bei 20°C in Gegenwart von 4A Molekularsieben unter Rühren gelöst. 5,0 ml SO₂Cl₂ (=60 mmol), gelöst in 5 ml 1,4-Dioxan, wird tropfenweise unter stetigem Rühren über eine Zeitdauer von 10 Minuten hinzugegeben, wobei die Temperatur bei 20°C gehalten wird. Nach einer Stunde ist die Reaktion vollständig und das Thionylchlorid wird durch Verdampfung, wobei N₂ durch die Lösung geleitet wird, wiedergewonnen. Das S-(-)-2-[4-Isobutylphenyl]-2-chlor-ethan muß nicht abgetrennt werden, weil die Lösung sofort für die Metallierung mit Mg oder Hg (OOC CH₃)₂ wiederverwendet wird. Zu dieser Lösung, enthaltend 11g an S-(-)-2-(-)-4-Isobutylphenyl-2-chlor-ethan wird 1,40g Mg (0,055 M) in Gegenwart von Jod bei 0°C hinzugegeben; nach 10 - 30 Minuten setzt eine heftige Reaktion ein, so daß manchmal eine Abkühlung erforderlich ist, um eine Wurtz-Synthese und die Bildung von Biradikalen zu vermeiden. Die Farbe der Lösung wechselt am Ende der Reaktion von hellgelb zu hellbraun, wenn Kohlendioxid bei 0 - 5°C unter stetigem Rühren durch die Reaktionsmischung geleitet wird. Die Grignard-Verbindung, die bei Verwendung von SO₂Br₂ S-(+)-2-[4-Isobutylphenyl]-chlorethan oder ein Bromid ist, wird durch Et₂O oder THF (oder Benzol, Toluol) verdünnt, wenn durch die Lösung unter stetigem Rühren trockenes CO₂ geleitet wird, was für das Erzielen hoher chemischer Ausbeuten an optisch reinem S-(+)-Ibuprofen essentiell ist. Die stetige Zugabe von CO₂ zur S-Grignard-Verbindung und die Herstellung der S-Carbonsäure macht es notwendig, trockenes 1,4-Dioxan kontinuierlich hinzuzugeben, während sich die S-Carbonsäure entwickelt und das Lösungsmittel sättigt. Nach 20 Minuten ist die Reaktion vollständig, wird von den festen Rückständen abgetrennt und einer Hochvakuumdestillation unterworfen. Die Lösung wird konzentriert und bei 2 mm Hg (0,06 - 2 mm Hg) und 120° - 98°C destilliert, um 9,30g (80%) an S-(+)-Ibuprofen zu ergeben: NMR (CDCl₃) S 0,91 (d,J=7H, 6H), 1,50 (d,J=8Hz, 3H) 1,84 (nonet, 1H), 2,96 (brd, 27H7, 2H), 3,72 (g, 1H), 7,01 - 7,32 (AA'BB', 4H), 9,78 (br. sl H). [α]_{O} 25, +58° (95%, EtOH).

### BEISPIEL 5

### Herstellung von R-(-)-2-[Isobutylphenyl]-2-propionsäure

Hier kann das gleiche Verfahren angewandt werden, wie es in Beispiel 3 beschrieben wurde. Jedoch kann das R-(+)-2-[4-Isobutylphenyl]-chlor-ethan leicht aus S-(-)-2-[4-Isobutylphenyl]-2-hydroxy-ethan durch die Umsetzung mit SO₂Cl₂ in Pyridin in Gegenwart von Wasser hergestellt werden. 10g von S-(-)-2-[4-Isobutylphenyl]-2-hydroxy-ethan (56 mmol) werden in 15 g Pyridin, enthaltend 10% (w/w) Wasser bei 20°C gelöst. Unter stetigem Mischen werden 6,7g SO₂Cl₂ (äquivalent zu 4,1 ml) zugegeben und 20 Minuten lang refluxiert. Nach der Entfernung des Überschusses an SO₂Cl₂ und Pyridin (Siedepunkt 116°C, 760 mmHg) wird das Chlorid mit 6 mm Hg (Siedepunkt 98,3°C) destilliert, um 9,59g an R-(+)-2-[4-Isobutylphenyl]-2-chlor-ethan (86,6%) zu ergeben: NMR (C Cl₄) 0,90 (d,J,7Hz, 6H), 1,84 (d,J7Hz, 3H), 1,86 (nonet, 1H), 2,48 (d,J=7Hz, 2H), 5,15 (q, 1H), 7,10-7,44 (AA'BB'4H). Analyse : kalkuliert für C₁₂H₁₇jCl : C 73, 26; H, 8,7, Cl, 18,01, gefunden : (73,40%H : 8,79% Cl 18,09% [α]_{D}25-29,5° (C 1,9%, (CCl₄);

Das entsprechende Grignard-Reagenz (0,9M - 1,5M) in Et₂O wird mit ungefähr 80% Ausbeute durch die langsame Zugabe einer Lösung des Halogenids in Et₂O zum Magnesium bei 4°C hergestellt, wie es oben in Beispiel 3 beschrieben wurde. Das Verfahren zur Carbonisierung oder Merkurisierung in Gegenwart von H₁ (OOC CH₃)₂, [Hg (CH)₂]₂ oder HgCl₂ ist ähnlich dem in Beispiel 4 beschriebenen. Die chemische Ausbeute an R-(-)-Ibuprofen beträgt 78% und die optische Reinheit fast 98%.

### BEISPIEL 6

### Sythese von S-(+)-Ibuprofen über Nitril und nachfolgender Hydrolyse

10 g R-(+)-2-[4-Isobutylphenyl]-2-chlor-ethan (50,5 mmol) werden in 25 ml EtOH und 15 ml Wasser gelöst und mit 2,95g (60 mmol) Natriumcyanid, gelöst in 10 ml Wasser, unter tropfenweiser Zugabe der Cyanidlösung bei stetigtem Rühren umgesetzt. Die Mischung wird für 1 Stunde refluxiert und auf 20°C abkühlengelassen. Das präzipitierte Natriumchlorid wird abgefiltert und der Überstand, enthaltend Wasser und EtOH, getrocknet und das EtOH aus der verbleibenden Flüssigkeit, welche das S-(+)-2-[4-Isobutylphenyl]-2-ethyl-cyanid enthält, destilliert (die chemische Ausbeute beträgt 88%). Dieses S-(+)-Cyanid wird im 15 ml EtOH und 30 ml Wasser, welches 9g (0,45 mol) Natriumhydroxid und 10 % (w/w) H₂O₂ enthält, gelöst und unter Rückflußbedingungen 1 Stunde lang erhitzt. Nach dem Abkühlen auf Raumtemperatur wird die Reaktionsmischung mit 100 ml Wasser verdünnt, bis sich eine klare und transparente Lösung ergibt. Diese Lösung wird auf 0°C abgekühlt und anschließend 100 ml verdünnter Salzsäure hinzugegeben, wenn S-(+)-Ibuprofen in Form kleiner Kristalle präzipitiert. Die S-(+)-Ibuprofen-Kristalle werden gesammelt, mit verdünnter Salzsäure gewaschen und über Ca Cl₂ getrocknet. Die chemische Ausbeute beträgt 94 % und der Schmelzpunkt lag bei 51 - 54°C [α]_{D} + 60° (95% EtOH).

### BEISPIEL 7

### Synthese von S-(+)-Ibuprofen aus R-(+)-2-[4-Isobutylphenyl]-2-chlor-ethan mit Natriumtetracarbonyl-ferrat und Kohlenmonoxid

10 ml R-(+)-2-[4-Isobutylphenyl]-2-chlor-ethan (50,5 mmol) werden in 150 ml Dimethylformamid (DMF) (0,033M) unter schnellem Mischen und einem N₂-Strom gelöst und 10,8g Natriumtetracarbonyl-ferrat-II, welches frisch durch die Behandlung von Eisen-pentacarbonyl Fe(CO)₅ mit Natriumamalgam und THF bei 20°C hergestellt wurde, unter stetigem Mischen hinzugegeben. Die Lösung wird auf 10°C abgekühlt und Kohlenmonoxid durch die Lösung geströmt. Normalerweise ist die Reaktion nach 1 - 2 Stunden beendet, und zwar abhängig von der Temperatur und den Lösungsmitteln (THF, DMF, DMSO); dies kann jedoch einfach beobachtet werden, wenn ein Überschuß an Kohlenmonoxid aus der Lösung in Gegenwart von N₂ entweicht. Die oxidative Spaltung zur entsprechenden S-(+)-2-[Isobutylphenyl]-propionsäure wird durch die Zugabe einer wäßrigen Lösung an Natriumhypochlorid mit nachfolgender Zugabe von 0,1M Salzsäure erreicht, wobei die Reaktionstemperatur bei 10°C gehalten wird. Es muß beachtet werden, daß genügend Salzsäure hinzugegeben wird, da der größte Teil der Protonen für die Präzipitation des S-(+)-Ibuprofens in der wäßrigen Lösung für die Wiedergewinnung der freien Säure verwendet wird.

Das entsprechende Amid von S-(+)-Ibuprofen kann hergestellt werden durch die Verwendung von Triphenylphosphin (Ph₃P) anstelle des Kohlenmonoxides in Gegenwart von Natriumtetracarbonyl-ferrat (II)(Na₂Fe(CO)₄). 10g von R-(+)-2-[4-Isobutylphenyl]-2-chlor-ethan werden in 30 ml Benzol in Gegenwart von 10,8g Natriumtetracarbonyl ferrat-(II) bei 20°C dispergiert. 13,4g Triphenylphosphin (0,051 mol), gelöst in Benzol, werden tropfenweise während einer Zeitdauer von 20 Minuten unter N₂ Atmosphäre zugegeben. Die Mischung wird unter stetigem Rühren 3 Stunden lang refluxiert; die Reaktionsmischung wird für 1 Stunde bei 20°C stehengelassen mit anschließendem "Quenschen" der Reaktion mit Methylbenzylamid. Die kleinen Kristalle an S-(+)-Ibuprofen-methyl-benzylamid werden abfiltriert, aus THF/DMF rekristallisiert und durch HPLC-Verfahren auf ihre optische Reinheit analysiert. Die HPLC-Analyse zeigt die Gegenwart von 98% des Diastereoisomers entsprechend der S-2-(4-Isobutylphenyl)-propionsäure bei Retentionszeiten von 2,79 Minuten und 2% des Diastereomers, entsprechend der R-7-(4-Isobutylphenyl)-propionsäure bei Retentionszeiten von 2,38 Minuten. Die chemischen Ausbeuten bei der Herstellung der S-2-Carbonsäure aus dem entsprechenden R-(+)-[4-Isobutylphenyl]-2-chlor-ethan sind, in Gegenwart von Kohlenmonoxid, fast 95% mit einer optischen Reinheit von 95-98% bzw. 90% in Gegenwart von Triphenylphosphin.

### BEISPIEL 8

### Herstellung von Komplexen zwischen S-(+)-Ibuprofen und 1-Amino-1-desoxy-D-glucit

206,27 (250,0) mg S-(+)-Ibuprofen und 236,72 (181,19) mg 1-Amino-1-desoxy-D-glucit werden in 6 ml Wasser gelöst und anschließend bei 45°C eine Stunde lang Ultraschall-behandelt. Die klare Lösung kann aufbewahrt und nach Sterilisation für medizinische Zwecke verwendet werden. Der Komplex kann aus der etherischen oder der alkoholischen Lösung kristallisiert werden durch Zugabe dieser Lösungsmittel bei 20°C unter ständigem Rühren zu einer wäßrigen Lösung aus S-(+)-Ibuprofen und 1-Amino-1-desoxy-D-glucit (pH 7,5). Das mikrokristalline Präzipitat kann durch Filtration mit anschließender Trocknung über CaCl₂ unter N₂-Atmosphäre gesammelt werden. Falls keine kristalline Formen erwünscht sind, kann das mikrokristalline Präzipitat zusätzlich zentrifugiert werden, der Überstand wird verworfen und das Präzipitat über P₂O₅/CaCl₂ bei 30°C getrocknet; der Schmelzpunkt des amorphen Komplexes ist 61°C, der der kristallinen Probe 59°C; bei Verwendung anderer Präzipitations-Lösungsmittel, z.B. Aceton oder Alkyl-aryl-ketonen, DMF und Petrolether, wurden unterschiedliche kristalline Formen beobachtet, was einen bestimmten Grad an Polymorphismus dieser speziellen Komplexe offenbart.

### BEISPIEL 9

### Synthese von S-(+)-2-(6-Methoxy-2-naphthyl)-propionsäure

Sehr gute chemische Ausbeuten (80%) dieser Verbindung werden in hoher optischer Reinheit (95%) gemäß den in den Beispielen 4 und 5 aufgezeigten Wegen erhalten, insbesondere bei Verwendung von Collman's-Reagenz in Gegenwart von Kohlenmonoxid mit anschließender Hypochlorit-Oxidation.

Die Rekristallisation des Ausgangsmaterials mit einem Schmelzpunkt von 252-253°C ergibt kristalline Objekte mit einem Schmelzpunkt von 154°C (genauer S.p.152-154°C); [α]_{D} 25 + 64,5° (C-1,08 CHCl₃), NMR (CHCl₃); 1,6 (D, 3H, CH-CH₃); 3,92 (S, 3H, OCH₃), 3,88 (g, 1H, CH) und 7-7,9 (m, 6H, aromatisch).

MS resultierte in den folgenden Spektra (FAB, Glycerin-Matrix): m/z = 23, [M + H]⁺, 185 [M-HCOOH+H]⁺, 323 [M+6+H]⁺, 115 [6+Na]⁺ und 229 [M-H]⁻ mit 6 = Glycerin.

Messungen der optischen Reinheit dieser Verbindung wurden durch die Umwandlung der Carbonsäure zum entsprechenden Amid unter Verwendung von S-(-)-Methylbenzylamin, wie oben beschrieben, vorgenommen, wobei HPLC-Techniken verwendet wurden, welche die folgenden Ergebnisse ergaben :

Die chromatographische Zusammensetzung der gebildeten Diastereomere ist 3,6% R-2-(6-Methoxy-2-naphthyl)-propionamid (6,15 min) und 96,4% S-2-(6-Methoxy-2-naphthyl)-propionamid (6,95 min).

### BEISPIEL 10

### Synthese von 2-S-(+)-(5-Brom-6-methoxy-2-naphthyl)-Propionsäure, als Methylester

Nach der stereospezifischen Reduktion des Ketons, beschrieben in den Beispielen 1 und 2, mit (+)-(2S,3R)-4-Dimethyl-amino-3-methyl-1-2-diphenyl-2-butanol und LiAlH₄ zum entsprechenden R-Carbinol, anschließend umgewandelt zum R-Halogenid und Behandlung mit Natriumtetracarbonyl-ferrat-II in Gegenwart von Triphenylphosphin, entsteht die entsprechende Carbonsäure in einer chemischen Ausbeute von 75% und einer optischen Reinheit von fast 95%. Der Schmelzpunkt wurde mit 168°C bestimmt, [α]_{D}20 = +42,7 (0,8%) in Chloroform. Der Methylester wird leicht durch die Umsetzung der Carbonsäure mit Diazomethan erhalten, mit nachfolgender Verdampfung des Lösungsmittels unter reduziertem Druck, wobei der optisch reine 2-S-(+)-(5-Brom-6-methoxy-2-naphthyl)-propionsäure methylester entsteht.

Der Schmelzpunkt beträgt 96°C; [α]_{D} 20 + 52,5 (c = 0,5, CHCl₃). Das Produkt ist gemäß ¹H-NMR (200 MHz)-Analyse optisch rein; die Analyse wurde in CHCl₃ ausgeführt, wobei, wie oben beschrieben, ein optisch aktives Verschiebungsreagenz verwendet wurde (EuIII-trix [3-Heptafluorpropylhydroxy-methylen)-d-camphorat].

### BEISPIEL 11

### Synthese von R-(-)-2-(5-Brom-6-methoxy-2-naphthyl)-propionsäure

Die Durchführung der Reduktion des Ketons mit dem (-)-2,2-Dihydroxy-1,1′-dinaphthyl-LiAlH₄-ROH-Komplex in der in Beispiel 2 beschriebenen Weise in Gegenwart von Molekularsieben ergibt ein optisch reines S-2-(5-Brom-6-methoxy-2-naphthyl)-hydroxy-ethan (98%) mit fast quantitativer Ausbeute.

Bei Durchführung der Synthese über das Nitril mit nachfolgender Oxidation zur entsprechenden Carbonsäure entstand bei fast 75% chemischen Ausbeuten die optisch reine 2-R-(-)-(5-Brom-6-methoxy-2-naphthyl)-propionsäure mit einem Schmelzpunkt von 168°C, [α]_{D} = -42,0 (c = 0,6%, Chloroform).

### BEISPIEL 12

### Synthese von 2-S-(+)-(4-Chlorphenyl)-3-methylbutansäure aus 1-(4-chlorphenyl)-3-methylbutan-1-one

Das Keton kann aus 3-Methyl-butyryl-chlorid (128,6g = 1,07 mol) durch Friedel-Crafts-Reaktion mit Aluminiumchlorid (153,7 g= 1,15 mol) in Methylenchlorid unter stetiger Hinzugabe von Chlorbenzol (100 g = 0,80 Mol) hergestellt werden. Die stereospezifische Reaktion dieses Keton mit (+)-(2S,3R)-4-Dimethylamino-3-methyl-1,2-diphenyl-2-butanol in Gegenwart von LiAlH₄ wird, wie im Beispiel 1 beschrieben, bei 20°C durchgeführt. Das R-Carbinol, welches bezüglich der optischen Reinheit zu 90% rein ist, wird mit SO₂Cl₂ und Pyridin zum entsprechenden R-Halogenid umgewandelt und anschließend mit Natriumtetracarbonyl-ferrat-II in Gegenwart von Kohlenmonoxid, wie im Beispiel 5 beschrieben, behandelt.

Die erhaltene Roh-Carbonsäure wird über DEAE-Sephadex-A50 aufgereinigt, wobei ein linearer Gradient bei pH 7,9 verwendet wird, und zwar in einem Bereich von 0,001M K₂HPO₄ bis 0,01M K₂HPO₄ in einem Gesamtvolumen von 1000 ml. Die die optisch aktiven Materialien (+) enthaltenden Fraktionen werden vereinigt, lyophilisiert und auf ihre optische Reinheit untersucht. Die gesamt-chemische Ausbeute beträgt 71%, [α]_{D} + 39,7 (c = 1 %, Chloroform).

### BEISPIEL 13

Die entsprechenden Biphenyl- und Phenoxy-Propionsäure-Derivate können gemäß den Verfahren der Beispiele 1-5 hergestellt werden. Im folgenden sind einige Verbindungen aufgeführt, welche in Übereinstimmung mit den in den Beispielen 1-5 beschriebenen Wegen hergestellt werden können, wobei auch die optische Aktivität und die chemischen Ausbeuten angegeben sind.

S-(+)-2-(2-Fluor-4-biphenyl)-propionsäure, [α]^{D} = +44,7, chemische Ausbeute 80%, S-(+)-2-(2-[4-(2-Fluorphenoxy)-phenyl])-propionsäure, mit [α]^{D} = +49 und einer chemischen Ausbeute von 70%, und S-(+)-2-(2-Hydroxy-4-biphenyl)-propionsäure, [α]^{D} = + 47.

### BEISPIEL 14

### Herstellung von festen Schmelzen von S-(+)-Ibuprofen und Polyethylenglycol 1500

500 g Polyethylenglycol 1500 werden in Abwesenheit von Wasser in einem Behälter bei 55°C+/-3°C unter stetigem Rühren geschmolzen. 500 g festes S-(+)-Ibuprofen wird zusätzlich unter stetigem Rühren hinzugegeben. Es ist auch möglich, 500 g Polyethylenglycol 1500 mit 500 g S-(+)-Ibuprofen zu mischen und die Mischung in einem Behälter unter Rühren bei 55°C zu schmelzen. Es bildet sich eine klare, flüssige Schmelze, welche bei Betrachtung unter Tageslicht transparent ist, wenn die flüssige Schmelze auf 40°C abgekühlt ist. Diese Flüssigkeit (40°C) kann leicht in jeder gewünschten Dosierungsform in Hartgelatinekapseln eingefüllt werden. Nach der geeigneten Füllung werden die Hartgelatinekapseln bei etwa 32°C stehengelassen, wobei ein Festkörper entsteht, welcher nicht mehr abgedichtet werden muß.

Um ein schelles Aushärten der flüssigen Bestandteile der Mischung zu erreichen, können Kristallkeime von entweder S-(+)-Ibuprofen oder (R,S)-Ibuprofen hinzugegeben werden, um die Zahl an Kristallkeimbildungsstellen zu erhöhen.

## Patentansprüche

1. Verfahren zur Herstellung einer pharmazeutisch aktiven Verbindung in stereospezifischer Form, ausgewählt aus der Gruppe von Verbindungen mit der Formel : 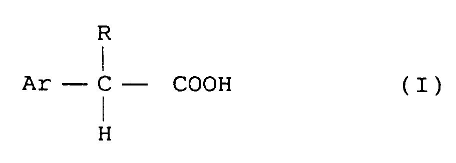 und deren physiologisch verträglichen Salzen und Estern, wobei R ein C₁ - C₉-Alkyl und Ar eine monozyklische, polyzyklische oder orthokondensierte polyzyklische aromatische Gruppe mit bis zu 12 Kohlenstoffen im aromatischen Ring ist, wobei der aromatische Ring substituiert oder nicht substituiert sein kann, bestehend aus den Schritten :
a) Umsetzen eines Carbonylsubstrats der Formel : 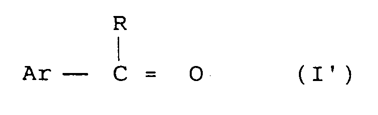 wobei R und Ar den oben gegebenen Bedeutungen entsprechen, mit einem stereospezifischen Reagenz in Gegenwart eines Reduktionsmittels, eines organischen Losungsmittels und eines Molekularsiebs zum enantiomeren R- oder S-Karbinol und
b) Umsetzen des enantiomeren R- oder S-Karbinols mit SO₂X₂ oder SOX₂ oder Cyanurchlorid, wobei X = Cl oder Br ist, zum entsprechenden enantiomeren R- oder S-Halogenid, und Umsetzen des Halogenids mit
(i) Magnesium in etherischer oder THF-Lösung, unter Beibehaltung der S- oder R-Konfiguration, zur entsprechenden R- oder S-metallorganischen Grignard-Verbindung und Durchströmen der die Grignard-Verbindung enthaltenden Lösung mit CO₂; oder
(ii) einer Alkyl- oder organometallischen Verbindung in Gegenwart von CO₂; oder
(iii)Natriumtetracarbonylferrat (II) (Na₂Fe(CO)₄) in Gegenwart von Triphenylphosphin (Ph₃P), wobei als Zwischenprodukt 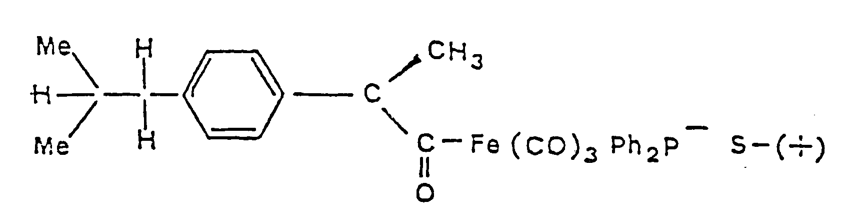 entsteht, welches anschließend mit Jod-Wasser oxidiert wird; oder
(iv) Natriumtetracarbonylferrat (II) (Na₂Fe(CO)₄) in Gegenwart molarer Mengen von Triphenylphosphin (Ph₃P) und eines sekundären Amins zum entsprechenden Amid, das anschließend hydrolysiert wird; oder
(v) (Na₂Fe(CO)₄) in Gegenwart von CO, wobei als Zwischenprodukt 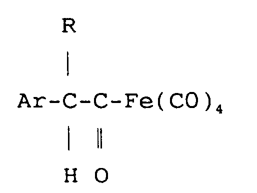 entsteht, das anschließend mit Sauerstoff oder NaOCl oxidiert und anschließend mit Säure hydrolysiert wird; oder
(vi) Nickelcarbonyl (Ni(CO)₄) in Gegenwart eines Alkohols und seiner konjugierten Base gemäß der nachfolgenden Reaktion 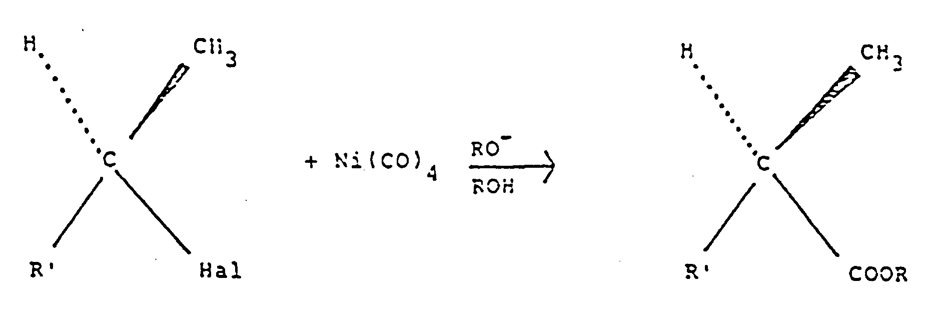 wobei
Hal = Halogenid;
ROH = Alkohol;
R' = ein Arylrest, wie oben näher definiert;
oder
(vii) in Wasser gelöstem Alkalizyanid und Umsetzung des Produkts mit einer Base und Wasserstoffperoxid; oder
(viii) Alkalizyanid mit nachfolgender Säurehydrolyse, um die entsprechende Säure (I) zu bilden.

2. Verfahren nach Anspruch 1,
dadurch gekennzeichnet,
daß R ein C₁ - C₄, bevorzugt ein C₁-Alkyl ist und die aromatische Gruppe eine Phenyl-, Diphenyl- oder Naphthylgruppe ist, wobei die Substituenten dieser aromatischen Gruppen aus einem oder mehreren Halogenatomen, C₁ - C₄ Alkylen, Benzyl-, Hydroxy-, C₁ - C₂ Alkoxy-, Phenoxy- und Benzoyl-Gruppen bestehen können,
daß Ar aus der Gruppe , bestehend aus
4-Isobutylphenyl
6-Methoxy-2-naphthyl
3-Phenoxy-phenyl
2'-Fluor-4-diphenyl
4'-Fluor-4-diphenyl
5-Chlor-6-methoxy-2-naphthyl
5-Brom-6-methoxy-2-napthyl
4-Chlor-phenyl
4-Difluor-methoxy-phenyl
6-Hydroxy-2-naphthyl und
5-Brom-6-hydroxy-2-naphthyl
ausgewählt wird, und bevorzugt,
daß das Carbonylsubstrat die Formel besitzt : 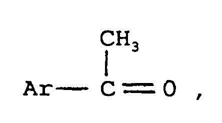 und bevorzugt,
daß das stereospezifische Reagenz (+)-4-Dimethylamino-3-methyl-1,2-diphenyl-2-butanol oder (1S,2S)-(+)-2-amino-1-phenyl-propan-1,3-diol oder (1R,2R)-(-)-2-amino-1-phenyl-propan-1,3-diol oder 2,2'-Dihydroxy-1,1'-dinaphthyl oder S-(-)-1,1-Diphenylprolinol ist,
wobei für das stereospezifische Reagenz die Reduktionsmittel im Zusammenhang mit den chiralen Komplexmitteln ausgewählt werden aus der Gruppe, umfassend:
a) LiAlH₄, LiBH₄, NaBH₄ sowie KBH₄, wobei LiAlH₄ bevorzugt wird,
b) NaAlH₄, AlH₃.THF, Mg(AlH₄)₂, BH₃.THF
c) Al(BH₄)₃.THF; Ca(BH₄)₂.THF; Sr(BH₄)₂.Et₂O; Ba(BH₄)₂-Et₂O(THF), wobei bevorzugt
das stereospezifische Reagenz einen Komplex aus BH₃.THF und S-(-)-1,1-Diphenyl-Prolinol oder
einen Komplex aus LiAlH₄ und (+)-4-Dimethylamino-3-methyl-1,2-diphenyl-2-butanol oder einen Komplex aus LiAlH₄ und 2,2'-Dihydroxy-1,1'-dinaphthyl bildet.

3. Verfahren nach Anspruch 1 oder 2,
dadurch gekennzeichnet,
daß das organische Lösungsmittel Tetrahydrofuran (THF) oder 1,2-Dimethoxyethan oder ein Ether, bevorzugt ein Diethylether ist
und daß, bevorzugt als Molekularsiebe Zeolithe verwendet werden,
die bevorzugt einen bestimmten Porenkanal besitzen, der durch ein 12 Ring, 10 Ring oder 8 Ring begrenzt wird, wobei die Molekularsiebe eine große Kapazität für Wasser und Sauerstoff haben,
sodaß bevorzugt die Adsorptionskapazität für Wasser zwischen 10 - 20 cm³/100 g aktivierter Molekularsiebe und die Adsorptionskapazität für Sauerstoff vorzugsweise bei 10 - 14 cm³/100 g aktivierter Molekularsiebe liegt, wobei weiterhin bevorzugt als Molekularsieb ein ZSM-5 Zeolith eingesetzt wird und bevorzugt
das Verhältnis von Molekularsieben zur stereospezifischen Katalysatormenge zwischen 1 - 15 g pro mmol, bevorzugt zwischen 2 - 10 g pro mmol, und weiterhin bevorzugt 2 g pro mmol, und insbesondere bevorzugt, daß das Verhältnis von ZSM-5 Zeolith zur stereospezifischen Katalysatormenge 5 g pro mmol oder 10 g pro mmol beträgt.

4. Verfahren nach einem oder mehreren der vorhergehenden Ansprüche,
dadurch gekennzeichnet,
daß das Carbonylsubstrat nach der Bildung des LiAlH₄-Komplexes zugegeben wird und bevorzugt, daß das stereospezifische Reagenz mit dem Lithiumaluminiumhydrid in der organischen Lösung als Komplex vorliegt und bevorzugt,
daß zur Bildung des LiAlH₄-Komplexes zu einem organischen Lösungsmittel unter Rühren ein stereospezifisches Reagenz und LiAlH₄ zugegeben werden, wobei die Bildung des LiAlH₄-Komplexes in Gegenwart der Molekularsiebe erfolgt.

5. Verfahren nach einem oder mehreren der vorhergehenden Ansprüche,
dadurch gekennzeichnet,
daß zur Bildung des LiAlH₄-Komplexes zu 10 bis 100 ml eines organischen Lösungsmittels 1,0 bis 100 ml eines stereospezifischen Reagenz und 0,5 bis 15 g, bevorzugt 0,5 bis 10 g, insbesondere bevorzugt 0,5 bis 5 g eines Molekularsiebes zugegeben und bei -60 bis 22°C gelöst werden und zu dieser Lösung bei -60 bis 22°C unter stetigem Rühren 10 bis 100 ml einer 1,0 bis 2,0 M Lösung von LiAlH₄ hinzugegeben werden, wobei bei einer Geschwindigkeit von 10 - 20 Upm solange weiter gerührt wird, bis sich eine homogene Suspension bildet,
wobei bevorzugt als Reduktionsmittel zur Komplexbildung eine der in Anspruch 2 gekennzeichneten Verbindungen verwendet wird und weiterhin bevorzugt die homogene Suspension bei einer Temperatur von -60 bis 22°C 5 bis 100 Min. refluxiert wird.

6. Verfahren nach einem oder mehreren der vorhergehenden Ansprüche,
dadurch gekennzeichnet,
daß der LiAlH₄-Komplex nach seiner Bildung 5 bis 100 Min. bei einer Temperatur von 0 bis - 60°C stehengelassen oder 5 bis 100 Min. gerührt wird, um bei der Umsetzung des Carbonylsubstrats die R-(-)-Form des Carbinols zu erhalten, oder
daß der LiAlH₄-Komplex nach seiner Bildung 10 bis 100 Min. bei einer Temperatur von 0 bis 22°C stehengelassen oder 10 bis 100 Min. gerührt wird, um die S-(+)-Form des Carbinols zu erhalten, oder
daß der LiAlH₄-Komplex 8 Stunden lang in etherischen oder THF-Lösungen bei Temperaturen zwischen -7°C bis 0°C stehengelassen wird, bevor das Carbonylsubstrat zugegeben wird, um das S-Enantiomer des Carbinols zu erhalten, oder daß die Bildung des LiAlH₄-Komplexes bei einer Temperatur von 0°C bis -60°C erfolgt und 0 bis 10 Min. nach Bildung des Komplexes das Carbonylsubstrat zugegeben wird, um das R-Enantiomer des Carbinols zu erhalten, wobei bevorzugt die Umsetzung des Carbonylsubstrats in Abwesenheit von Sauerstoff und Wasser durchgeführt wird.

7. Verfahren nach einem oder mehreren der vorhergehenden Ansprüche,
dadurch gekennzeichnet,
daß das Molverhältnis von LiAlH₄ zum stereospezifischen Reagenz zwischen 1,0 : 0,2 bis 1,0 : 3,0 liegt, bevorzugt daß das Molverhältnis von LiAlH₄ zum stereospezifischen Reagenz zwischen 1,0 : 2,3 bis 1,0 : 2,5 liegt, weiterhin bevorzugt,
daß das Molverhältnis des LiAlH₄-Komplexes zum Carbonylsubstrat 1,0 : 0,2 - 1,0 : 2,5 beträgt, ebenfalls bevorzugt
daß das Molverhältnis des LiAlH₄-Komplexes zum Carbonylsubstrat 1,0 : 2,5 beträgt, oder
daß das Molverhältnis von LiAlH₄ zum stereospezifischen Reagenz 1,0 zu 0,7 beträgt, oder
daß das Molverhältnis von LiAlH₄ zum stereospezifischen Reagenz 1,0 zu 1,0 beträgt, oder
daß das Molverhältnis von LiAlH₄ zum stereospezifischen Reagenz 1,0 zu 2,0 beträgt, oder
daß das Molverhältnis von LiAlH₄ zum stereospezifischen Reagenz 1,0 zu 2,5 beträgt, oder
daß das Molverhältnis von LiAlH₄ zum stereospezifischen Reagenz 1,0 zu 3,0 beträgt, und daß vorzugsweise als organisches Lösungsmittel Benzol oder Toluol oder Pentan oder Hexan verwendet wird, wenn Methoxy- und/oder Chlor- und/oder Brom- und/oder Fluor-Substituenten in den Arylgruppen des Carbonylsubstrats lokalisiert sind.

8. Verfahren nach einem oder mehreren der vorhergehenden Ansprüche,
dadurch gekennzeichnet,
daß die optische Reinheit / Aktivität des Halogenids wenigstens 80 % ist, bevorzugt
daß das S-Carbinol mit SO₂Cl₂, SO₂Br₂, SOBr₂, SOCl₂ oder Cyanurchlorid zum S-Halogenid umgewandelt wird, welches zu wenigstens 95 - 98 Gew.-% die S-Konfiguration des Halogenids aufweist, oder
daß das R-Carbinol durch SO₂Cl₂, SO₂Br₂, SOCl₂, SOBr₂ oder Cyanurchlorid zum R-Halogenid umgewandelt wird, welches zu wenigstens 95 -98 Gew.-% die R-Konfiguration des Halogenids aufweist, oder
daß das R- oder S-Carbinol durch SO₂Cl₂ oder SO₂Br₂ oder SOCl₂ oder SOBr₂ in Gegenwart von Pyridin und H₂O zum S-oder R-Halogenid umgewandelt wird, wobei wenigstens 95 - 98 Gew.-% der Halogenide in S- oder R-Konfiguration als Ausbeute erhalten werden.

9. Verfahren nach einem oder mehreren der vorhergehenden Ansprüche,
dadurch gekennzeichnet,
daß die stereospezifische Halogenierung in wasserfreiem 1,4-Dioxan oder in trockenem Pyridin in Gegenwart von Thionylchlorid oder Thionylbromid oder Cyanurchlorid durchgeführt wird, weiterhin bevorzugt daß das enantiomere Carbinol in Gegenwart von pulverisiertem Cyanurchlorid (1 Mol) oder in Gegenwart einer Base auf 10°C - 20°C über den Siedepunkt der Carbinole erhitzt wird, und nach 1 bis 1,5 Stunden dieses Reaktionsgemisch abgekühlt, gefiltert und unter Hochvakuum destilliert wird, worauf die Metallierung und Carbonisierung erfolgen, weiterhin bevorzugt daß die stereospezifische Halogenierung mit entsprechendem molarem Thionylchlorid oder Thionylbromid bei einer Temperatur von -10 bis -20°C, bevorzugt -20°C durchgeführt wird, wobei anschließend die Metallierung und die Carbonisierung erfolgen.

10. Verfahren nach einem oder mehreren der vorhergehenden Ansprüche,
dadurch gekennzeichnet,
daß
a) das R- oder S-Halogenid mit Magnesium in etherischen oder THF-Lösungen bei einer Temperatur von 4°C bis 15°C unter Beibehaltung der S- bzw. R-Konfiguration zur entsprechenden R- oder S-Metall-organischen-Grignard-Verbindung umgesetzt wird, und
b) durch diese die Grignard-Verbindung enthaltende Lösung CO₂ geleitet wird, um das Endprodukt zu erhalten, wobei bevorzugt
die S-Halogenide zu den organischen S-Magnesiumhalogeniden umgewandelt werden, welche zu wenigstens 90 Gew.-% die S-Konfiguration der organischen Magnesiumhalogenide aufweisen, weiterhin bevorzugt,
daß die R-Halogenide zu den organischen R-Magnesiumhalogeniden umgewandelt werden, welche zu wenigstens 90 Gew.-% die R-Konfiguration der organischen Magnesiumhalogenide aufweisen, weiterhin bevorzugt,
daß die S- oder R-Enantiomere nach Anspruch 1 dadurch erhalten werden, daß Kohlendioxid durch eine Lösung aus S- oder R-Magnesiumhalogeniden geleitet wird, wobei S-oder R-Carbonsäuren entstehen, welche zu wenigstens 95 - 98 Gew.-% die S- oder R-Konfiguration der Carbonsäuren aufweisen.

11. Verfahren nach einem oder mehreren der vorhergehenden Ansprüche,
dadurch gekennzeichnet,
daß die Metallierung mit Methyllithium durchgeführt wird, oder
daß die Metallierung mit Quecksilberverbindungen durchgeführt wird, und bevorzugt,
daß als Quecksilberverbindungen HgCl₂, HgBr₂, Hg(CN)₂ oder Hg(SCN)₂ verwendet werden, weiterhin bevorzugt, daß die R-Halogenide zu den organischen R-Quecksilberhalogeniden umgewandelt werden, welche zu wenigstens 95 - 98 Gew.-% die R-Konfiguration der organischen Quecksilberhalogenide aufweisen, ebenfalls bevorzugt,
daß die S-Halogenide zu den organischen S-Quecksilberhalogeniden umgewandelt werden, welche zu wenigstens 95 - 98 Gew.-% die S-Konfiguration der organischen Quecksilberhalogenide aufweisen, oder daß die S- oder R-Halogenide zu den S- oder R-quecksilberorganischen Halogeniden umgewandelt werden, welche zu wenigstens 95 Gew.-% die S- oder R-Konfiguration der quecksilberorganischen Halogenide aufweisen.

12. Verfahren nach einem oder mehreren der vorhergehenden Ansprüche,
dadurch gekennzeichnet,
daß das Natriumtetracarbonylferrat (II) durch die Behandlung von Fe(CO)₅ mit Natriumamalgam (NaHg) in THF hergestellt wird, und
als sekundäre Amine bevorzugt niedere n-Dialkylamine mit Alkylresten von C₁ - C₆ verwendet werden und insbesondere bevorzugt,
daß als sekundäre Amine Dimethylamin, Diethylamin oder Dibutylamin verwendet werden.

13. Verfahren nach einem oder mehreren der vorhergehenden Ansprüche,
dadurch gekennzeichnet,
daß das R-Halogenid zur entsprechenden S-Carbonsäure umgewandelt wird, welche zu wenigstens 95 - 98 Gew.% die S-Konfiguration der Carbonsäure aufweist, bevorzugt, daß das R-Halogenid mit Natriumcyanid oder Kaliumcyanid, gelöst in Wasser, zum entsprechenden S-Cyanid und weiter mit NaOH und H₂O₂ zur entsprechenden S-Carbonsäure umgesetzt wird, weiterhin bevorzugt,
daß das S-Halogenid mit Natriumcyanid oder Kaliumcyanid, gelöst in Wasser, zum entsprechenden R-Cyanid und weiter mit NaOH und H₂O₂ zur entsprechenden R-Carbonsäure umgesetzt wird, weiterhin bevorzugt,
daß das S-Halogenid durch Quecksilber-(II)-Cyanid zum entsprechenden S-Quecksilbercyanid umgesetzt wird, welches zu wenigstens 95 Gew.% die S-Konfiguration des Quecksilber-organischen Cyanids aufweist, ebenfalls bevorzugt,
daß die optisch aktiven R-Carbinole in Gegenwart von Quecksilber-(II)-Cyanid zu den entsprechenden S-Quecksilber-organischen Cyaniden umgesetzt werden, welche zu wenigstens 95 Gew.% die S-Konfiguration der organischen Quecksilbercyanide aufweisen.

14. Verfahren nach einem oder mehreren der vorhergehenden Ansprüche,
dadurch gekennzeichnet,
daß das Endprodukt Ibuprofen oder Naproxen, bevorzugt S(+)-Ibuprofen oder S(+)-Naproxen ist.

15. Verfahren nach einem oder mehreren der vorhergehenden Ansprüche,
dadurch gekennzeichnet,
daß die Salze als Komplexe aus 2-Aryl-alkansäuren und D-Glukamin im stöchiometrischen Verhältnis von 1 : 1 hergestellt werden, wobei sie zu wenigstens 70 - 95 Gew.-% , insbesondere bevorzugt zu wenigstens 90 Gew.-%, die S-Konfigurationen des Komplexes aufweisen, oder ebenfalls bevorzugt, daß die Salze als Komplexe aus 2-Aryl-alkansäuren und D-Ribamin im stöchiometrischen Verhältnis von 1 : 1 hergestellt werden, wobei sie zu wenigstens 70 - 98 Gew.-%, insbesondere bevorzugt zu wenigstens 90 Gew.-%,die S-Konfiguration des Komplexes aufweisen, oder ebenfalls bevorzugt, daß die Salze als Komplexe aus 2-Aryl-alkansäuren und kationischen Detergentien mit einer Kettenlänge von C₁₄ - C₁₆ hergestellt werden, oder ebenfalls bevorzugt, daß die Salze als Komplexe aus 2-Aryl-alkansäuren und Hexadecylpyridinium im stöchiometrischen Verhältnis 1 : 1 hergestellt werden, wobei sie zu wenigstens 70 - 98 Gew.-% die S-Konfiguration des Komplexes aufweisen, oder ebenfalls bevorzugt, daß die Salze als Komplexe aus 2-Aryl-alkansäuren und Benzethonium im stöchiometrischen Verhältnis von 1 : 1 hergestellt werden, wobei sie zu wenigstens 70 - 98 Gew.-% die S-Konfiguration des Komplexes aufweisen.

16. Verfahren nach einem oder mehreren der vorhergehenden Ansprüche,
dadurch gekennzeichent,
daß die Komplexe Oberflächenaktivitäten mit einer Kritischen Mizellbildungskonzentration (KMK) von 1 x 10⁻² M/l bis 1 x 10⁻⁴ M/l aufweisen, bevorzugt,
daß die 1 : 1 Komplexe aus S-(+)-Ibuprofen und R-Lysin bestehen, wobei die optische Reinheit des S-(+)-Ibuprofens 94 - 98 % beträgt, weiterhin bevorzugt,
daß die 1 : 1 Komplexe aus S-(+)-Naproxen und R-Lysin bestehen, wobei die optische Reinheit des S-(+)-Naproxens 94 - 98 % beträgt, weiterhin bevorzugt,
daß die 1 : 1 Komplexe aus S-(+)-Ibuprofen und R-Arginin bestehen, wobei die optische Reinheit des S-(+)-Ibuprofens 94 - 98 % beträgt, weiterhin bevorzugt,
daß die 1 : 1 Komplexe aus S-(+)-Naproxen und R-Arginin bestehen, wobei die optische Reinheit des S-(+)-Naproxens 96 - 98 % beträgt, weiterhin bevorzugt,
daß die 1 : 1 Komplexe aus S-(+)-Ibuprofen und N-Methyl-2-D-Glucosamin bestehen, wobei die optische Reinheit des S-(+)-Ibuprofens 94 - 98 % beträgt, weiterhin bevorzugt, daS die 1 : 1 Komplexe aus S-(+)-Naproxen und N-Methyl-α-D-Glukosamin bestehen, weiterhin bevorzugt,
daß die 1 : 1 Komplexe aus S-(+)-Ibuprofen und N-Methyl-α-D-Galaktosamin bestehen, weiterhin bevorzugt,
daß die 1 : 1 Komplexe aus S-(+)-Naproxen und N-Methyl-α-D-Galaktosamin bestehen, weiterhin bevorzugt,
daß die 1 : 1 Komplexe aus S-(+)-Ibuprofen und Cholin bestehen, weiterhin bevorzugt,
daß die 1 : 1 Komplexe aus S-(+)-Naproxen und Cholin bestehen, wobei vorzugsweise
als anorganische Salze insbesondere Alkali- und Erdalkali-Salze des S-Ibuprofen und S-Naproxen verwendet werden,
weiterhin bevorzugt die Magnesiumsalze des S-(+)-Ibuprofen und S-(+)-Naproxen verwendet werden und bevorzugt die Natriumsalze des S-(+)-Ibuprofen und S-(+)-Naproxen in Gegenwart von 10 % (w/w) von Natriumkarbonat verwendet werden.

17. Verwendung der nach einem oder mehreren der vorhergehenden Ansprüche hergestellten Erzeugnisse zur Herstellung von pharmazeutischen Zubereitungen mit antiinflammatorischen und antipyretischen Aktivitäten.

## Claims

1. A process for preparing a pharmaceutically active compound in stereospecific form selected from the group of compounds having the formula: 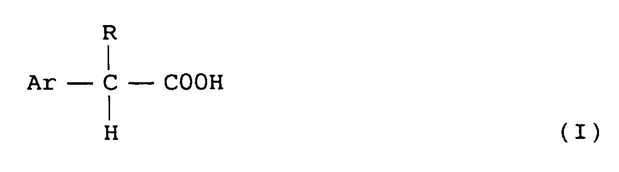 and their physiologically compatible salts and esters, wherein R is a C₁ - C₉ alkyl and Ar is a monocyclic, polycyclic or orthocondensed polycyclic aromatic group having up to 12 carbon atoms in the aromatic ring, and which may be substituted or unsubstituted in the aromatic ring, comprising the steps:
(a) reacting a carbonyl substrate of the formula: 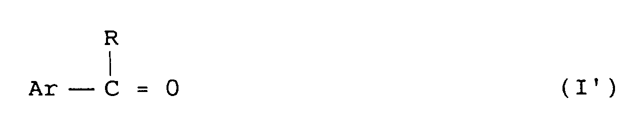 where R an Ar have the meanings given above, with a stereospecific reagent in the presence of a reducing agent, and organic solvent and a molecular sieve to form the enantiomeric R or S carbinol; and
(b) reacting the enantiomeric R or S carbinol with SO₂X₂ or SOX₂ or cyanuric chloride, wherein X is Cl or Br, to form the corresponding enantiomeric R or S halide, and, reacting said halide with:
(i) magnesium in ethereal or THF solutions, retaining the S or R-configuration, to the corresponding R or S-metal organic Grignard compound, and passing CO₂ through said solution containing the Grignard compound; or,
(ii) an alkyl or organo metallic compound in the presence of carbon dioxide; or
(iii) sodium tetracarbonyl ferrate (II) (Na₂Fe(CO)₄) in the presence of triphenyl phosphine (Ph₃P), forming as intermediate product 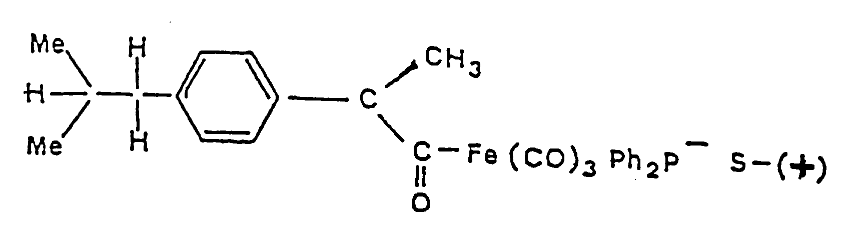 which is thereafter oxidized whith iodine water; or
(iv) sodium tetracarbonyl ferrate (II) (Na₂Fe(CO)₄) in the presence of molar amounts of triphenyl phosphine (Ph₃P) and a secondary amine to the corresponding amide, which is then hydrolyzed; or
(v) Na₂Fe(CO)₄ in the presence of CO, forming as intermediate product 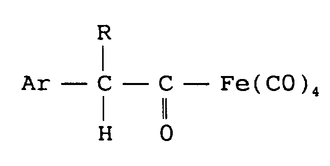 which is oxidized with oxygen or NaOCl and subsequently acid hydrolyzed; or
(vi) nickel carbonyl (Ni(CO)₄) in the presence of an alcohol and its conjugated base in accordance with the reaction 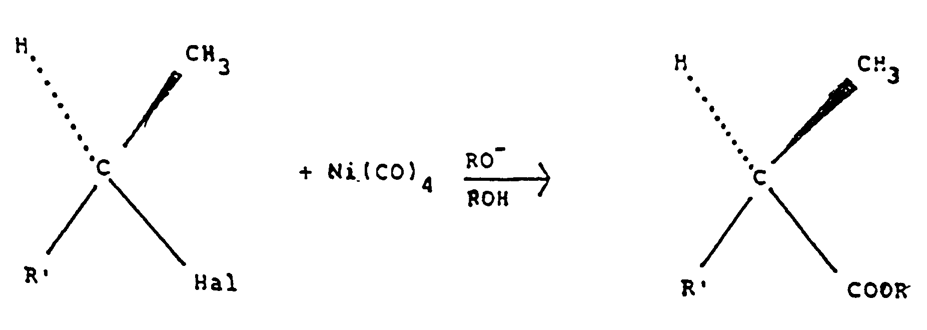 Hal = halogenide
ROH = alcohol
R' = aryl, as defined above; or
(vii) alkali cyanide dissolved in water, and that corresponding product is reacted with base and hydrogen peroxide; or
(viii) alkali cyanide followed by acid hydrolysis, in order to form the corresponding acid (I).

2. Process according to claim 1,
characterized in
that R is a C₁ - C₄, particularly preferably a C₁ alkyl, and the aromatic group is a phenyl, diphenyl or naphthyl group, and the substituents of said aromatic groups may consist of one or more halogen atoms, C₁ - C₄ alkylene, benzyl, hydroxy, C₁ - C₂ alkoxy, phenoxy, and benzoyl groups, and
Ar is selected from the group consisting of
4-isobutylphenyl
6-methoxy-2-naphthyl
3-phenoxy-phenyl
2'-fluoro-4-diphenyl
4'-fluoro-4-diphenyl
5-chloro-6-methoxy-2-naphthyl
5-bromo-6-methoxy-2-naphthyl
4-chloro-phenyl
4-difluoro-methoxy-phenyl
6-hydroxy-2-naphthyl and
5-bromo-6-hydroxy-2-naphthyl,
and preferably
that the carbonyl substrate has the formula: 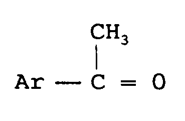 and preferably
that the stereospecific reagent is (+)-4-dimethyl-amino-3-methyl-1,2-diphenyl-2-butanol or 2,2'-dihydroxy-1,1'-dinaphthyl or (1S,2S)-(+)-2-amino-1-phenyl-propane-1,3-diol or S-(-)-1,1-diphenyl-prolinol or (1R,2R)-(-)-2-amino-1-phenyl-propane-1,3-diol, wherein for the stereospecific reagent the reducing agents in conjunction with the chiral complexing agents are selected from the group comprising:
a) LiAlH₄, LiBH₄, NaBH₄ and KBH₄, LiAlH₄ being preferred,
b) NaAlH₄, AlH₃.THF, Mg(AlH₄)₂, BH₃.THF
c) Al(BH₄)₃.THF; Ca(BH₄)₂.THF; Sr(BH₄)₂.Et₂O; Ba(BH₄)₂-Et₂O(THF), wherein preferably the stereospecific reagent forms a complex of BH₃.THF and S-(-)-1,1-diphenyl-prolinol or a complex of LiAlH₄ and (+)-4-dimethylamino-3-methyl-1,2-diphenyl-2-butanol or a complex of LiAlH₄ and 2,2'-dihydroxy-1,1'-dinaphthyl.

3. Process according to claim 1 or 2,
characterized in
that the organic solvent is tetrahydrofuran (THF) or 1,2-dimethoxyethane or an ether, preferably a diethylether, and that preferably zeolites are used as molecular sieves, the zeolites having preferably a specific pore passage which is defined by a 12 ring, 10 ring or 8 ring, wherein
the molecular sieves have a large capacity for water and oxygen,
so that the adsorption capacity for water preferably lies between 10 - 20 cm³/100 g activated molecular sieves, and that the adsorption capacity for oxygen preferably lies between 10 - 14 cm³/100 g activated molecular sieves, wherein
as molecular sieve further preferably a ZSM-5 zeolite is used, and
that the ratio of molecular sieves to the stereospecific catalyst amount is preferably between 1 -15 g per mmol, preferably between 2 - 10 g per mmol and more preferably 2 g per mmol, and particularly preferably that the ratio of ZSM-5 zeolite to the stereospecific catalyst amount is 5 g per mmol or 10 g per mmol.

4. Process according to one or more of the preceding claims,
characterized in
that the carbonyl substrate is added after formation of the LiAlH₄ complex and preferably that the stereospecific reagent is present with the lithium aluminium hydride in the organic solution as complex, and preferably that for forming the LiAlH₄ complex a stereospecific reagent and LiAlH₄ is added to an organic solvent while stirring, the formation of the LiAlH₄ complex preferably taking place in the presence of molecular sieves.

5. Process according to one or more of the preceding claims,
characterized in
that for forming the LiAlH₄ complex 1.0 to 100 ml of a stereospecific reagent and 0.5 to 15 g, preferably 0.5 to 10 g, particularly preferably 0.5 to 5 g of a molecular sieve are added to 10 to 100 ml of an organic solvent and dissolved at -60 to 22°C and to said solution at -60 to 22°C with continuous stirring 10 to 100 ml of a 1.0 to 2.0 M solution of LiAlH₄ are added, stirring continuing at a speed of 10 - 20 rpm until a homogeneous suspension is formed, wherein preferably
as reducing agent for the complex formation one of the compounds characterized in claim 2 is used and further preferably
that the homogeneous suspension is refluxed at a temperature of -60 to 22°C for 5 to 100 min.

6. Process according to one or more of the preceding claims,
characterized in
that after its formation the LiAlH₄ complex is allowed to stand for 5 to 100 min. at a temperature of 0 to -60°C or stirred for 5 to 100 min. to obtain the R-(-) form of the carbinol in the reaction of the carbonyl substrate, or that after its formation the LiAlH₄ complex is allowed to stand for 10 to 100 min. at a temperature of 0 to 22°C or is stirred for 10 to 100 min. to obtain the S-(+) form of the carbinol, or
that the LiAlH₄ complex is allowed to stand 8 hours in ethereal or THF solutions at temperatures between -7°C to 0°C before the carbonyl substrate is added to obtain the S-enantiomer of the carbinol, or
that the formation of the LiAlH₄ complex takes place at a temperature of 0°C to -60°C and 0 to 10 min. after formation of the complex the carbonyl substrate is added to obtain the R-enantiomer of the carbinol, wherein preferably
the reaction of the carbonyl substrate is carried out in the absence of oxygen and water.

7. Process according to one or more of the preceding claims,
characterized in
that the molar ratio of LiAlH₄ to the stereospecific reagent lies between 1.0 : 0.2 to 1.0 - 3.0, preferably that the molar ratio of LiAlH₄ to the stereospecific reagent lies between 1.0 : 2.3 to 1.0 - 2.5, further preferably
that the molar ratio of the LiAlH₄ complex to the carbonyl substrate lies between 1.0 : 0.2 to 1.0 - 2.5, furthermore preferably
that the molar ratio of the LiAlH₄ complex to the carbonyl substrate is 1.0 : 2.5, or
that the molar ratio of LiAlH₄ to the stereospecific reagent is 1.0 to 0.7, or
that the molar ratio of LiAlH₄ to the stereospecific reagent is 1.0 to 1.0, or
that the molar ratio of LiAlH₄ to the stereospecific reagent is 1.0 to 2.0, or
that the molar ratio of LiAlH₄ to the stereospecific reagent is 1.0 to 2.5, or
that the molar ratio of LiAlH₄ to the stereospecific reagent is 1.0 to 3.0, and
that preferably as organic solvent benzene or toluene or pentane or hexane is used if methoxy and/or chlorine and/or bromine and/or fluorine substituents are localized in the aryl groups of the carbonyl substrate.

8. Process according to one or more of the preceding claims,
characterized in
that the optical purity/activity of the halide is at least 80 %, preferably
that the S-carbinol is converted with SO₂Cl₂, SO₂Br₂, SOBr₂, SOCl₂ or cyanuric chloride to the S-halogenide having at least 95 - 98 % by weight of the S-configuration of the halide, or
that the R-carbinol is converted by SO₂Cl₂, SO₂Br₂, SOCl₂, SOBr₂ or cyanuric chloride to the R-halide having at least 95 - 98 % by weight of the R-configuration of the halide, or
that the R or S-carbinol is converted by SO₂Cl₂ or SO₂Br₂ or SOBr₂ or SOCl₂ in the presence of pyridine and H₂O to the S or R-halide, at least 95 - 98 % by weight of the halides being obtained in the S or R-configuration.

9. Process according to one or more of the preceding claims,
characterized in
that the stereospecific halogenation is carried out in anhydric 1,4-dioxane or in dry pyridine in the presence of thionyl chloride or thionyl bromide or cyanuric chloride, further preferably
that the enantiomeric carbinol is heated in the presence of pulverized cyanuric chloride (1 mol) or in the presence of a base to 10°C - 20°C above the boiling point of the carbinols and after 1 to 1.5 hours this reaction mixture is cooled, filtered and distilled under high vacuum, whereupon the metallization and carbonizing are carried out, further preferably
that the stereospecific halogenation is carried out with corresponding molar thionyl chloride or thionyl bromide at a temperature of -10 to -20°C, preferably -20°C, the metallization and the carbonizing thereafter being carried out.

10. Process according to one or more of the preceding claims,
characterized in
that
a) the R or S-halide is reacted with magnesium in ethereal or THF solutions at a temeprature of 4°C to 15°C, retaining the S or R-configuration, to the corresponding R or S-metal organic Grignard compound, and
b) CO₂ is passed through said solution containing the Grignard compound to obtain the end product, wherein preferably the S-halides are converted to the organic S-magnesium halides having at least 90 % by weight of the S-configuration of the organic magnesium halides, further preferably
that the R-halides are converted to the organic R-magnesium halides having at least 90 % by weight of the R-configuration of the organic magnesium halides, further preferably
that the S or R-enantiomers according to claim 1 are obtained by passing carbon dioxide through a solution of S or R-magnesium halides, giving S or R-carboxylic acids having at least 95 - 98 % by weight of the S or R-configuration of the carboxylic acids.

11. Process according to one or more of the preceding claims,
characterized in
that the metallization is carried out with methyl lithium, or
that the metallization is carried out with mercury compounds, and preferably
that as mercury compounds HgCl₂, HgBr₂, Hg(CN)₂ or Hg(SCN)₂ are used, further preferably
that the R-halides are converted to the organic R-mercury halides having at least 95 - 98 % of the R-configuration of the organic mercury halides, further preferably
that the S-halides are converted to the organic S-mercury halides having at least 95 - 98 % of the S-configuration of the organic mercury halides, or
that the S or R-halides are converted to the organic S or R-mercury halides having at least 95 % by weight of the S or R- configuration of the organic mercury halides.

12. Process according to one or more of the preceding claims,
characterized in
that the sodium tetracarbonyl ferrate (II) is prepared by treating Fe(CO)₅ with sodium amalgam (NaHg) in THF, and that as secondary amines lower n-dialkyl amines having alkyl radicals of C₁ - C₆ are preferably used and that as secondary amines dimethyl amine, diethyl amine or dibutyl amine are used.

13. Process according to one or more of the preceding claims,
characterized in
that the R-halide is converted to the corresponding S-carboxylic acid having at least 95 - 98 % by weight of the S-configuration of the carboxylic acid, preferably that the R-halide is converted with sodium cyanide or potassium cyanide, dissolved in water to the corresponding S-cyanide and furthermore with NaOH and H₂O₂ to the corresponding S-carboxylic acid, further preferably that the S-halide is converted with sodium cyanide or potassium cyanide, dissolved in water to the corresponding R-cyanide and furthermore with NaOH and H₂O₂ to the corresponding R-carboxylic acid, further preferably that the S-halide is converted by mercury (II) cyanide to the corresponding S-mercury cyanide having at least 95 % by weight or the S-configuration of the mercury organic cyanide, further preferably
that the optically active R-carbinols are converted in the presence of mercury (II) cyanide to the corresponding S-mercury organic cyanides having at least 95 % by weight of the S-configuration of the organic mercury cyanides.

14. Process according to one or more of the preceding claims,
characterized in
that the end product is ibuprofen or naproxen, preferably S-(+)-ibuprofen or S-(+)-naproxen.

15. Process according to one or more of the preceding claims,
characterized in
that the salts are prepared as complexes of 2-aryl-alkanoic acids and D-glucamine in the stoichiometric ratio 1:1, having at least 70 - 95 % by weight, particularly preferably at least 90 % by weight, of the S-configurations of the complex, or further preferably
that the salts are prepared as complexes of 2-aryl-alkanoic acids and D-ribamine in the stoichiometric ratio 1:1, having at least 70 to 98 % by weight, preferably at least 90 % by weight, of the S-configurations of the complex, or further preferably
that the salts are prepared as complexes of 2-aryl-alkanoic acids and cationic detergents having a chain length of C₁₄ - C₁₆, or further preferably
that the salts are prepared as complexes of 2-aryl-alkanoic acids and hexadecylpyridinium in the stoichiometric ratio of 1:1, having at least 70 - 98 % by weight of the S-configuration of the complex, or further preferably that the salts are prepared as complexes of 2-aryl-alkanoic acids and benzethonium in the stoichiometric ratio of 1:1, having at least 70 - 98 % by weight of the S-configuration of the complex.

16. Process according to one or more of the preceding claims,
characterized in
that the complexes have surface activities with a criterical micelle concentration (CMC) of 1 x 10⁻² M/l to 1 x 10⁻⁴ M/l, preferably
that the 1:1 complexes consist of S-(+)-ibuprofen and R-lysine, the optical purity of the S-(+)-ibuprofen being 94 - 98 %, further preferably
that the 1:1 complexes consist of S-(+)-naproxen and R-lysine, the optical purity of the S-(+)-naproxen being 94 - 98 %, further preferably
that the 1:1 complexes consist of S-(+)-ibuprofen and R-arginine, the optical purity of the S-(+)-ibuprofen being 94 - 98 %, further preferably
that the 1:1 complexes consist of S-(+)-naproxen and R-arginine, the optical purity of the S-(+)-naproxen being 96 - 98 %, further preferably
that the 1:1 complexes consist of S-(+)-ibuprofen and N-methyl-2-D-glucosamine, the optical purity of the S-(+)-ibuprofen being 94 - 98 %, further preferably
that the 1:1 complexes consist of S-(+)-naproxen and N-methyl-α-D-glucosamine, further preferably
that the 1:1 complexes consist of S-(+)-ibuprofen and N-methyl-α-D-galactosamine, further preferably
that the 1:1 complexes consist of S-(+)-naproxen and N-methyl-α-D-galactosamine, further preferably
that the 1:1 complexes consist of S-(+)-ibuprofen and choline, further preferably
that the 1:1 complexes consist of S-(+)-naproxen and choline, wherein preferably
as inorganic salts in particular alkaline and alkaline earth salts of S-ibuprofen and S-naproxen are used, furthermore
that preferably the magnesium salts of S-(+)-ibuprofen and S-(+)-naproxen are used, and
that preferably the sodium salts of S-(+)-ibuprofen and S-(+)-naproxen are used in the presence of 10 % (w/w) sodium carbonate.

17. Use of the products prepared according to one or more of the preceding claims for the preparation of pharmaceutical compounds with anti-inflammatory and antipyretic activities.

## Revendications

1. Procédé de préparation d'un composé pharmaceutiquement actif sous une forme stéréospécifique, choisi dans le groupe des composés répondant à la formule : 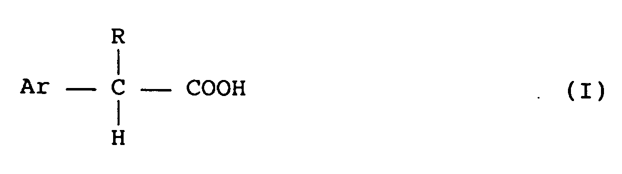 et de leurs sels et esters physiologiquement acceptables, où R est un groupe alkyle en C₁-C₉, et Ar est un groupe monocyclique, polycyclique ou polycyclique condensé en position ortho, contenant jusqu'à 12 atomes de carbone dans le noyau aromatique, ledit noyau aromatique pouvant être substitué ou non substitué,
comprenant les étapes de :
a) mise en réaction d'un substrat de carbonyle de formule : 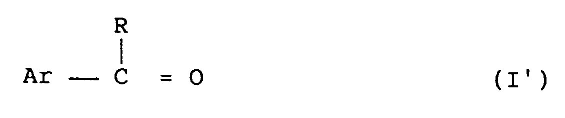 où R et Ar correspondent aux définitions indiquées ci-dessus, avec un réactif stéréospécifique, en présence d'un réducteur, d'un solvant organique ou d'un tamis moléculaire, pour former l'énantiomère R- ou S-carbinol, et
b) mise en réaction de l'énantiomère R- ou S-carbinol avec SO₂X₂ ou SOX₂, où X = Cl ou Br, ou avec du chlorure de cyanuryle, pour former l'énantiomère R- ou S-halogénure, et mise en réaction de l'halogénure avec
(i) du magnésium dans une solution éthérée ou une solution de THF, avec maintien de la configuration S ou R, pour former le composé organométallique R ou S correspondant de Grignard, et barbotage de CO₂ dans la solution contenant le composé de Grignard ; ou
(ii) un groupe alkyle ou un composé organométallique en présence de CO₂ ; ou
(iii) du tétracarbonylferrate (II) de sodium (Na₂Fe(CO)₄) en présence de triphénylphosphine (Ph₃P), avec formation du produit intermédiaire 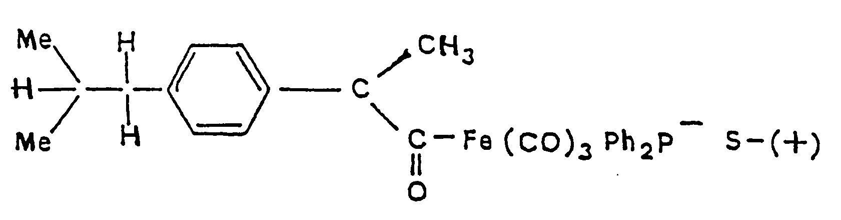 qu'on oxyde ensuite avec de l'eau iodée ; ou
(iv) du tétracarbonylferrate (II) de sodium (Na₂Fe(CO)₄) en présence de quantités molaires de triphénylphosphine (Ph₃P) et d'une amine secondaire pour former l'amide correspondant qu'on hydrolyse ensuite ; ou
(v) (Na₂Fe(CO)₄) en présence de CO, avec formation du produit intermédiaire 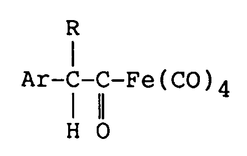 qu'on oxyde ensuite avec de l'oxygène ou NaOCl et qu'on hydrolyse ensuite avec un acide ; ou
(vi) du nickel-carbonyle (Ni(CO)₄) en présence d'un alcool et de sa base conjuguée, conformément à la réaction suivante : 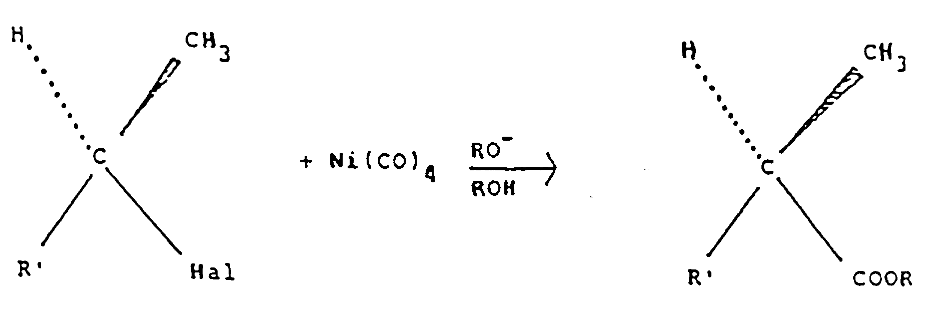 où :
Hal = halogénure ;
ROH = alcool ;
R' = un reste aryle tel que défini ci-dessus de façon plus détaillée ; ou
(vii) un cyanure alcalin dissous dans de l'eau, et mise en réaction du produit avec une base et du peroxyde d'hydrogène ; ou
(viii) un cyanure alcalin, cette réaction étant suivie d'une hydrolyse acide pour former l'acide (I) correspondant.

2. Procédé selon la revendication 1, caractérisé en ce que R est un groupe en C₁-C₄, de préférence un groupe alkyle en C₁, et le groupe aromatique est un groupe phényle, diphényle ou naphtyle, les substituants de ces groupes aromatiques pouvant être formés par un ou plusieurs atomes d'halogène ou des groupes alkylène en C₁-C₄, benzyle, hydroxy, alcoxy en C₁-C₂, phénoxy et benzoyle,
en ce que Ar est choisi dans l'ensemble constitué par les groupes 4-isobutylphényle, 6-méthoxy-2-naphtyle, 3-phénoxyphényle, 2'-fluoro-4-diphényle, 4'-fluoro-4-diphényle, 5-chloro-6-méthoxy-2-naphtyle, 5-bromo-6-méthoxy-2-naphtyle, 4-chlorophényle, 4-difluorométhoxyphényle, 6-hydroxy-2-naphtyle et 5-bromo-6-hydroxy-2-naphtyle, et, de préférence, en ce que le substrat de carbonyle possède la formule : 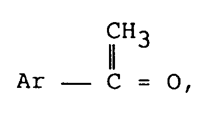 et de préférence en ce que le réactif stéréospécifique est le (+)-4-diméthylamino-3-méthyl-1,2-diphényl-2-butanol ou le (1S,2S)-(+)-2-amino-1-phényl-propane-1,3-diol ou le (1R,2R)-(-)-2-amino-1-phényl-propane-1,3-diol ou le 2,2'-dihydroxy-1,1'-dinaphtyle ou le S-(-)-1,1-diphénylprolinol, et pour le réactif stéréospécifique, les réducteurs étant choisis en rapport avec les complexants chiraux dans l'ensemble comprenant :
a) LiAlH₄, LiBH₄, NaBH₄, ainsi que KBH₄, de préférence LiAlH₄,
b) NaAlH₄, AlH₃.THF, Mg(AlH₄)₂, BH₃.THF,
c) Al(BH₄)₃.THF, Ca(BH₄)₂.THF, Sr(BH₄)₂.Et₂O, Ba(BH₄)₂-Et₂O(THF),
et de préférence, le réactif stéréospécifique forme un complexe de BH₃.THF et S-(-)-1,1-diphénylprolinol, ou un complexe de LiAlH₄ et (+)-4-diméthylamino-3-méthyl-1,2-diphényl-2-butanol, ou un complexe de LiAlH₄ et 2,2'-dihydroxy-1,1'-dinaphtyle.

3. Procédé selon la revendication 1 ou 2, caractérisé en ce que le solvant organique est le tétrahydrofuranne (THF) ou le 1,2-diméthoxyéthane ou un éther, de préférence l'éther diéthylique, et en ce qu'on utilise, comme tamis moléculaires, de préférence, des zéolithes qui possèdent, de préférence, un canal de pores qui est limité par un noyau 12, 10 ou 8, les tamis moléculaires ayant une grande capacité pour l'eau et l'oxygène, de sorte que, de préférence, la capacité d'adsorption pour l'eau est comprise entre 10 et 20 cm³/100 g de tamis moléculaire activé, et la capacité d'adsorption pour l'oxygène est comprise, de préférence, entre 10 et 14 cm³/100 g de tamis moléculaire activé, et de préférence, on utilise aussi, comme tamis moléculaire, une zéolithe ZSM-5, le rapport des tamis moléculaires à la quantité de catalyseur stéréospécifique étant compris, de préférence, entre 1 et 15 g/mmol, de préférence entre 2 et 10 g/mmol, d'une manière plus préférée 2 g/mmol, et d'une manière particulièrement préférée, en ce que le rapport de la zéolithe ZMS-5 à la quantité de catalyseur stéréospécifique est de 5 g/mmol ou de 10 g/mmol.

4. Procédé selon une ou plusieurs des revendications précédentes, caractérisé en ce que le substrat de carbonyle est ajouté après la formation du complexe de LiAlH₄ et, de préférence, en ce que le réactif stéréospécifique est présent dans la solution organique sous forme de complexe avec l'hydruroaluminate de lithium, et de préférence, en ce que, pour la formation du complexe de LiAlH₄, on ajoute un réactif stéréospécifique et LiAlH₄ à un solvant organique, sous agitation, la formation du complexe de LiAlH₄ étant effectuée en présence du tamis moléculaire.

5. Procédé selon une ou plusieurs des revendications précédentes, caractérisé en ce que, pour la formation du complexe de LiAlH₄, on ajoute, à 10 à 100 ml d'un solvant organique, 1,0 à 100 ml d'un réactif stéréospécifique et 0,5 à 15 g, de préférence 0,5 à 10 g, d'une manière particulièrement préférée 0,5 à 5 g, d'un tamis moléculaire, qu'on dissout à une température comprise entre -60 et 22°C, et on ajoute à cette solution, sous agitation constante et à une température comprise entre -60 et 22°C, 10 à 100 ml d'une solution 1,0 à 2,0 M de LiAlH₄, et on continue d'agiter la solution résultante à une vitesse de 10 à 20 tr/min jusqu'à ce qu'une suspension homogène soit formée, de préférence en utilisant un des composés caractérisés dans la revendication 2, comme réducteur pour la formation du complexe, et de préférence, on chauffe la suspension homogène au reflux, à une température de -60 à 22°C, pendant 5 à 100 minutes.

6. Procédé selon une ou plusieurs des revendications précédentes, caractérisé en ce qu'après la formation du complexe de LiAlH₄, on laisse reposer celui-ci pendant 5 à 100 minutes, à une température de 0 à -60°C, ou on l'agite pendant 5 à 100 minutes, pour obtenir la forme R-(-) du carbinol lors de la réaction du substrat de carbonyle, ou en ce qu'après la formation du complexe de LiAlH₄, on laisse reposer celui-ci pendant 10 à 100 minutes, à une température de 0 à 22°C, ou on l'agite pendant 10 à 100 minutes, pour obtenir la forme S-(+) du carbinol, ou en ce qu'on laisse reposer le complexe de LiAlH₄ pendant 8 heures dans des solutions éthérées ou des solutions de THF, à des températures comprises entre -7°C et 0°C, avant d'ajouter le substrat de carbonyle, pour obtenir l'énantiomère S du carbinol, ou en ce qu'on effectue la formation du complexe de LiAlH₄ à une température de 0°C à -60°C et que 0 à 10 minutes après la formation du complexe, on ajoute le substrat de carbonyle pour obtenir l'énantiomère R du carbinol, la mise en réaction du substrat de carbonyle étant effectuée, de préférence, en l'absence d'oxygène et d'eau.

7. Procédé selon une ou plusieurs des revendications précédentes, caractérisé en ce que le rapport molaire de LiAlH₄ au réactif stéréospécifique est compris entre 1,0 : 0,2 et 1,0 : 3,0, de préférence que le rapport molaire de LiAlH₄ au réactif stéréospécifique est compris entre 1,0 : 2,3 et 1,0 : 2,5, de préférence que le rapport molaire du complexe de LiAlH₄ au substrat de carbonyle est compris entre 1,0 : 0,2 et 1,0 : 2,5, de préférence aussi que le rapport molaire du complexe de LiAlH₄ au substrat de carbonyle est de 1,0 : 2,5, ou que le rapport molaire de LiAlH₄ au réactif stéréospécifique est de 1,0 : 0,7, ou que le rapport molaire de LiAlH₄ au réactif stéréospécifique est de 1,0 : 1,0, ou que le rapport molaire de LiAlH₄ au reactif stéréospécifique est de 1,0 : 2,0, ou que le rapport mo-laire de LiAlH₄ au réactif stéréospécifique est de 1,0 : 2,5, ou que le rapport molaire de LiAlH₄ au réactif stéréospécifique est de 1,0 : 3,0, et que, de préférence, comme solvant organique, on utilise du benzène ou du toluène ou du pentane ou de l'hexane quand des substituants méthoxy et/ou chlore et/ou brome et/ou fluor sont situés dans les groupes aryles du substrat de carbonyle.

8. Procédé selon une ou plusieurs des revendications précédentes, caractérisé en ce que la pureté optique/activité de l'halogénure est d'au moins 80 %, de préférence en ce que le S-carbinol est transformé avec SO₂Cl₂, SO₂Br₂, SOBr₂, SOCl₂ ou du chlorure de cyanuryle, pour donner l'halogénure S dont 95 à 98 % en poids présentent la configuration S de l'halogénure, ou en ce que le R-carbinol est transformé au moyen de SO₂Cl₂, SO₂Br₂, SOCl₂, SOBr₂ ou du chlorure de cyanuryle, pour donner l'halogénure R dont 95 à 98 % en poids présentent la configuration R de l'halogénure, ou en ce que le R-carbinol ou le S-carbinol est transformé par SO₂Cl₂ ou SO₂Br₂ ou SOCl₂ ou SOBr₂, en présence de pyridine et de H₂O, pour donner l'halogénure S ou R, au moins 95 à 98 % en poids des halogénures étant obtenus avec la configuration S ou R.

9. Procédé selon une ou plusieurs des revendications précédentes, caractérisé en ce qu'on effectue l'halogénation stéréospécifique dans du 1,4-dioxanne anhydre ou dans de la pyridine anhydre, en présence de chlorure de thionyle ou de bromure de thionyle ou de chlorure de cyanuryle, de préférence aussi en ce qu'on chauffe l'énantiomère de carbinol, en présence de chlorure de cyanuryle pulvérisé (1 mole) ou en présence d'une base, à 10 à 20°C au-dessus du point d'ébullition des carbinols, et après 1 à 1,5 heure, on refroidit ce mélange de réaction, on le filtre et on le distille sous un vide poussé, après quoi on effectue la métallisation et la carbonisation, et de préférence, on effectue l'halogénation stéréospécifique avec une quantité molaire correspondante de chlorure de thionyle ou de bromure de thionyle, à une température de -10 à -20°C, de préférence à -20°C, et ensuite, on effectue la métallisation et la carbonisation.

10. Procédé selon une ou plusieurs des revendications précédentes, caractérisé en ce que
a) on fait réagir l'halogénure R ou S avec du magnésium dans des solutions éthérées ou des solutions de THF, à une température de 4°C à 15°C, avec le maintien de la configuration S ou R, pour obtenir le composé organométallique de Grignard R ou S correspondant, et
b) on amène du CO₂ dans cette solution contenant le composé de Grignard pour obtenir le produit final,
et de préférence, on transforme les halogénures S en les halogénures organiques S de magnésium, dont au moins 90 % en poids présentent la configuration S des halogénures organiques de magnésium, de préférence, on transforme les halogénures R en les halogénures organiques R de magnésium, dont au moins 90 % en poids présentent la configuration R des halogénures organiques de magnésium, et de préférence, on obtient les énantiomères S ou R selon la revendication 1 en faisant passer du dioxyde de carbone dans une solution d'halogénures S ou R de magnésium, des acides S- ou R-carboxyliques étant formés, dont au moins 95 à 98 % en poids présentent la configuration S ou R des acides carboxyliques.

11. Procédé selon une ou plusieurs des revendications précédentes, caractérisé en ce qu'on effectue la métallisation avec du méthyl-lithium ou on effectue la métallisation avec des composés du mercure, et de préférence, on utilise, comme composés du mercure, HgCl₂, HgBr₂, Hg(CN)₂ ou Hg(SCN)₂, de préférence on transforme les halogénures R en halogénures organiques R de mercure dont 95 à 98 % en poids présentent la configuration R des halogénures organiques de mercure, et de préférence, on transforme les halogénures S en halogénures organiques S de mercure dont 95 à 98 % en poids présentent la configuration S des halogénures organiques de mercure, ou on transforme les halogénures S ou R en halogénures organiques S ou R de mercure, dont au moins 95% en poids présentent la configuration S ou R des halogénures organiques de mercure.

12. Procédé selon une ou plusieurs des revendications précédentes, caractérisé en ce qu'on prépare le tétracarbonylferrate (II) de sodium grâce au traitement de Fe(CO)₅ avec un amalgame de sodium (NaHg) dans du THF, et, comme amines secondaires, on utilise, de préférence, des n-dialkylamines inférieures ayant des restes alkyles en C₁-C₆, et d'une manière particulièrement préférée, on utilise, comme amines secondaires, la diméthylamine, la diéthylamine ou la la dibutylamine.

13. Procédé selon une ou plusieurs des revendications précédentes, caractérisé en ce qu'on transforme l'halogénure R en l'acide S-carboxylique correspondant, dont 95 à 98 % en poids présentent la configuration S de l'acide carboxylique, de préférence, on fait réagir l'halogénure R avec du cyanure de sodium ou du cyanure de potassium, dissous dans de l'eau, pour former le cyanure S correspondant, et, en outre, avec NaOH et H₂O₂ pour former l'acide S-carboxylique correspondant, de préférence on fait réagir l'halogénure S avec du cyanure de sodium ou du cyanure de potassium, dissous dans de l'eau, pour former le cyanure R correspondant, et, en outre, avec NaOH et H₂O₂ pour former l'acide R-carboxylique correspondant, de préférence, on fait réagir l'halogénure S avec du cyanure de mercure (II) pour former le cyanure S de mercure correspondant, dont au moins 95 % en poids présentent la configuration S du cyanure organique de mercure, de préférence on fait réagir les R-carbinols optiquement actifs en présence de cyanure de mercure (II) pour former les cyanures organiques S de mercure correspondants, dont au moins 95 % en poids présentent la configuration S des cyanures organiques de mercure.

14. Procédé selon une ou plusieurs des revendications précédentes, caractérisé en ce que le produit final est l'ibuprofène ou le naproxène, de préférence le S-(+)-ibuprofène ou le S-(+)-naproxène.

15. Procédé selon une ou plusieurs des revendications précédentes, caractérisé en ce qu'on prépare les sels sous forme de complexes d'acides 2-arylalcanoïques et de D-glucamine à un rapport stoechiométrique de 1:1, dont au moins 70 à 95 % en poids, d'une manière particulièrement préférée au moins 90 % en poids présentent la configuration S du complexe, ou, d'une manière également préférée, on prépare les sels sous forme de complexes d'acides 2-arylalcanoïques et de D-ribamine à un rapport stoechiométrique de 1:1, dont au moins 70 à 98 % en poids, d'une manière particulièrement préférée au moins 90 % en poids présentent la configuration S du complexe, ou, d'une manière également préférée, on prépare les sels sous forme de complexes d'acides 2-arylalcanoïques et de détergents cationiques ayant une longueur de chaîne de C₁₄ à C₁₆, ou, d'une manière également préférée, on prépare les sels sous forme de complexes d'acides 2-arylalcanoïques et d'hexadécylpyridinium à un rapport stoechiométrique de 1:1, dont au moins 70 à 98 % en poids présentent la configuration S du complexe, ou, d'une manière également préférée, on prépare les sels sous forme de complexes d'acides 2-arylalcanoïques et de benzéthonium à un rapport stoechiométrique de 1:1, dont au moins 70 à 98 % en poids présentent la configuration S du complexe.

16. Procédé selon une ou plusieurs des revendications précédentes, caractérisé en ce que les complexes présentent des activités superficielles avec une concentration critique de formation de micelles (KMK) de 1 x 10⁻² M/l à 1 x 10⁻⁴ M/l, de préférence, les complexes sont formés de S-(+)-ibuprofène et de R-lysine (1:1), la pureté optique du S-(+)-ibuprofène étant de 94 à 98 %, de préférence, les complexes sont formés de S-(+)-naproxène et de R-lysine (1:1), la pureté optique du S-(+)-naproxène étant de 94 à 98 %, de préférence, les complexes sont formés de S-(+)-ibuprofène et de R-arginine (1:1), la pureté optique du S-(+)-ibuprofène étant de 94 à 98 %, de préférence, les complexes sont formés de S-(+)-naproxène et de R-arginine (1:1), la pureté optique du S-(+)-naproxène étant de 96 à 98 %, de préférence, les complexes sont formés de S-(+)-ibuprofène et de N-méthyl-2-D-glucosamine (1:1), la pureté optique du S-(+)-ibuprofène étant de 94 à 98 %, de préférence, les complexes sont formés de S-(+)-naproxène et de N-méthyl-α-D-glucosamine (1:1), de préférence, les complexes sont formés de S-(+)-ibuprofène et de N-méthyl-α-D-galactosamine (1:1), de préférence, les complexes sont formés de S-(+)-naproxène et de N-méthyl-α-D-galactosamine (1:1), de préférence, les complexes sont formés de S-(+)-ibuprofène et de choline (1:1), de préférence, les complexes sont formés de S-(+)-naproxène et de choline (1:1), et, de préférence, comme sels inorganiques, on utilise des sels alcalins et des sels alcalino-terreux du S-ibuprofène et du S-naproxène, et de préférence, on utilise les sels de magnésium du S-(+)-ibuprofène et du S-(+)-naproxène en présence de 10 % (en poids/poids) de carbonate de sodium.

17. Utilisation des produits préparés selon une ou plusieurs des revendications précédentes pour la préparation de compositions pharmaceutiques ayant des activités anti-inflammatoires et antipyrétiques.
